# EUROPÄISCHE PATENTSCHRIFT

(11) **EP 0 481 274 B1**
(45) Veröffentlichungstag und Bekanntmachung des Hinweises auf die Patenterteilung: **23.10.1996**
(21) Anmeldenummer: 91116651.0
(22) Anmeldetag: 30.09.1991
(51) Int. Cl.: C07D 487/04, C07D 487/14, C07D 513/14, C07D 471/14, C07D 519/00, A61K 31/47

(54) **7-(2,7-Diazabicyclo[3,3.0]octyl)-3-chinolon- und -naphthyridoncarbonsäure-Derivate**
7-(2,7-Diazabicyclo[3.3.0]octyl)-3-quinolone- and -naphthyridonecarboxylic acid derivatives
Dérivés des acides 7-(2,7-diazabicyclo[3.3.0]octyl)-3-quinolone- et -naphtyridonecarboxyliques

(30) Priorität: 13.10.1990 DE 4032560
(43) Veröffentlichungstag der Anmeldung: 22.04.1992
(73) Patentinhaber: BAYER AG, 51368 Leverkusen (DE)
(72) Erfinder: Petersen, Uwe, Dr., W-5090 Leverkusen 1 (DE); Schenke, Thomas, Dr., W-5060 Bergisch Gladbach (DE); Schriever, Michael, Dr., W-5068 Odenthal (DE); Grohe, Klaus, Dr., W-5068 Odenthal (DE); Krebs, Andreas, Dr., W-5068 Odenthal (DE); Haller, Ingo, Dr., W-5600 Wuppertal 1 (DE); Metzger, Karl Georg, Dr., W-5600 Wuppertal (DE); Bremm, Klaus-Dieter, Dr., W-5600 Wuppertal (DE); Endermann, Rainer, Dr., W-5600 Wuppertal 1 (DE); Zeiler, Hans-Joachim, Dr., W-5600 Wuppertal (DE)

(56) Entgegenhaltungen:
- EP-A- 0 106 489
- EP-A- 0 172 651
- EP-A- 0 284 935
- EP-A- 0 343 524
- EP-A- 0 350 733
- J.Clin.Pharmacol. 28, 156 (1988)
- Antimicrob.Agents Chemother. 33, 131 (1989)

## Beschreibung

Die Erfindung betrifft neue 7-(2,7-Diazabicyclo[3.3.0]octyl)-3-chinolon- und -naphthyridoncarbonsäure-Derivate, Verfahren zu ihrer Herstellung sowie diese enthaltende antibakterielle Mittel und Futterzusatzstoffe.

Es sind bereits aus der EP-A-0 350 733 3-Chinolon-und Naphthyridoncarbonsäuren bekanntgeworden, die in 7-Position durch einen 2,7-Diazabicyclo[3.3.0]octyl-Ring substituiert sind.

Auch aus EP-A-0 343 524, EP-A-0 106 489, EP-A-0 284 935, EP-A-0 172 651, J. Clin. Pharmacol. 28 156(1988) und Antimcrob. Agents Chemother. 33, 131 (1989) sind antibakteriell wirksame Chinolon- und Naphthyridoncarbonsäure-Derivate bekannt geworden, jedoch ist deren Wirkstärke und Wirkspektrum nicht zufriedenstellend. Es wurde gefunden, daß die neuen 7-(2,7-Diazabicyclo[3.3.0]octyl)-3-chinolon- und -naphthyridoncarbonsäure-Derivate der Formel (I) 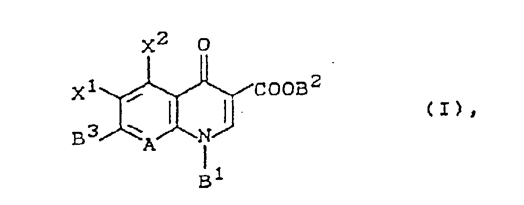 in welcher
X¹ für Halogen,
X² für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen, Methyl,
B¹ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
B² für Wasserstoff, Alkyl mit 1 bis 4 Kohlenstoffatomen oder (5-Methyl-2-oxo-1,3-dioxol-4-yl)methyl
B³ für einen Rest der Struktur 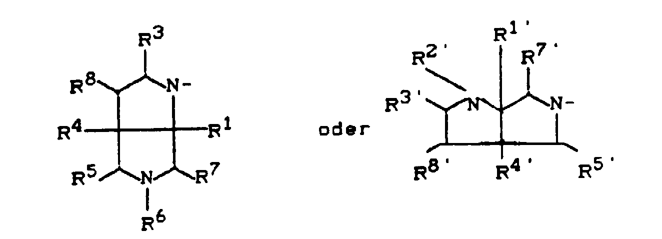 steht,
   worin
R¹, R³; R⁴, R⁵, R⁷ und R⁸ gleich oder verschieden sind und jeweils für Wasserstoff oder Methyl, das gegebenenfalls durch Halogen oder Hydroxy substituiert sein kann, C₁-C₂-Alkoxycarbonyl oder Carboxy stehen,
R⁴ und R⁸ zusätzlich für Halogen, Amino, Hydroxy oder Methoxy stehen können,
R⁶ für Wasserstoff, C₁-C₆-Alkyl, Benzyl, C₆-C₁₂-Aryl, C₁-C₃-Alkanoyl, Benzoyl, C₁-C₅-Alkoxycarbonyl steht,
R¹', R²', R³', R⁵', R7 und R8 gleich oder verschieden sind und jeweils für Wasserstoff oder Methyl stehen,
R⁴' für Halogen, Amino, Hydroxy, Methoxy oder für durch Halogen, Amino, Hydroxy oder Methoxy substituiertes Methyl steht oder dann für Wasserstoff steht, wenn
R²' und R³' zusammen eine C₂-C₄-Alkylenbrücke oder eine CH₂₋S-CH₂-Brücke bedeuten, die gegebenenfalls durch Hydroxy, Methoxy, Amino oder Methyl ein-oder zweifach substituiert sein kann,
A für N oder C-R⁹ steht, worin
R⁹ für H, Halogen, Methyl, Cyano, Nitro, Hydroxy oder Methoxy steht

und deren pharmazeutisch verwendbaren Hydrate und Säureadditionssalze sowie die Alkali-, Erdalkali-, Silber- und Guanidiniumsalze der zugrundeliegenden Carbonsäuren eine hohe antibakterielle Wirkung insbesondere im grampositiven Bereich aufweisen.

Bevorzugt sind die Verbindungen der Formel (I),
in welcher
X¹ für Fluor oder Chlor,
X² für Wasserstoff, Amino, Alkylamino mit 1 bis 2 Kohlenstoffatomen, Dimethylamino, Hydroxy, Methoxy, Mercapto, Methylthio, Fluor, Methyl,
B¹ für Alkyl mit 1 bis 3 Kohlenstoffatomen, Alkenyl mit 2 bis 3 Kohlenstoffatomen, Cycloalkyl mit 3 bis 5 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
B² für Wasserstoff, Alkyl mit 1 bis 3 Kohlenstoffatomen oder (5-Methyl-2-oxo-1,3-dioxol-4-yl)methyl,
B³ für einen Rest der oben beschriebenen Struktur stehen, worin
R¹, R³, R⁴, R⁵, R⁷ und R⁸ gleich oder verschieden sind und jeweils für Wasserstoff oder Methyl, das gegebenenfalls durch Halogen substituiert sein kann, C₁-C₃₋Alkoxycarbonyl stehen,
R⁴ zusätzlich für Halogen, wie Fluor, Chlor oder Brom stehen kann,
R⁸ für Wasserstoff, C₁-C₃-Alkyl, Benzyl, Phenyl, Acetyl, Benzoyl, C₁-C₄-Alkoxycarbonyl steht,
R¹', R²', R³', R⁵', R⁷' und R⁸' gleich oder verschieden sind und jeweils für Wasserstoff oder Methyl stehen,
R⁴' für Halogen wie Chlor oder Brom steht oder dann für Wasserstoff steht, wenn
R²' und R³' zusammen eine C₂-C₄-Alkylenbrücke oder eine CH₂-S-CH₂-Brücke bedeuten, die gegebenenfalls durch Hydroxy oder Methyl ein- oder zweifach substituiert sein kann,
A für N oder C-R⁹ steht, worin
_{R}9 für H, Halogen oder Methoxy steht

und deren pharmazeutisch verwendbaren Hydrate und Säureadditionssalze sowie die Alkali- und Erdalkalisalze der zugrundeliegenden Carbonsäuren.

Besonders bevorzugt sind die Verbindungen der Formel (I),
in welcher
X¹ für Fluor,
X² für Wasserstoff, Amino, Fluor, Methyl,
B¹ für Alkyl mit 1 bis 2 Kohlenstoffatomen, Vinyl, Cyclopropyl, 2-Hydroxyethyl, 2-Fluorethyl, Methoxy Methylamino, 4-Fluorphenyl, 2,4-Difluorphenyl,
B² für Wasserstoff, Alkyl mit 1 bis 2 Kohlenstoffatomen,
B³ für einen Rest der oben beschriebenen Struktur steht,
   worin
R¹, R³; R⁴, R⁵, R⁷ und R⁸ gleich oder verschieden sind und jeweils für Wasserstoff oder Methyl stehen, das gegebenenfalls durch Fluor substituiert sein kann,
R⁴ zusätzlich für Fluor, Chlor oder Brom stehen kann,
R⁶ für Wasserstoff, Methyl, Acetyl, C₁-C₄-Alkoxycarbonyl steht,
R¹', R²', R³', R⁵', R⁷' und R⁸' gleich oder verschieden sind und jeweils für Wasserstoff oder Methyl stehen,
R⁴' für Chlor oder Brom steht oder dann für Wasserstoff steht, wenn
R²' und R³' zusammen eine C₂-C₄-Alkylenbrücke oder eine CH₂-S-CH₂-Brücke bedeuten, die gegebenenfalls durch Hydroxy oder Methyl ein- oder zweifach substituiert sein kann,
A für N oder C-R⁹ steht, worin
_{R}9 für H, Halogen oder Methoxy steht

und deren pharmazeutisch verwendbaren Hydrate und Säureadditionssalze sowie die Alkalisalze der zugrundeliegenden Carbonsäuren

Weiterhin wurde gefunden, daß man die Verbindungen der Formel (I) erhält, wenn man Verbindungen der Formel (II) 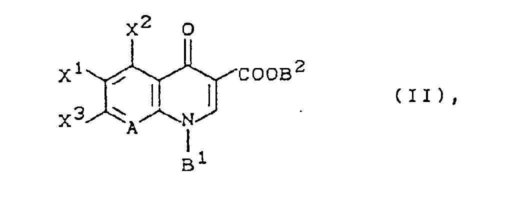 in welcher
A, B¹, B², X¹ und X² die oben angegebene Bedeutung haben und X³ für Halogen, insbesondere Fluor oder Chlor steht, mit Verbindungen der Formel (III) 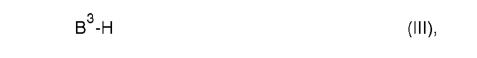 in welcher
B³ die oben angegebene Bedeutung hat,

gegebenenfalls in Gegenwart von Säurefängern umsetzt, und gegebenenfalls in B³ enthaltene Schutzgruppen abspaltet (Methode A).

Erfindungsgemäße Verbindungen der Formel (I)
in welcher
X¹, B¹, B², B³ und A die oben angegebene Bedeutung haben und
X² für Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen oder Arylthio steht,
   können auch erhalten werden, indem man eine Verbindung der Formel (IV) 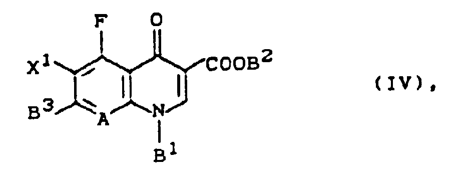 in welcher
mit Verbindungen der Formel (V) 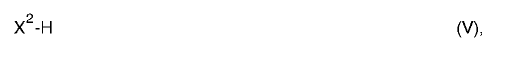 in welcher
X² die oben angegebene Bedeutung hat, gegebenenfalls in Gegenwart von Säurefängern umsetzt (Methode B).

Verwendet man beispielsweise 1-Cyclopropyl-6,7,8-trifluor-1,4-dihydro-4-oxo-3-chinolincarbonsäure und 1,4-Diazatricyclo[6.2.0.0^{2.6}]decan als Ausgangsstoffe, so kann der Reaktionsverlauf durch folgendes Formelschema wiedergegeben werden:

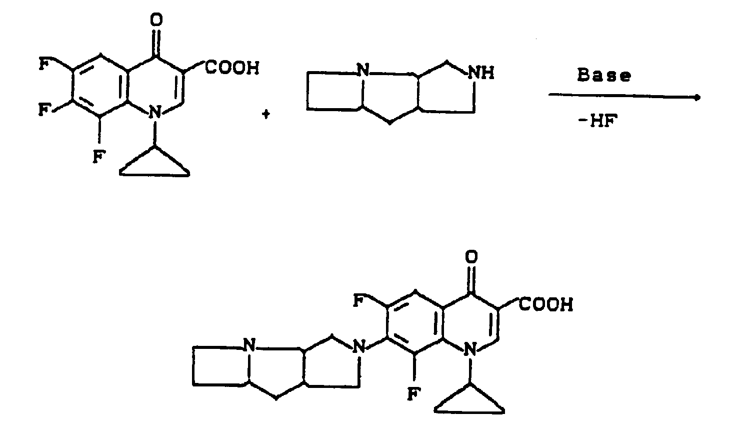

Verwendet man beispielsweise 7-Chlor-6-fluor-1-(2,4-difluorphenyl)-1,4-dihydro-4-oxo-1,8-naphthyridin-3-carbonsäure und 7-tert.-Butoxycarbonyl-5-methyl-2,7-diazabicyclo[3.3.0]octan als Ausgangsstoffe, so kann der Reaktionsverlauf durch das folgende Formelschema wiedergegeben werden:

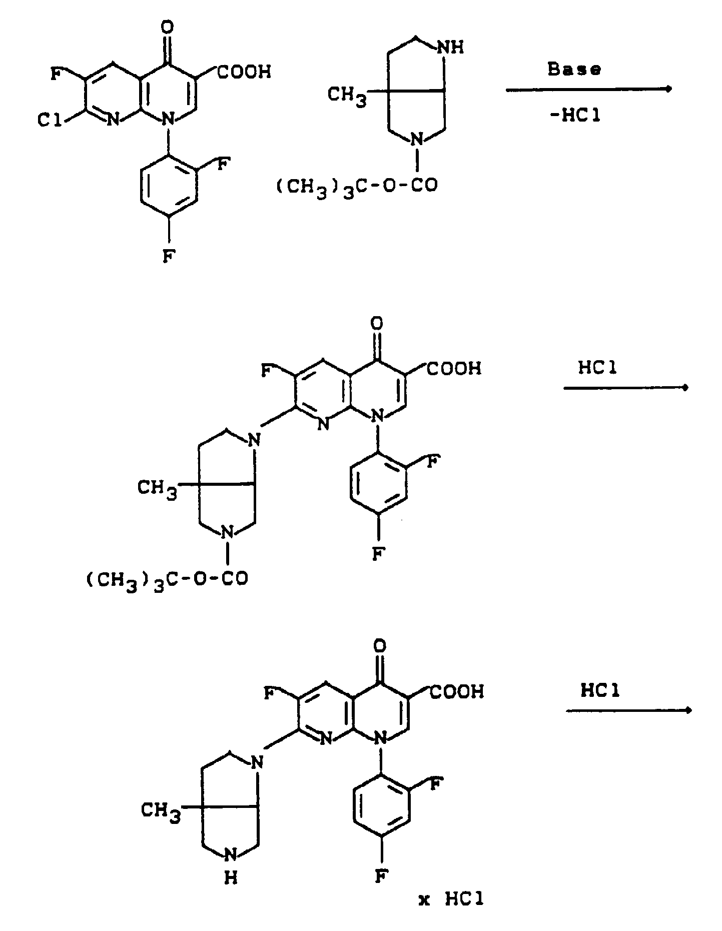

Verwendet man beispielsweise 1-Cyclopropyl-5;6,8-trifluor-1;4-dihydro-7-(7-methyl-2,7-diazabicyclo[3.3.0]oct-2-yl)-4-oxo-3-chinolincarbonsäure und Ammoniak als Ausgangsstoffe, so kann der Reaktionsablauf durch folgendes Formelschema wiedergegeben werden:

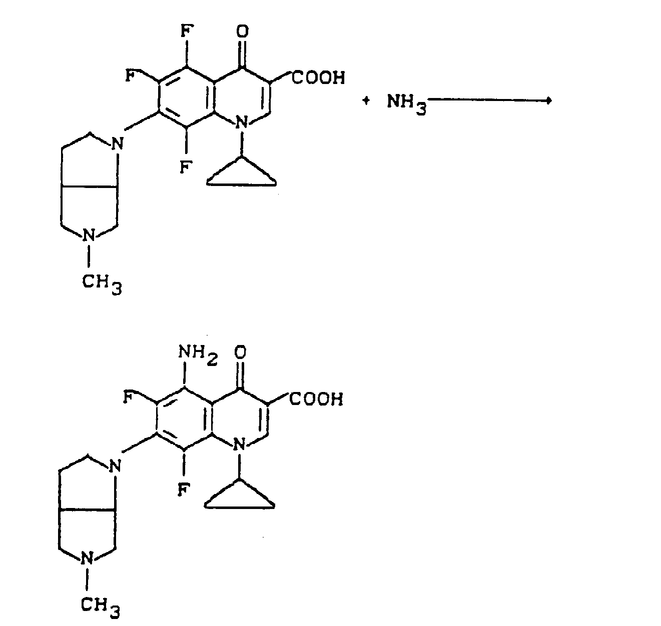

Die als Au^{g}an^{g}sstoffe verwendeten Verbindungen der Formel (II) sind bekannt oder können nach bekannten Methoden hergestellt werden. Als Beispiele seien genannt:
7-Chlor-1-cyclopropyl-6-fluor-1,4-dihydro-4-oxo-3-chinolincarbonsäure (DE-A-3 142 854),
1 -Cyclopropyl-6,7-difluor-1,4-dihydro-4-oxo-3-chinolincarbonsäure (EP-A-113 091),
6-Chlor-1-cyclopropyl-7,8-difluor-1,4-dihydro-4-oxo-3-chinolincarbonsäure (DE-A-3 420 743),
8-Chlor-1-cyclopropyl-6,7-difluor-1,4-dihydro-4-oxo-3-chinolincarbonsäure (DE-A-3 420 743),
1-Cyclopropyl-6,7,8-trifluor-1,4-dihydro-4-oxo-3-chinolincarbonsäure (DE-A-3 318 145),
6,8-Dichlor-1-cyclopropyl-7-fluor-1,4-dihydro-4-oxo-3-chinolincarbonsäure (DE-A-3 420 743),
1-Cyclopropyl-6,7-difluor-1,4-dihydro-8-methyl-4-oxo-3-chinolincarbonsaure,
1-Cyclopropyl-7-chlor-6-fluor-1,4-dihydro-8-nitro-4-oxo-3-chinolincarbonsäure,
6,7-Difluor-1-ethyl-1,4-dihydro-4-oxo-3-chinolincarbonsäure,
7-Chlor-6-fluor-1-ethyl-1,4-dihydro-4-oxo-3-chinolincarbonsäure,
7-Chlor-6-fluor-1,4-dihydro-1-(2-hydroxyethyl)-4-oxo-3-chinolincarbonsäure,
6,7-Difluor-1-(2-fluorethyl)-1,4-dihydro-4-oxo-3-chinolincarbonsäure,
7-Chlor-6-fluor-1,4-dihydro-1-methoxy-4-oxo-3-chinolincarbonsäure,
7-Chlor-6-fluor-1,4-dihydro-1-methylamino-4-oxo-3-chinolincarbonsäure,
6,7-Difluor-1,4-dihydro-4-oxo-1 -phonyl-3-chinolincarbonsäure,
7-Chlor-1-cyclopropyl-6-fluor-1,4-dihydro-4-oxo-1,8-naphthyridin-3-carbonsäure,
6,7-Dichlor-1-cyclopropyl-1,4-dihydro-4-oxo-1,8-naphthyridin-3-carbonsäure,
1-Cyclopropyl-6,7,8-trifluor-1,4-dihydro-4-oxo-3-chinolincarbonsäureethylester (EP-A-3 318 145),
7-Chlor-6-fluor-1-phenyl-1,4-dihydro-4-oxo-1,8-naphthyridin-3-carbonsäure (EP-A-153 580),
7-Chlor-6-fluor-1-(4-fluorphenyl)-1,4-dihydro-4-oxo-1,8-naphthyridin-3-carbonsäure (EP-A-153 580),
6,7,8-Trifluor-1,4-dihydro-1-methylamino-4-oxo-3-chinolincarbonsäure (DE-A-3 409 922),
7-Chlor-6-fluor-1,4-dihydro-8-nitro-4-oxo-1-phenyl-3-chinolincarbonsäure,
7-Chlor-6-fluor-1-(4-fluorphenyl)-1,4-dihydro-8-nitro-4-oxo-3-chinolincarbonsäure,
6,7-Difluor-1-(4-fluorphenyl)-1,4-dihydro-8-methyl-4-oxo-3-chinolincarbonsäure,
6-Chlor-7-fluor-1-(4-fluorphenyl)-1,4-dihydro-4-oxo-3-chinolincarbonsäure (EP-A-131 839),
6-Chlor-7-fluor-1-(2,4-difluorphenyl)-1,4-dihydro-4-oxo-3-chinolincarbonsäure (EP-A-131 839),
6,7,8-Trifluor-1-(4-fluorphenyl)-1,4-dihydro-4-oxo-3-chinolincarbonsäure (EP-A-154 780),
6,7,8-Trifluor-1-(2,4-difluorphonyl)-1,4-dihydro-4-oxo-3-chinolincarbonsäure (EP-A-154 780),
6,7,8-Trifluor-1,4-dihydro-4-oxo-1-phenyl-3-chinolincarbonsäure (EP-A-154 780),
7-Chlor-1-ethyl-6-fluor-1,4-dihydro-4-oxo-1,9-naphthyridin-3-carbonsäure,
6,7-Difluor-1,4-dihydro-4-oxo-1-vinyl-3-chinolincarbonsäure,
1-Cyclopropyl-5,6,7,8-tetrafluor-1,4-dihydro-4-oxo-3-chinolincarbonsäure,
5-Amino-1-cyclopropyl-6,7;8-trifluor-1,4-dihydro-4-oxo-3-chinolincarbonsäure,
1-Cyclopropyl-6,7,8-trifluor-1,4-dihydro-5-hydroxy-4-oxo-3-chinolincarbonsäure,
1-Cyclopropyl-6,7-difluor-1,4-dihydro-8-methoxy-4-oxo-3-chinolincarbonsäure.

Die als Ausgangsverbindungen verwendeten Verbindungen der Formel (III) können dadurch hergestellt werden, daß man ungesättigte Carbonylverbindungen der Formel (VI) mit Aminosäurederivaten der Formel (VII) in einer intramolekularen 1,3-dipolaren Cycloaddition umsetzt, 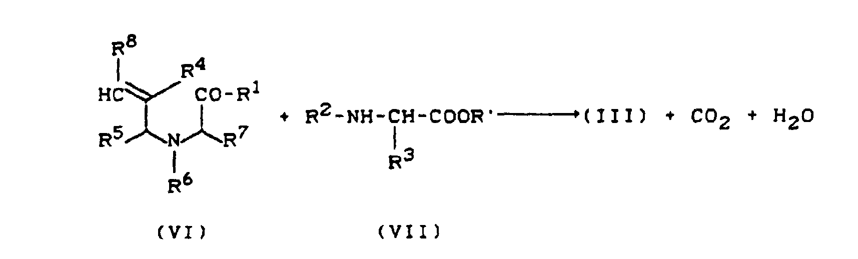 worin
R' für H oder C₁-C₂-Alkyl steht und

wobei R^{l -} R⁸ die oben angegebene Bedeutung haben. Anstelle von R^{1 -} R⁸ können auch die oben für R¹'-R⁸' angegebenen Bedeutungen stehen. Anschließend können die Substituenten R² oder R⁶, welche eine Schutzgruppenfunktion haben, abgespalten werden.

Die ungesättigten Carbonylverbindungen der Formel (VI) können nach folgenden Verfahren hergestellt werden:
1. Ausgehend von käuflichem Aminoacetaldehyddimethylacetal wird die Aminogruppe acyliert, das Amid in Gegenwart von starken Basen mit Allylhalogeniden alkyliert und das Acetal sauer gespalten.
2. Geht man von käuflichen oder bekannten (EP-A-249 530) a-Oxoaldehydmonoacetalen aus, kann man diese mit Allylaminen reduktiv aminieren. Nach Einführen einer (Schutz-)gruppe R⁶ durch Alkylieren oder Acylieren wird die Acetalgruppe sauer verseift 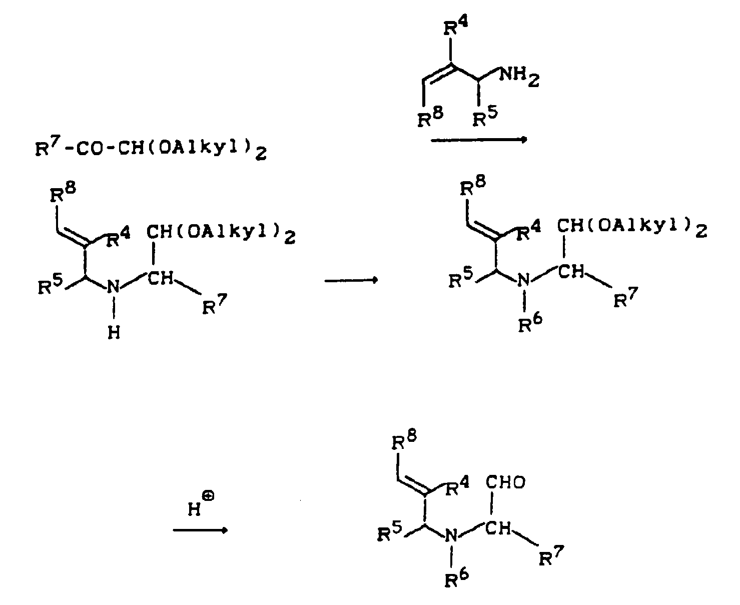
3. Ungesättigte Aldehyde oder Ketone können mit Aminoacetaldehyddimethylacetal reduktiv aminiert werden. Nach Einführen der Gruppe R⁶ durch Alkylieren oder Acylieren kann die Acetalgruppe sauer gespalten werden. 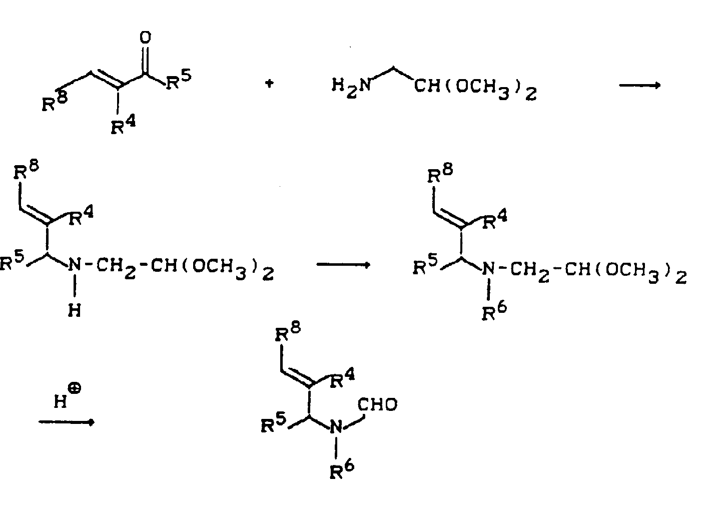
4. N-Allylaminoalkohole erhält man
   a) durch Ringöffnung von Epoxiden mit Allylaminen (J. pharm. Soc. Japan 73, 1330 (1953)),
   b) durch Alkylieren von substituierten Ethanolaminen mit Allylhalogeniden (J. Am. Chem. Soc. 64, 1692 (1942); 72, 3536 (1950)),
   c) durch reduktive Aminierung von ungesättigten Aldehyden oder Ketonen mit substituierten Ethanolaminen.
      Nach Einführung der Gruppe R⁸ durch Alkylieren oder Acylieren wird die Alkoholfunktion mit geeigneten Oxidationsmitteln zu Verbindungen der Formel (VI) oxidiert. 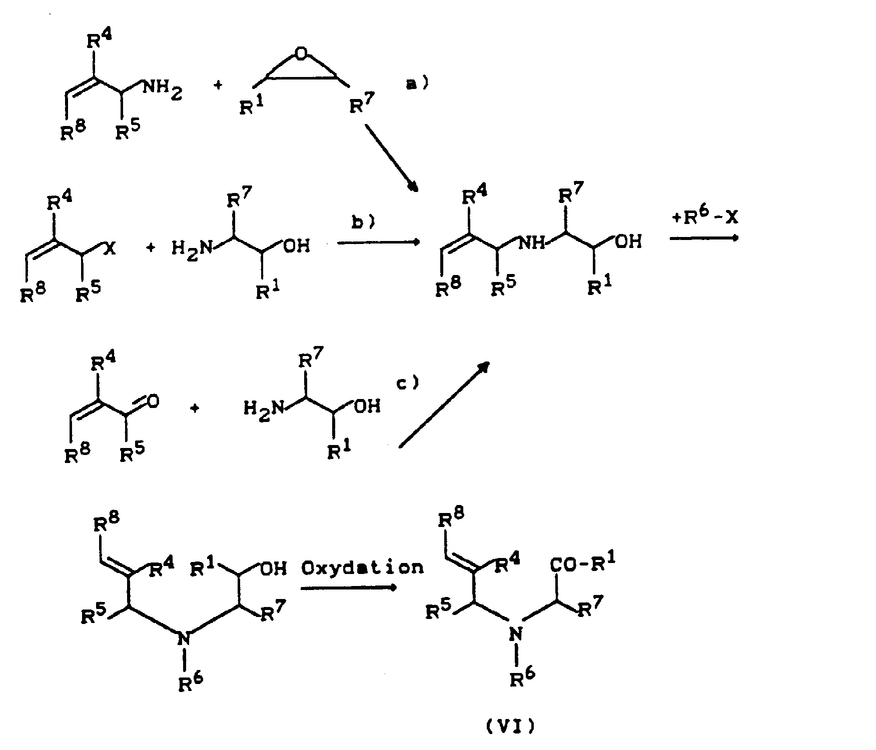
5. Enantiomerenreine Edukte der Formel (VI) erhält man durch Alkylieren von N-acylierten Aminosäureestern mit Allylhalogeniden in Gegenwart starker Basen. Die Esterfunktion kann mit geeigneten Reduktionsmitteln
   a) zur Aldehydfunktion reduziert oder
   b) zum Alkohol reduziert und dann mit geeigneten Oxidationsmitteln zur Aldehydfunktion oxidiert werden

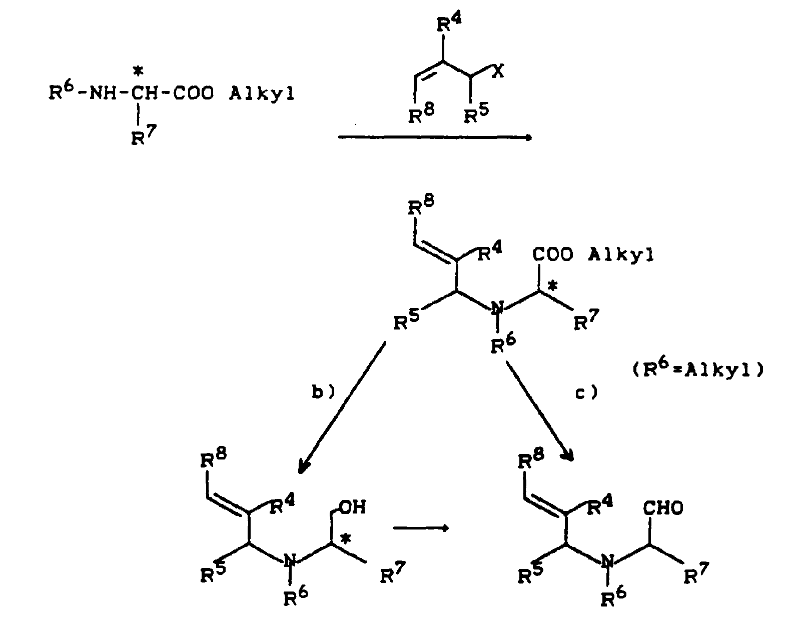

Die Aminosäuren der Formel (VII) sind literaturbekannt und größtenteils kommerziell erhältlich.

Die Umsetzung von (VI) mit (VII) wird in einem Lösungsmittel durchgeführt. Es können Kohlenwasserstoffe wie Benzol, Toluol, Xylole oder Tetralin, Ether wie Dioxan, Dibutylether, Dimethoxyethan, Diethylenglykoldimethylether, Diethylenglykoldiethylether, Alkohole wie Butanol, Pentanol, Ethylenglykol, Ethylenglykolmonomethylether und Ethylenglykolmonoethylether, und dipolar aprotische Lösungsmittel wie Dimethylformamid, Dimethylsulfoxid, N-Methylpyrrolidon und Sulfolan verwendet werden, Besonders bevorzugt sind Toluol, Xylole und Dimethylformamid.

Die Reaktionstemperatur kann in einem größeren Bereich variiert werden. Im allgemeinen führt man die Umsetzungen zwischen 20°C und 200°C, vorzugsweise zwischen 80°C und 150°C durch.

Die Umsetzung kann bei Normaldruck, aber auch bei erhöhtem Druck durchgeführt werden. Allgemein arbeitet man bei Drucken zwischen etwa 1 bar und 100 bar, vorzugsweise zwischen etwa 1 bar und 10 bar.

Bei der Durchführung des erfindungsgemäßen Verfahrens setzt man auf 1 Mol ungesättigter Carbonylverbindung (VI) 0,5 bis 6 Mol, vorzugsweise 0,5 bis 2 Mol Aminosäurederivat (VII) ein.

Die Reaktion kann so durchgeführt werden, daß die ungesättigte Carbonylverbindung zu einer Suspension oder Lösung des Aminosäurederivates (VII) in einem der angegebenen Lösungsmittel zugetropft wird. Man kann aber auch beide Komponenten in einem Lösungsmittel vorlegen und die Reaktion im angegebenen Temperaturbereich durchführen. Das bei der Reaktion freiwerdende Reaktionswasser kann mit dem Lösungsmittel als Azeotrop abdestilliert werden. Der Reaktionsverlauf ist durch die stattfindende C0₂-Entwicklung gut zu verfolgen. Die Aufarbeitung erfolgt, gegebenenfalls nach Abtrennen nicht umgesetzter Aminosäure (VII), durch Entfernen des Lösungsmittels und Destillation. Es ist auch möglich, die basischen Produkte mit einer Säure, wie zum Beispiel Salzsäure, aus dem organischen Lösungsmittel zu extrahieren, um neutrale Verunreinigungen abzutrennen.

In einem weiteren Schritt des erfindungsgemäßen Verfahrens können die Substituenten R² und R⁶, soweit sie Schutzgruppenfunktion besitzen, abgespalten werden.

Acylreste werden hydrolytisch entfernt. Zur Hydrolyse eignen sich starke Säuren oder starke Basen. Zur sauren Hydrolyse verwendet man bevorzugt wäßrige Salzsäure, Bromwasserstoffsäure oder Trifluoressigsäure. Die basische Hydrolyse führt man mit Alkalimetallhydroxiden oder Erdalkalimetallhydroxiden aus, bevorzugt sind Lithiumhydroxid, Natriumhydroxid, Kaliumhydroxid, Calciumhydroxid und Bariumhydroxid. Als Lösungsmittel dienen Wasser und Alkohole, bevorzugt sind Wasser, Ethanol oder Gemische aus diesen Lösungsmitteln. Die Hydrolyse kann bei Temperaturen zwischen 0° und 200°C, bevorzugt zwischen 20° und 140°C durchgeführt werden. Dabei arbeitet man bei Drucken zwischen etwa 1 bar und 100 bar, bevorzugt zwischen etwa 1 bar und 10 bar.

Sind die Reste R² oder R⁶ Benzylreste, so können diese Reste hydrogenolytisch abgespalten werden. Als Lösungsmittel können Wasser, Alkohole, Carbonsäuren, alkoholische Salzsäure, cyclische Ether oder deren Gemische verwendet werden. Als Katalysatoren verwendet man Palladium, sowohl als Schwamm als auch auf Trägern wie Aktivkohle, Calciumcarbonat oder Bariumsulfat, und Palladiumhydroxid auf Aktivkohle. Man arbeitet bei Temperaturen zwischen etwa 0° und 200°C und Wasserstoffdrucken von 1 bar bis 200 bar.

Das Verfahren beinhaltet darüber hinaus die Überführung der Reste R² und R⁶, sofern es sich um Acylreste handelt, in Alkylreste durch Reduktion. Die Reduktion kann sowohl katalytisch als auch mit Hydriden oder komplexen Hydriden der Elemente der dritten Hauptgruppe erfolgen. Bevorzugt erfolgt die Reduktion mit Diboran, Lithiumaluminiumhydrid und Natriumborhydrid, im letzten Fall unter Zusatz von Lewis-Säuren wie Titantetrachlorid, Aluminiumtrichlorid oder Bortrifluorid.

Die Reaktion erfolgt in inerten organischen Lösungsmitteln wie Ethern, zum Beispiel Diethylether, Dibutylether, Methyl-tert.-butylether, Tetrahydrofuran, Dioxan oder Ethylenglykoldimethylether oder Kohlenwasserstoffen wie Toluol oder Xylol. Die Temperaturen können zwischen etwa 0° und 200°C variiert werden. Zur Erzielung hoher Reaktionstemperaturen kann man bei Drucken bis zu 100 bar arbeiten.

Bevorzugt reduziert man mit Lithiumaluminiumhydrid oder Natriumborhydrid/Bortrifluorid-Etherat in Tetrahydrofuran oder Dioxan bei der Rückflußtemperatur des Lösungsmittels.

Die Umsetzung von (11) mit (111) gemäß Methode A des erfindungsgemäßen Verfahrens, bei der die Verbindungen (111) auch in Form ihrer Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.40] undec-7-en (DBU) oder überschüssiges Amin (III). Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Carbonsäure (11) 1 bis 15 Mol, vorzugsweise 1 bis 6 Mol der Verbindung (III) ein.

Freie Hydroxygruppen können während der Umsetzung durch eine geeignete Hydroxyschutzgruppe, zum Beispiel durch den Tetrahydropyranylrest, geschützt und nach Beendigung der Reaktion wieder freigesetzt werden (siehe J.F. W. McOmie, Protective Groups in Organic Chemistry (1973), Seite 104).

Freie Aminofunktionen können während der Umsetzung durch eine geeignete Aminoschutzgruppe, zum Beispiel durch den Ethoxycarbonyl- oder den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der organischen Chemie, Band E4, Seite 144 (1983); J.F.W. McOmie, Protective Groups in Organic Chemistry (1973), Seite 43).

Die Umsetzung von (IV) mit (V) gemäß Methode B des erfindungsgemäßen Verfahrens wird vorzugsweise in einem Verdünnungsmittel wie Dimethylsulfoxid, Dioxan, N,N-Dimethylformamid, N-Methylpyrrolidon, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden

Als Säurebinder können alle üblichen anorganischen und organischen Säurebindemittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo [2.2.2]octan (DABCO) oder 1,8-Diazabicyclo[5.4.0]undec-7-en (DBU).

Die Reaktionstemperaturen können in einem größeren Bereich variiert werden. Im allgemeinen arbeitet man zwischen etwa 70 und etwa 200°C, vorzugsweise zwischen 100 und 180°C.

Die Umsetzung kann bei Normaldruck, aber auch bei erhöhtem Druck durchgeführt werden. Im allgemeinen arbeitet man bei Drucken zwischen etwa 1 bar und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 50 Mol, vorzugsweise 1 bis 30 Mol der Verbindung (V) ein.

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20° bis 200°C, vorzugsweise etwa 60° bis 120°C umgesetzt. Das entstehende Reaktionswasser kann auch durch azeotrope Destillation mit Chloroform, Tetrachlormethan, Benzol oder Toluol entfernt werden.

Die Herstellung von Estern gelingt auch vorteilhaft durch Erhitzen der zugrundeliegenden Säure mit Dimethylformamiddialkylacetal in einem Lösungsmittel wie Dimethylformamid.

Die als Prodrug verwendeten (5-Methyl-2-oxo-1,3-dioxol-4-yl-methyl)-ester werden durch Umsetzung eines Alkalisalzes der zugrundeliegenden Carbonsäure mit 4-Brommethyl- oder4-Chlormethyl-5-methyl-1 ,3-dioxol-2-on in einem Lösungsmittel wie Dimethylformamid, Dimethylacetamid, N-Methylpyrrolidon, Dimethylsulfoxid oder Tetramethylharnstoff bei Temperaturen von etwa 0° bis 100°C, vorzugsweise 0° bis 50°C, erhalten.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium-oder Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können ebenfalls die in Tabelle 1 beispielhaft aufgeführten Verbindungen hergestellt werden. Die erfindungsgemäßen Verbindungen können sowohl als Diastereomerengemische als auch als diastereomerenreine oder enantiomerenreine Verbindungen vorliegen.

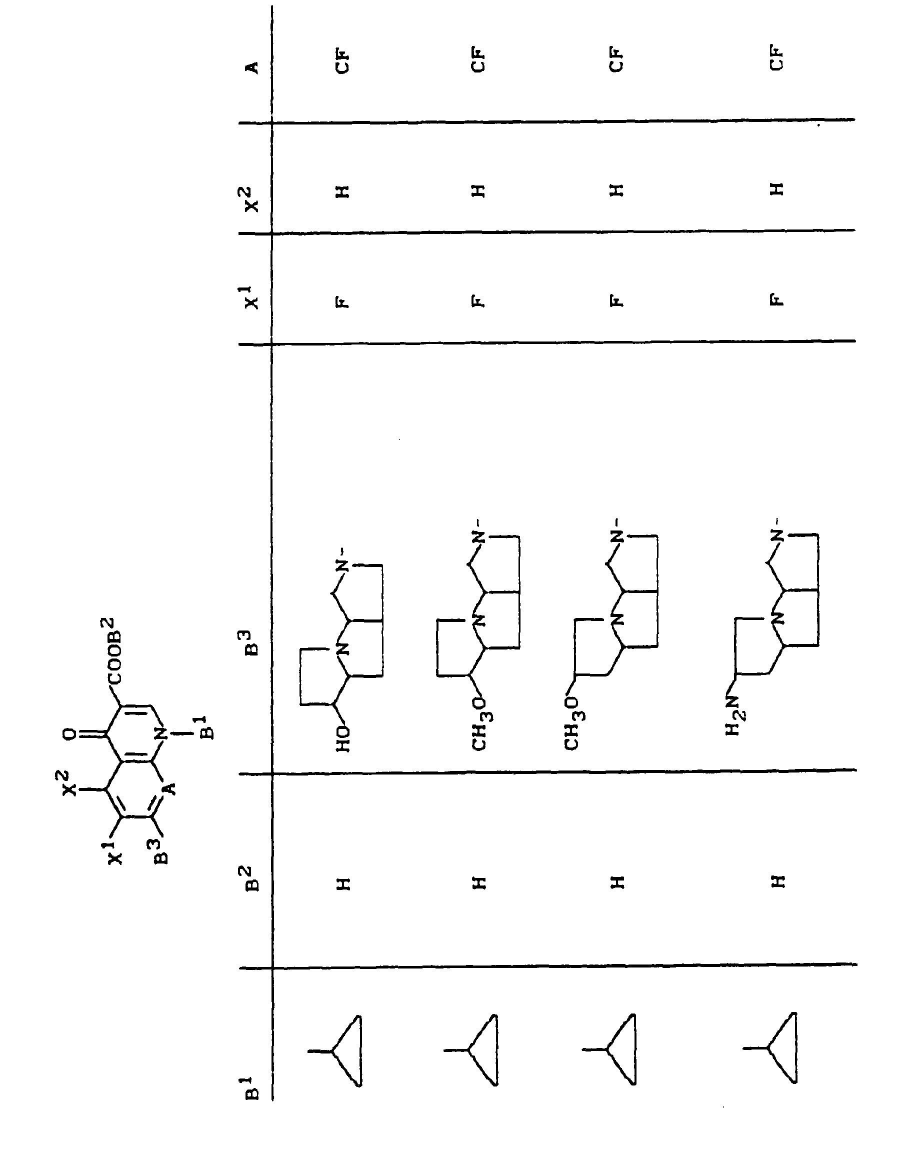

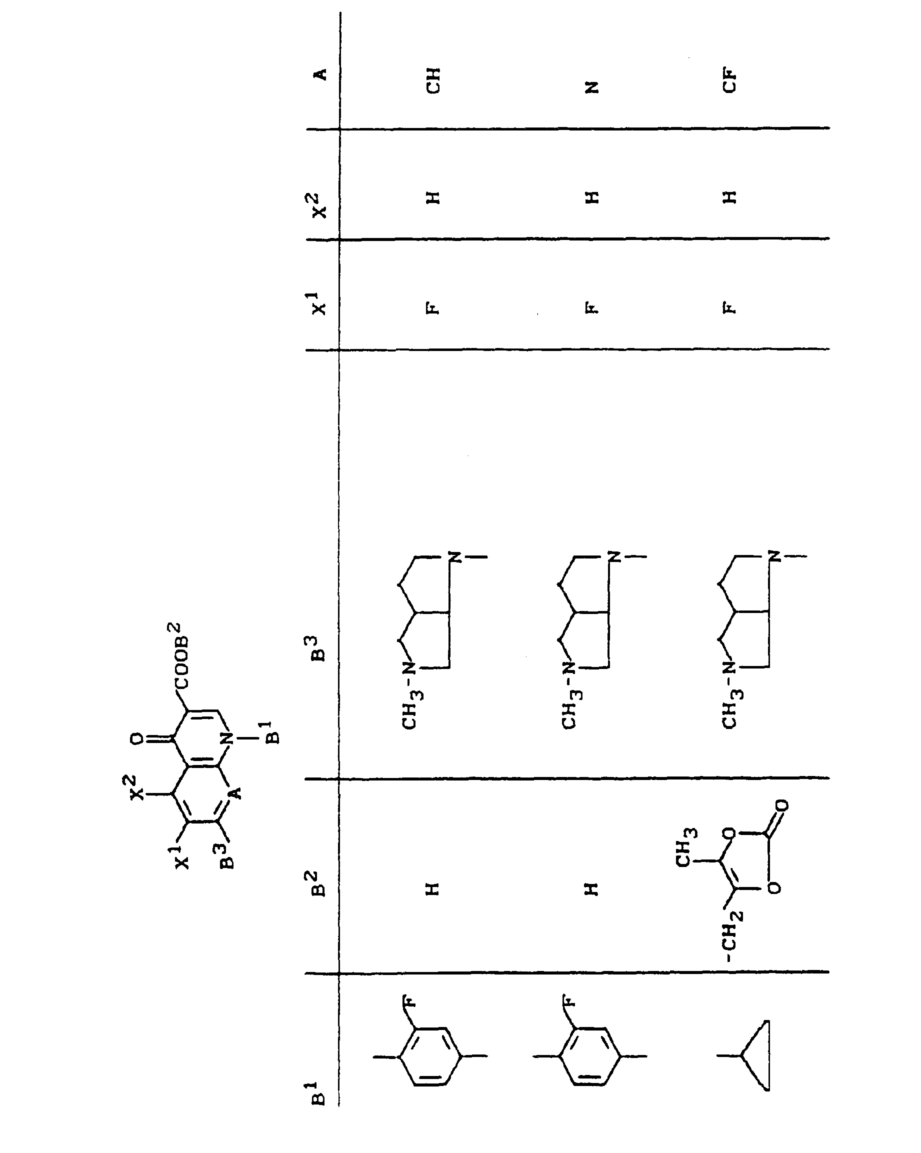

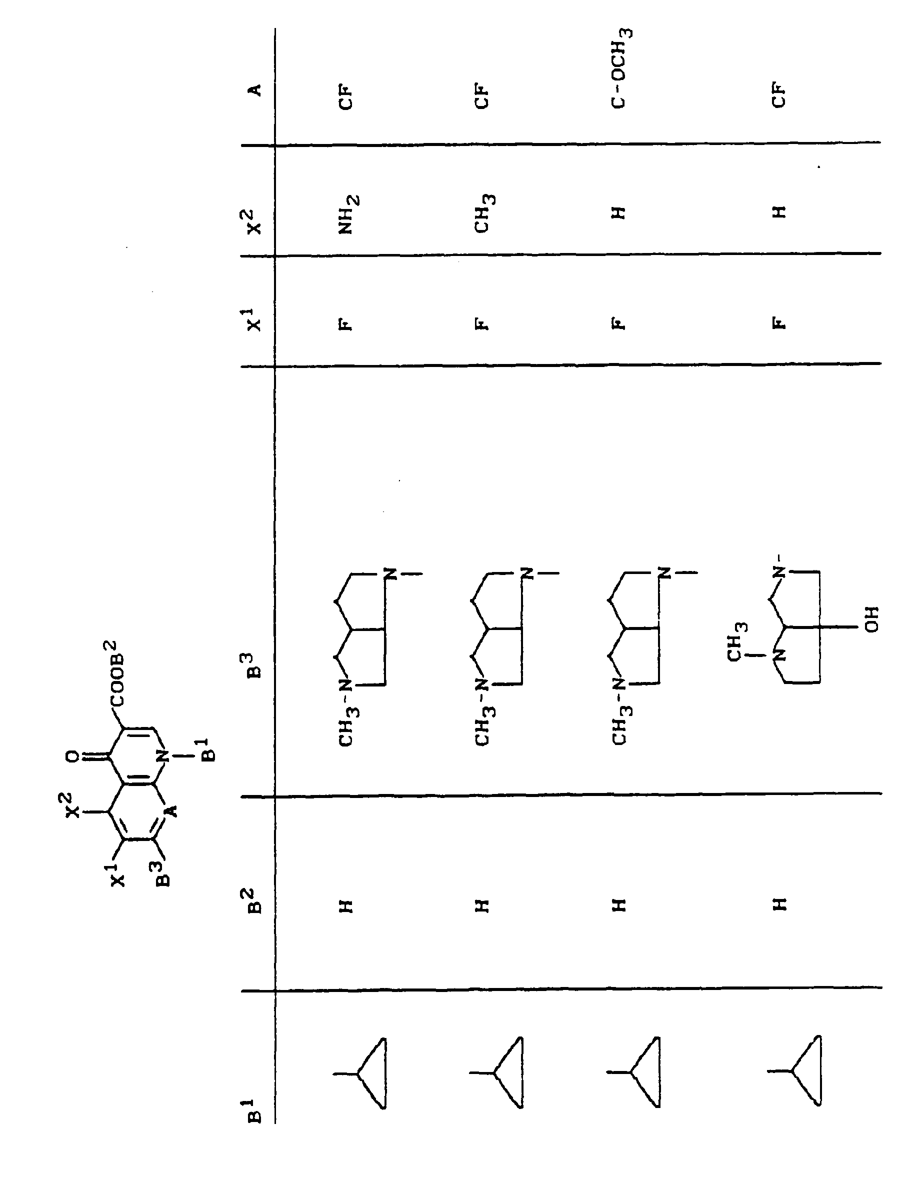

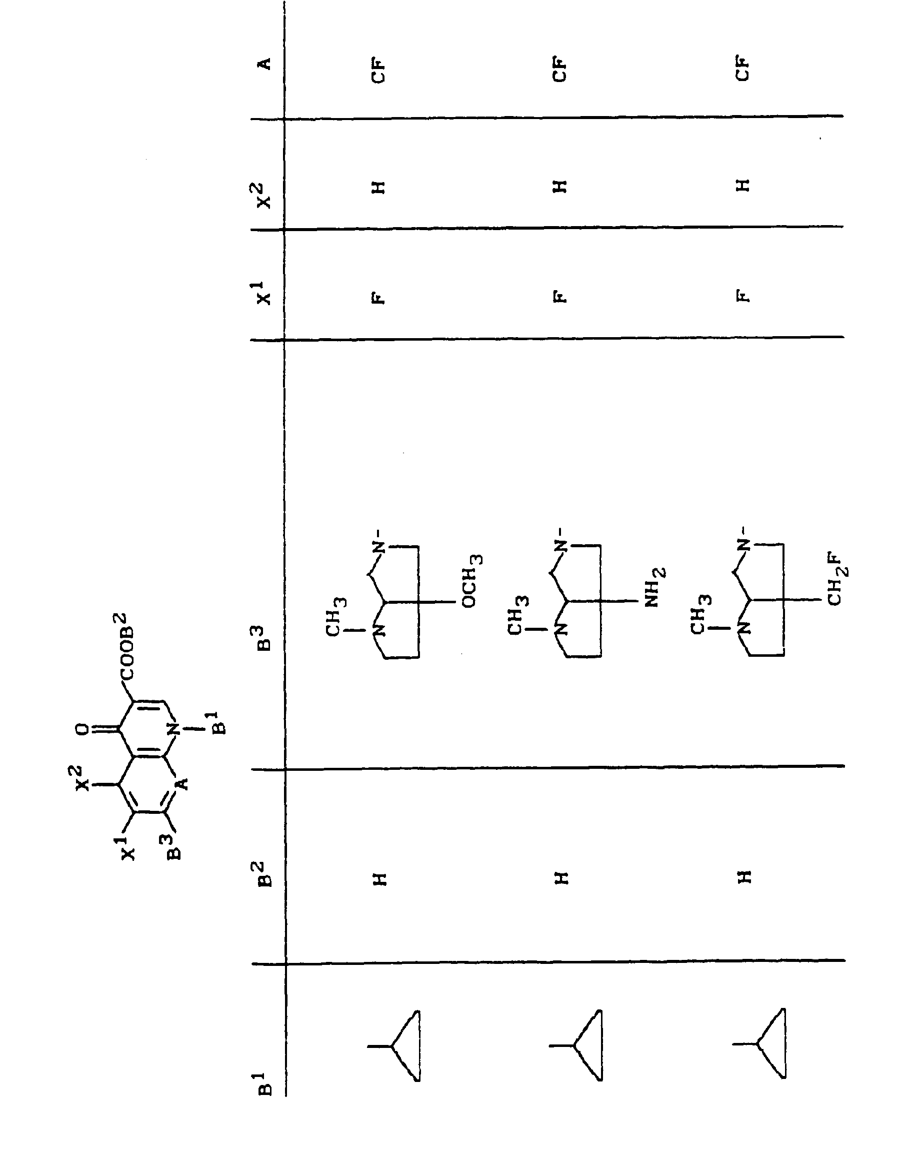

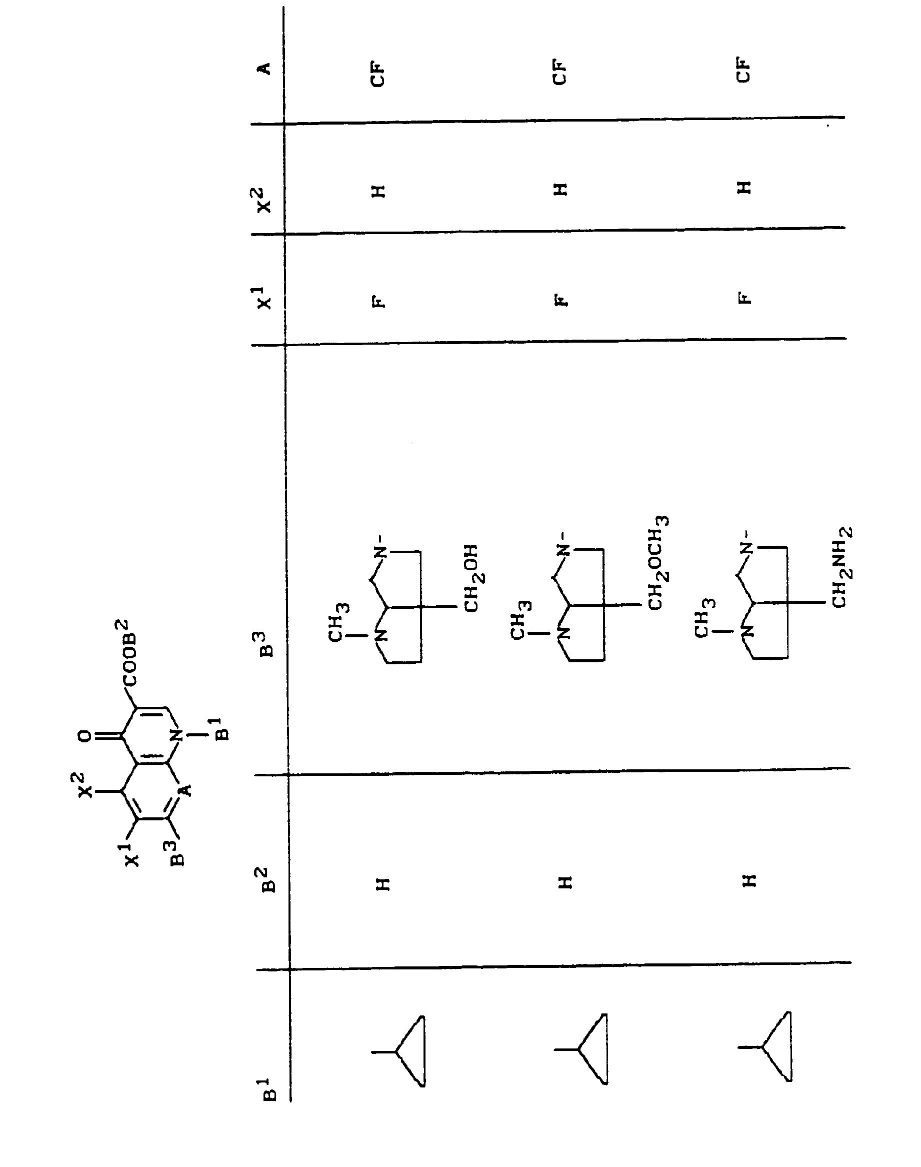

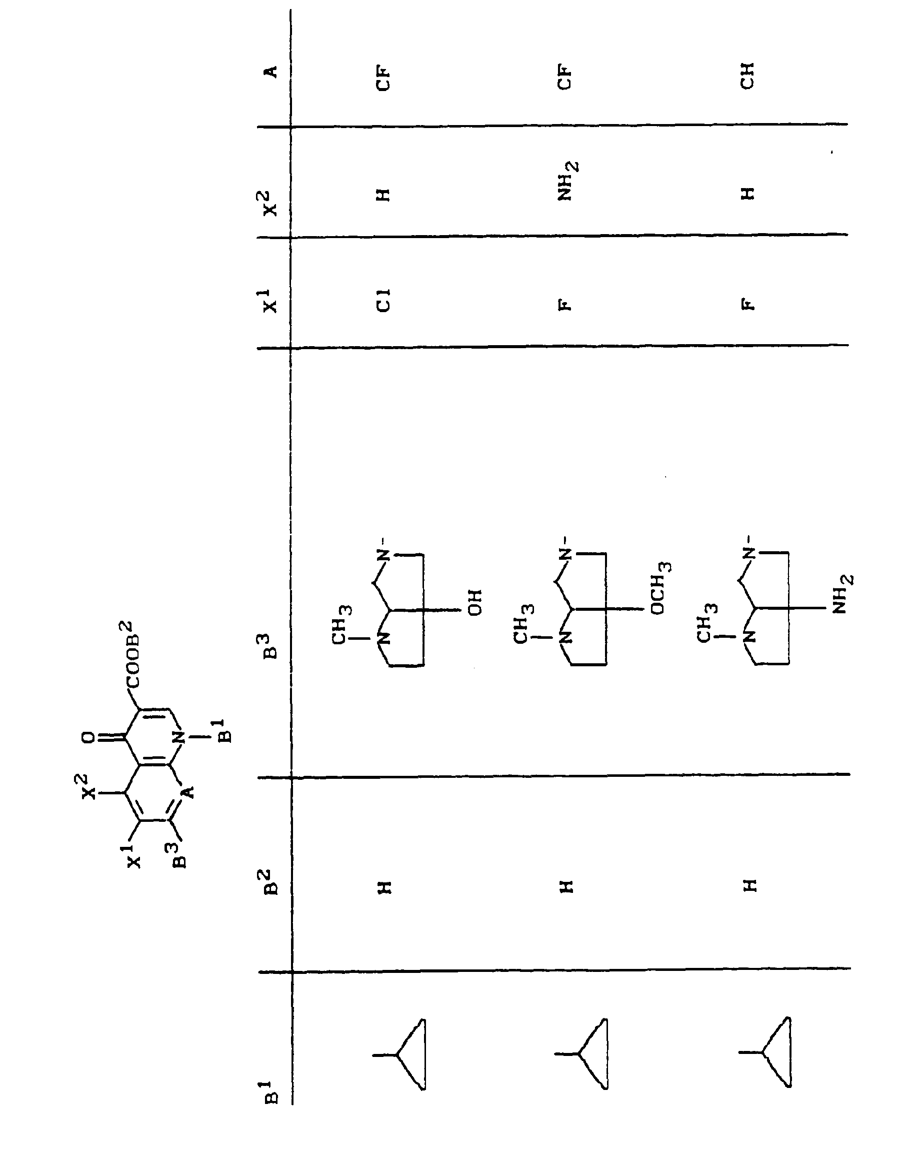

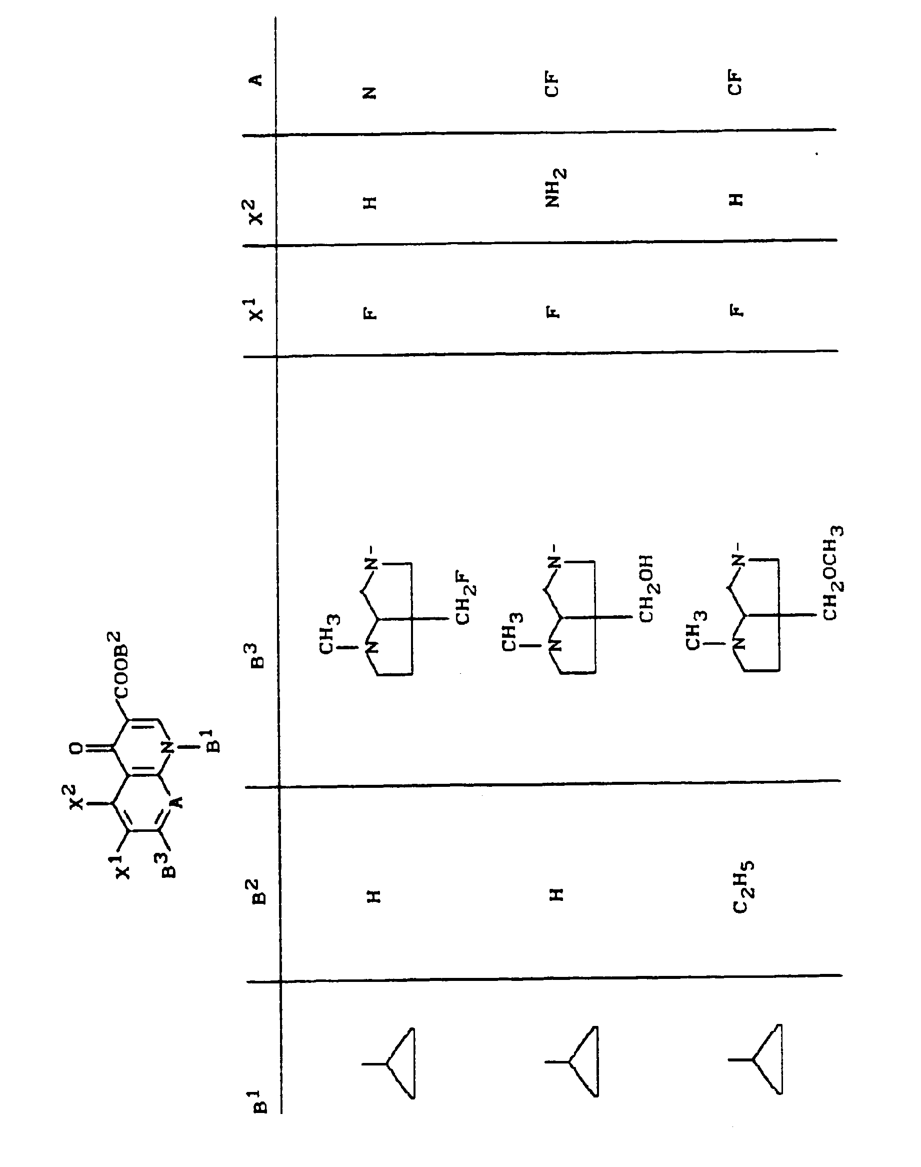

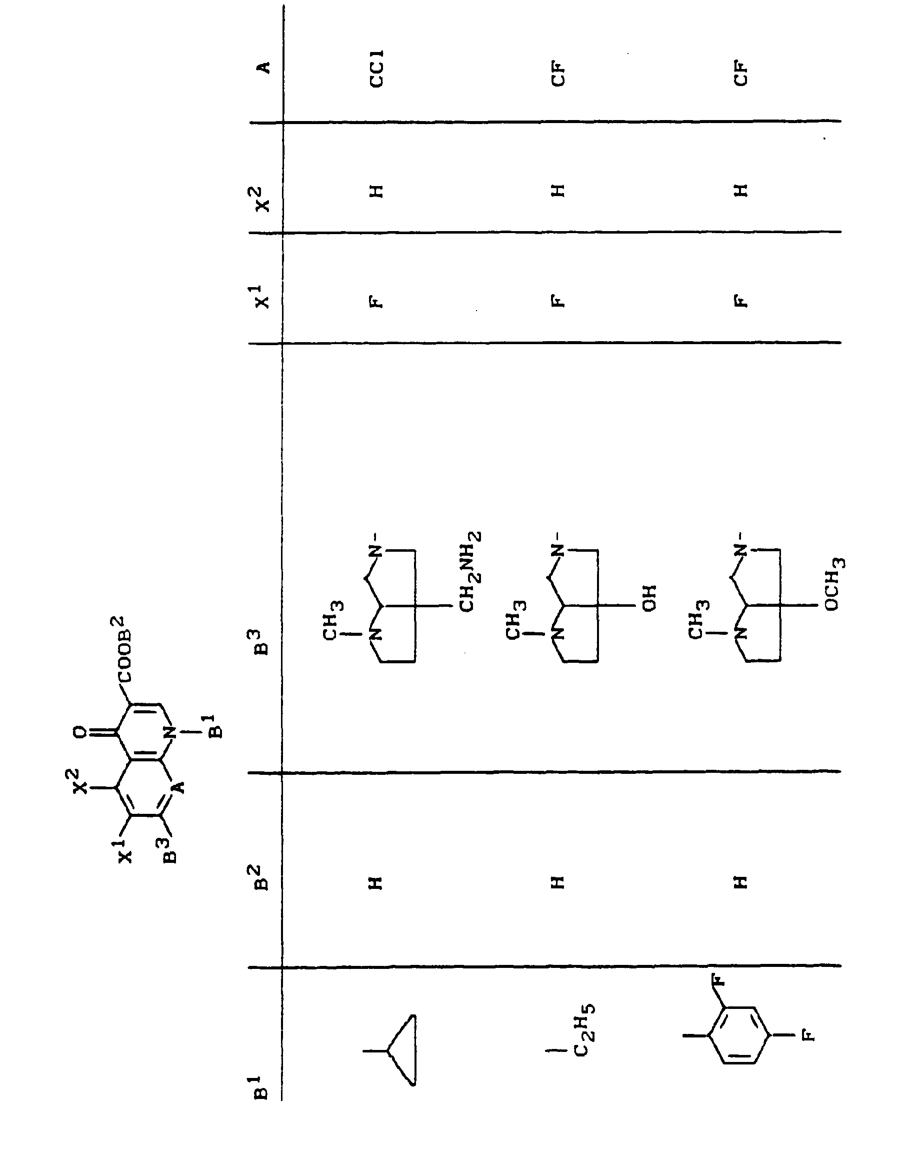

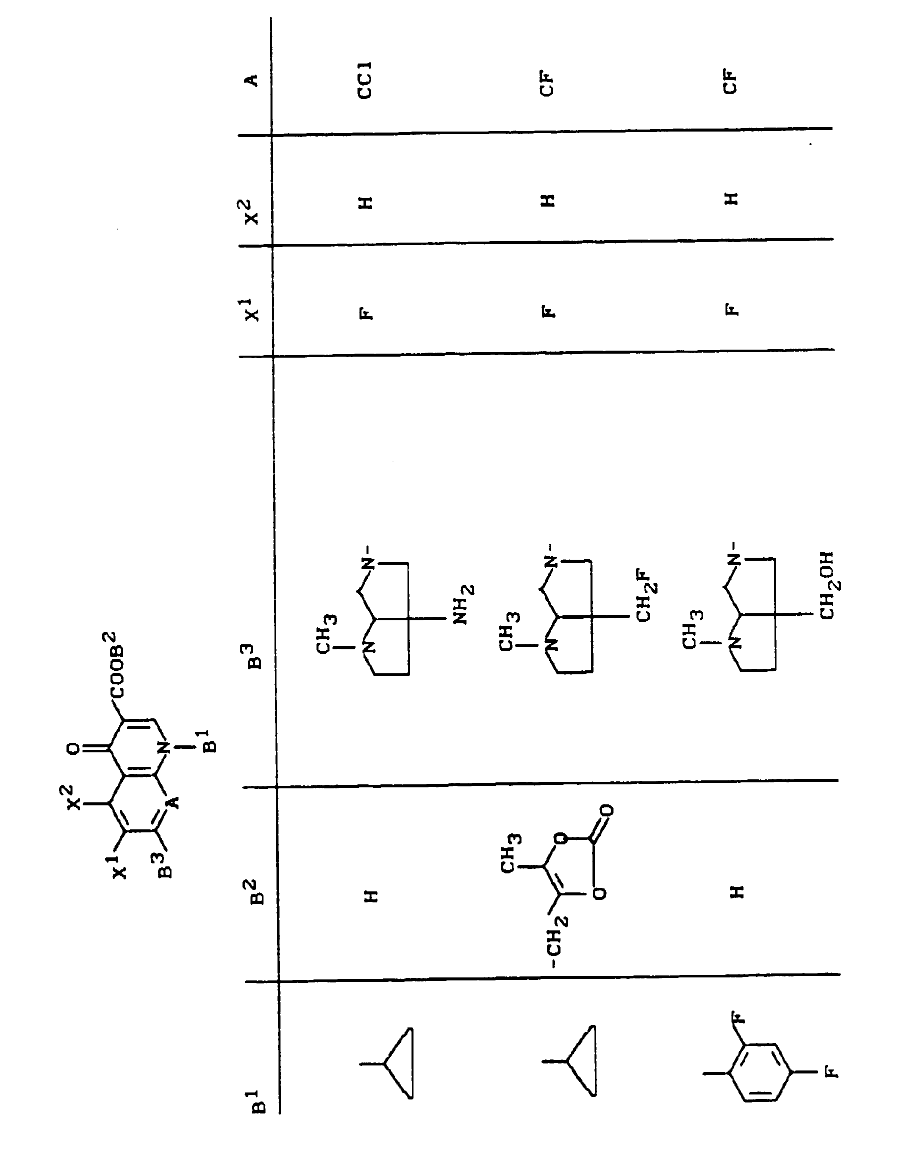

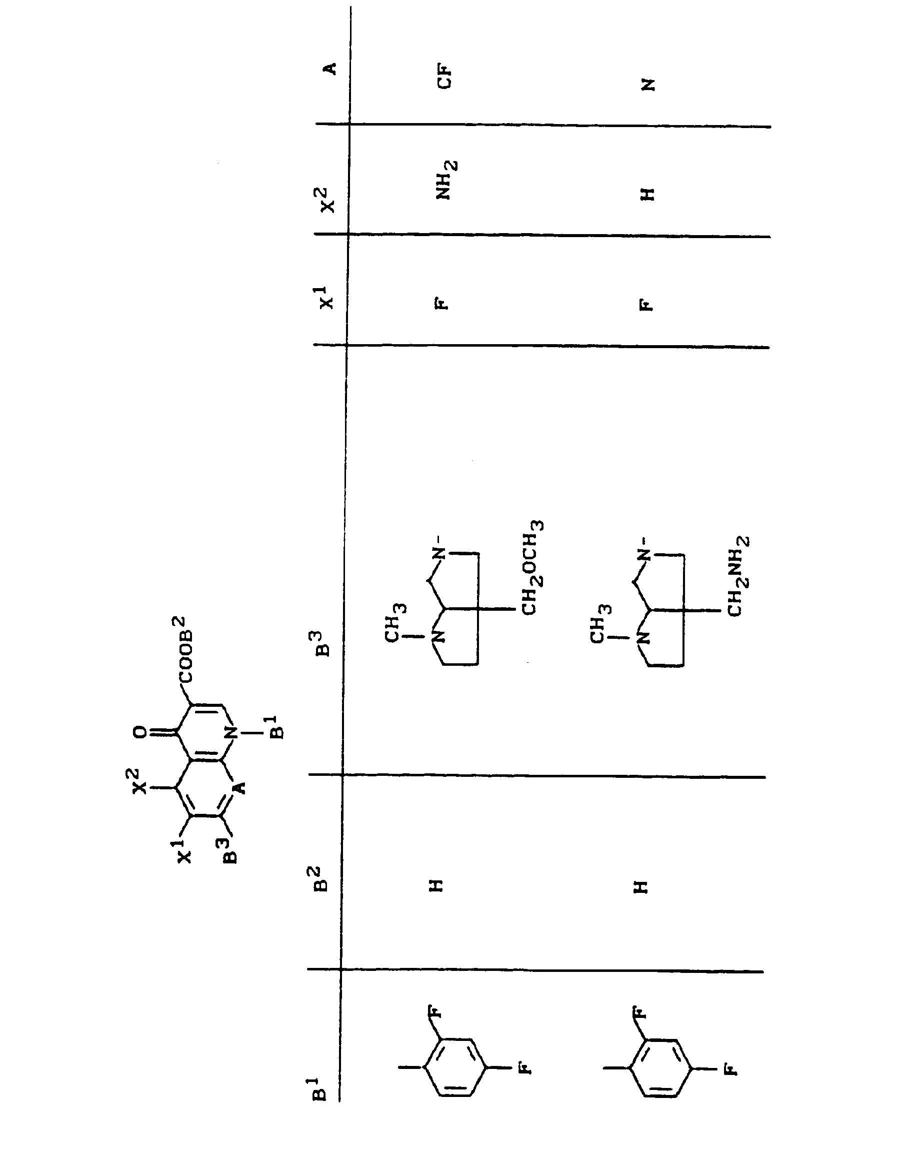

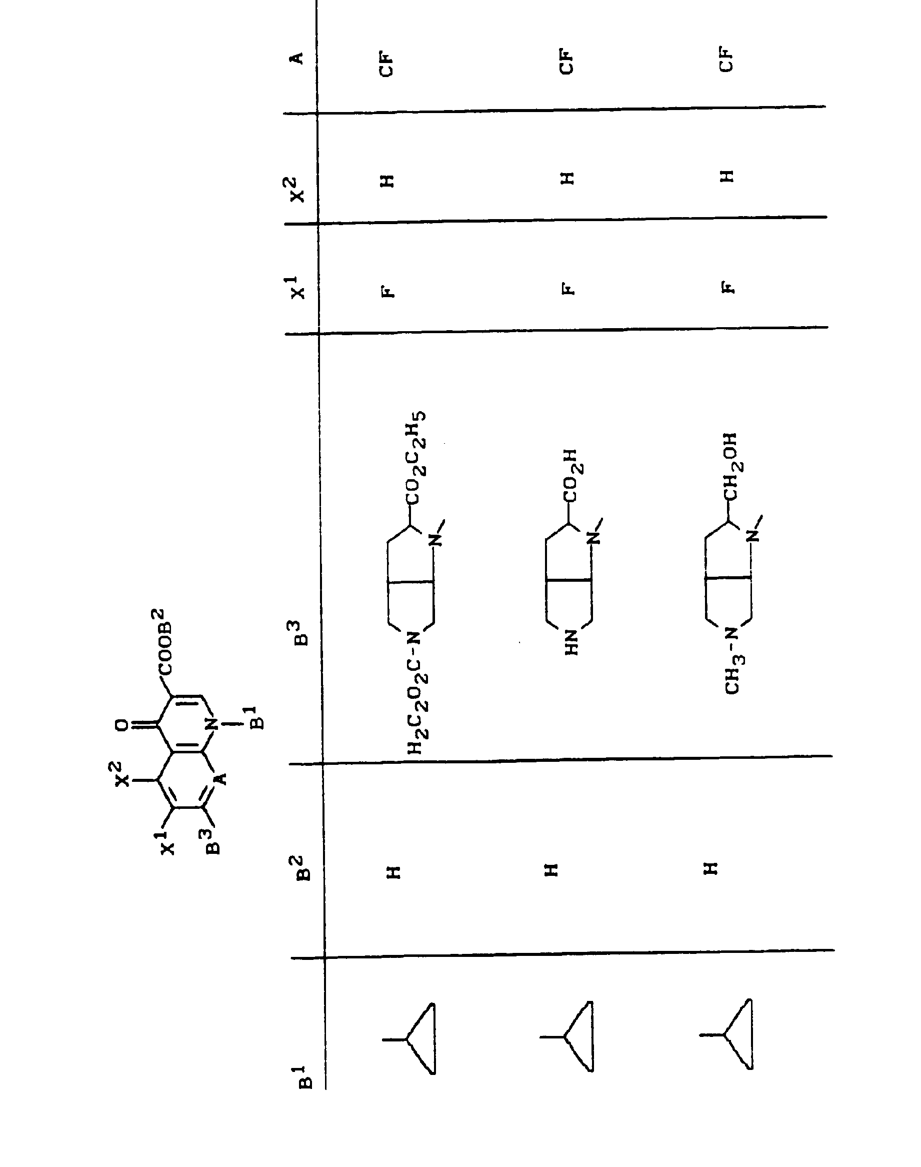

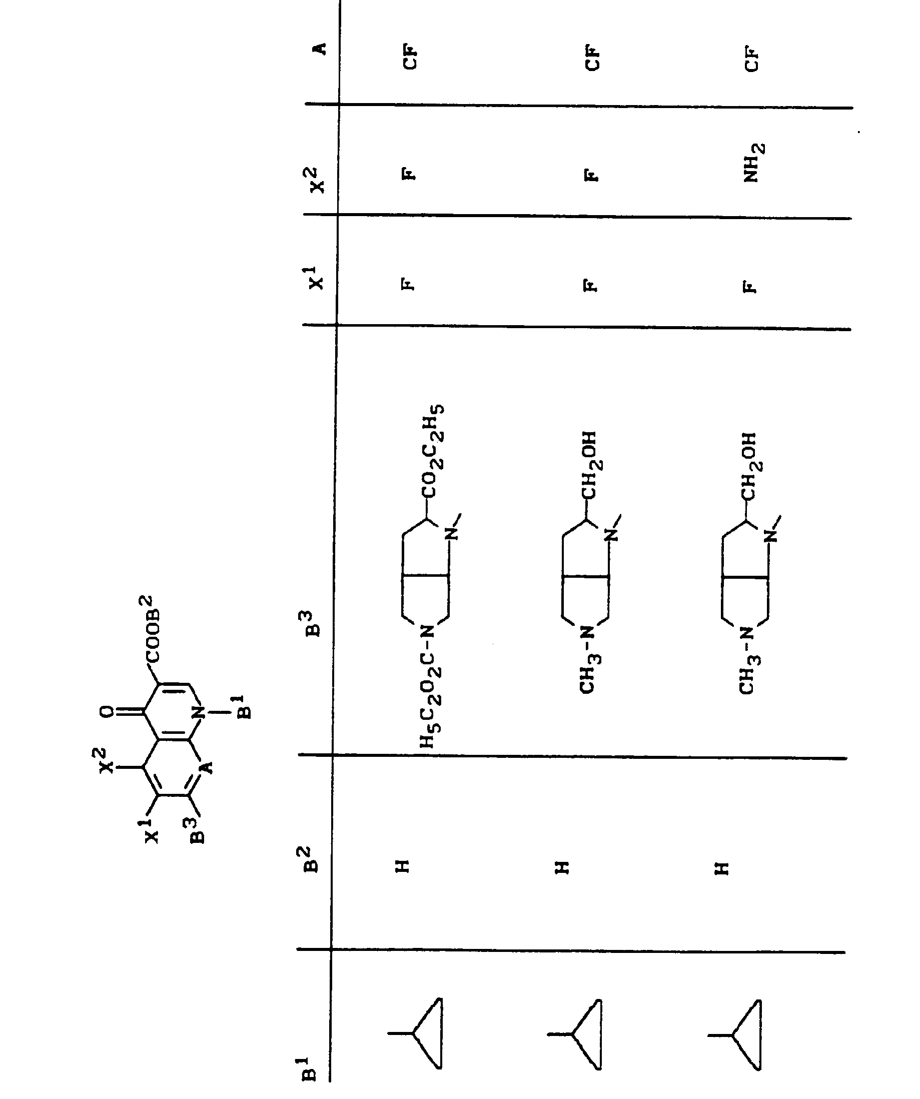

Die erfindungsgemäßen Verbindungen wirken stark antibiotisch und zeigen bei geringer Toxizität ein breites antibakterielles Spektrum gegen gram-positive und gramnegative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen Sie sind daher besonders gut zur Prophylaxe und Chemotherapie von lokalen und systemischen Infektionen in der Human- und Tiermedizin geeignet, die durch diese Erreger hervorgerufen werden.

Die erfindungsgemäßen Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen, Emulsionen, Pasten, Salben, Gele, Cremes, Lotionen, Puder und Sprays genannt.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen im Vergleich zu 1-Cyclopropyl-7-(2; 3-dimethyl-2,7-diazabicyclo[3.3.0]oct-7-yl-6, 8-difluor-1,4-dihydro-4-oxo-3-chinolincarbonsäure-Hydrochlorid (Beispiel 37 b aus EP-A-0 350 733) angegeben:
MHK-Werte (mg/I) bestimmt durch Agar-Verdünnungstest (Denley Multipoint Inoculator; Iso-Sensitest-Agar)

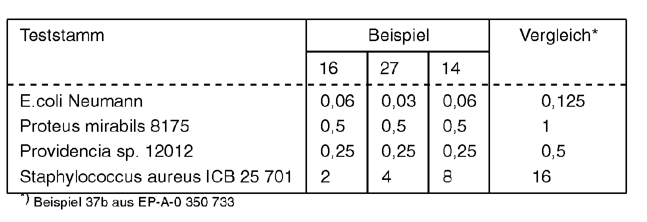

### Herstellunq der Zwischenprodukte:

### Beispiel A

### 2-Benzyl-2,7-diazabicyclo[3.3.0]octan

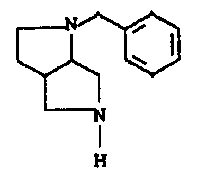

### a) N-(2,2-Dimethoxyethyl)-carbamidsäureethylester

Zu 214 g (2 Mol) Aminoacetaldehyddimethylacetal in 1 I Toluol und 90 g NaOH in 500 ml Wasser tropft man 214 g (2 Mol) Chlorameisensäureethylester bei 10°C. Man rührt noch 2 Stunden bei Raumtemperatur, trennt die wäßrige Phase ab, sättigt sie mit Kochsalz und extrahiert mit Toluol. Die Toluollösungen werden über Magnesiumsulfat getrocknet, eingeengt und destilliert.
Ausbeute: 338 g (95,4 % der Theorie)
Siedepunkt: 60°C/0,03 mbar

### b) N-Allyl-N-(2.2-dimethoxyethyl)-carbamidsäureethylester

Man legt 500 g (2,82 Mol) N-(2,2-Dimethoxyethyl)-carbamidsäureethylester, 625 g gepulvertes Kaliumhydroxid und 10 g Triethylbenzylammoniumchlorid in 2,7 1 Toluol vor und tropft 345 g (2,85 Mol) Allylbromid bei Raumtemperatur hinzu. Man rührt über Nacht bei Raumtemperatur, saugt die Salze ab, wäscht das Filtrat einmal mit gesättigter Kochsalzlösung, trocknet über Kaliumcarbonat, engt ein und destilliert.
Ausbeute: 582 g (95 % der Theorie)
Siedepunkt: 64°C/0,1 mbar

### c) N-Allyl-N-(2-oxoethyl)-carbamidsäureethylester

Man erhitzt 68 g (0,313 Mol) N-Allyl-N-(2,2-dimethoxyethyl)-carbamidsäureethylester mit 150 ml Ameisensäure eine Stunde auf 100°C. Man gießt auf Eis, extrahiert mehrfach mit Methylenchlorid, wäscht die organischen Phasen mit Natriumhydrogencarbonatlösung, trocknet über Magnesiumsulfat, engt ein und destilliert.
Ausbeute: 46,7 g (87,2 % der Theorie)
Siedepunkt: 58°C / 0,09 mbar

### d) N-Benzylqlycin

Man erhitzt 225,8 g (1,17 Mol) N-Benzylglycinethylester (J. Am. Chem. Soc.72, 1238 (1950)) in 600 ml Wasser über Nacht unter Rückfluß. Man saugt auskristallisiertes Produkt ab und extrahiert das Filtrat einmal mit tert.-Butylmethylether. Man engt die wäßrige Phase ein und trocknet die erhaltenen Kristalle zusammen mit dem abfiltrierten Produkt im Exsikkator über Phosphorpentoxid.
Ausbeute: 184 g (95 % der Theorie)
Schmelzpunkt: 199°C

### e) 2-Benzyl-2,7-diazabicyclo[3.3.0]octan-7-carbonsäurethylester

Man erhitzt 42,8 g (0,25 Mol) N-Allyl-N-(2-oxoethyl)-carbamidsäureethylester mit 41,3 g (0,25 Mol) N-Benzylglycin in 750 ml Toluol über Nacht unter Rückfluß. Man engt ein und destilliert den Rückstand.
Ausbeute 59,6 g (87% der Theorie)
Siedepunkt: 140°C / 0,09 mbar

### f) 2-Benzyl-2,7-diazabicyclo[3.3.3]-octan

Man erhitzt 55,6 g (0,2 Mol) 2-Benzyl-2,7-diazabicyclo[3.3.0]octan-7-carbonsäureethylester mit 300 ml konzentrierter Salzsäure über Nacht unter Rückfluß. Dann stellt man den Ansatz mit Kaliumcarbonat alkalisch, extrahiert fünfmal mit je 100 ml Chloroform, trocknet über Kaliumcarbonat, engt ein und destilliert.
Ausbeute: 31 g (76,6 % der Theorie)
Siedepunkt: 105°C / 0,45 mbar

### Beispiel B

### 2,7-Diazabicyclo[3.3.0]octan-2-carbonsäureethylester

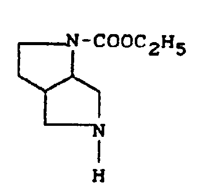

### a) 2-Benzyldiazabicyclo[3.3.0]octan-7-carbonsäure-tert.-butylester

Man löst 20,2 g (0,1 Mol) 2-Benzyl-2,7-diazabicyclo[3.3.0]octan in 125 ml tert.-Butanol; setzt eine Lösung von 4,2 g Natriumhydroxid in 100 ml Wasser hinzu und tropft bei Raumtemperatur 23 g (0,105 Mol) Pyrokohlensäure- di-tert.-butylester hinzu. Man rührt über Nacht bei Raumtemperatur, extrahiert fünfmal mit je 100 ml Chloroform, trocknet über Kaliumcarbonat, engt ein und destilliert.
Ausbeute: 24,8 g (82 % der Theorie)
Siedepunkt: 145 - 149°C / 0,8 mbar

### b) 2,7-Diazabicyclo[3.3.0[octan-7-carbonsäure-tert.-butylester

Man hydriert 24 g (79,4 mmol) 2-Benzyldiazabicyclo-[3.3.0]octan-7-carbonsäure-tert.-butylester in 400 ml Ethanol an 3 g Palladium-Aktivkohle (10 % Pd) bei 100°C und 100 bar. Der Katalysator wird abfiltriert, das Filtrat eingeengt und der Rückstand destilliert.
Ausbeute: 13,1 g (77,7 % der Theorie)
Siedepunkt: 87°C/0,1 mbar

### c) 2,7-Diazabicyclo[3.3.0]octan-2,7-dicarbonsäure-2-ethylester-7-tert.-butylester

Man löst 13 g (61,2 mmol) 2,7-Diazabicyclo[3.3.0]-octan-7-carbonsäure-tert.-butylester in 100 ml Toluol, setzt eine Lösung von 3 g Natriumhydroxid in 20 ml Wasser hinzu und tropft bei Raumtemperatur 7 g (64,5 mmol) Chlorameisensäureethylester hinzu. Man rührt drei Stunden bei Raumtemperatur, trennt die wäßrige Phase ab und extrahiert sie zweimal mit je 100 ml Methylenchlorid. Man trocknet die organischen Lösungen über Magnesiumsulfat, engt ein und destilliert.
Ausbeute 16 g (91,9 % der Theorie)
Siedepunkt: 125°C / 0,13 mbar

### d) 2,7-Diazabicyclo[3.3.0]octan-2-carbonsäureethylester

Man erhitzt 15,2 g (53,5 mmol) 2,7-Diazabicyclo[3.3.0]-octan-2,7-dicarbonsäure-2-ethylester-7-tert.-butylester in 100 ml Chloroform mit 10,5 g (55,3 Mol) para-Toluolsulfonsäure fünf Stunden unter Rückfluß. Man wäscht mit 50 ml 10%iger Natronlauge, trocknet die organische Phase über Kaliumcarbonat, engt ein und destilliert.
Ausbeute 9,5 g (96,4 % der Theorie)
Siedepunkt: 80 - 90°C / 0,1 mbar

### Beispiel C

### 2-Methyl-2,7-diazabicyclo[3.3.0]octan

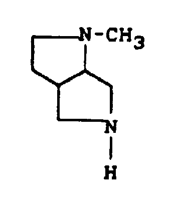

### a) 2-Methyl-2,7-diazabicyclo[3.3.0]octan-7-carbonsäuremethylester

Man erhitzt 8,6 g (50 mmol) N-Allyl-N-(2-oxoethyl)-carbamidsäureethylester mit 4,5 g (50 mmol) Sarkosin in 200 ml Toluol über Nacht unter Rückfluß. Man engt ein und destilliert den Rückstand.
Ausbeute: 7,5 g (75,7 % der Theorie)
Siedepunkt: 80 - 82°C / 0,1 mbar

### b) 2-Methyl-2,7-diazabicyclo[3.3.0]octan

Man erhitzt 9 g (45,4 mmol) 2-Methyl-2,7-diazabicyclo[3.3.0]octan-7-carbonsäureethylester mit 50 ml konzentrierter Salzsäure über Nacht unter Rückfluß. Man stellt den Ansatz mit Kaliumcarbonat alkalisch, extrahiert zehnmal mit je 50 ml Chloroform, trocknet über Kaliumcarbonat, engt ein und destilliert.
Ausbeute: 4,5 g (78 % der Theorie)
Siedepunkt: 72°C / 25 mbar

### Beispiel D

### 2-Phenyl-2,7-diazabicyclo[3,3,0]octan

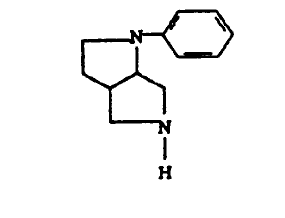

### a) 2-Phenyl-2,7-diazabicyclo[3.3.0]octan-7-carbonsäureethylester

Man erhitzt 8,6 g (50 mmol) N-Allyl-N-(2-oxoethyl)-carbamidsäureethylester und 7,6 g (50 mmol) Phenylglycin in 200 ml Toluol über Nacht unter Rückfluß. Man dekantiert von harzigem Material, engt ein und destilliert den Rückstand.
Ausbeute: 8,1 g (62,2 % der Theorie)
Siedepunkt: 151°C/0,12 mbar

### b) 2-Phenyl-2,7-diazabicyclo[3.3.0]octan

Man erhitzt 7,6 g (31,6 mmol) 2-Phenyl-2,7-diazabicyclo[3.3.0]octan-7-carbonsäureethylester mit 50 ml konzentrierter Salzsäure über Nacht unter Rückfluß. Man engt ein, nimmt den Rückstand in 50 ml 10%iger

Natronlauge auf und extrahiert fünfmal mit je 50 ml Chloroform. Man trocknet über Kaliumcarbonat, engt ein und destilliert den Rückstand.
Ausbeute: 3,7 g (62 % der Theorie)
Siedepunkt: 103°C / 0,08 mbar

### Beispiel E

### 3-Methyl-2,7-diazabicyclo[3.3.0]octan

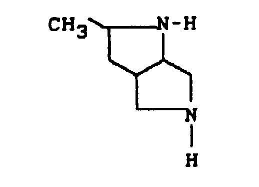

### a)

### N-Benzylalanin

Man erhitzt 333 g (1,72 mol) N-Benzylalaninmethylester (J. Chem. Soc. 4374 (1952)) mit 860 ml Wasser über Nacht unter Rückfluß. Man saugt ausgeschiedenes Produkt ab und extrahiert das Filtrat einmal mit tert.-Butylmethylether. Die wäßrige Lösung wird eingeengt und die erhaltenen Kristalle mit der ersten Kristallfraktion im Exsikkator über Phosphorpentoxid getrocknet.
Ausbeute: 280 g (91 % der Theorie)
Schmelzpunkt: 270 - 276°C (Zersetzung)

### b) 2-Benzyl-3-methyl-2,7-diazabicyclo[3.3.0]octan-7-carbonsäureethylester

Man erhitzt 42,8 g (0,25 mol) N-Allyl-N-(2-oxoethyl)-carbamidsäureethylester mit 44,8 g (0,25 mol) N-Benzylalanin in 750 ml Toluol über Nacht unter Rückfluß. Man engt ein und destilliert den Rückstand zweimal.
Ausbeute: 32 g (44,4 % der Theorie)
Siedepunkt: 128-133°C/0,06 mbar

Das Produkt besteht zu 96 % aus einem Stereoisomeren.

### c) 3-Methyl-2,7-diazabicyclo[3.3.0]octan-7-carbonsäureethylester

Man hydriert 32 g (0,11 mol) 2-Benzyl-3-methyl-2,7-diazabicyclo[3.3.0]octan-7-carbonsäureethylester in 560 ml Ethanol an 4,5 g Palladium-Aktivkohle bei 100°C und 100 bar. Der Katalysator wird abgesaugt, das Filtrat eingeengt und der Rückstand destilliert.
Ausbeute: 17,1 g (77,7 % der Theorie)
Siedepunkt: 140-145°C/8 mbar

### d) 3-Methyl-2,7-diazabicyclo[3.3.0]octan

Man erhitzt 17 g (85,7 mmol) 3-Methyl-2,7-diazabicyclo[3.3.0]octan-7-carbonsäureethylester mit 100 ml konzentrierter Salzsäure über Nacht unter Rückfluß. Man engt ein, nimmt in 50 ml Wasser auf, stellt mit Kaliumcarbonat alkalisch und extrahiert zehnmal mit je 50 ml Chloroform. Man trocknet über Kaliumcarbonat, engt ein und destilliert den Rückstand.
Ausbeute: 6 g (55 % der Theorie)
Siedepunkt: 68-70°C/6 mbar

### Beispiel

### 2,3-Dimethyl-2,7-diazabicyclo[3.3.0]octan

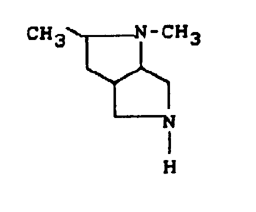

### a) 2,3-Dimethyl-2,7-diazabicyclo[3.3.0]octan-7-carbonsäureethylester

Man erhitzt 17,2 g (0,1 mol) N-Allyl-N-(2-oxoethyl)-carbamidsäureethylester mit 10,5 g (0,1 mol) N-Methylalanin in 300 ml Toluol über Nacht unter Rückfluß. Man engt ein und destilliert den Rückstand.
Ausbeute 11,3 g (53,2 % der Theorie)
Siedepunkt: 81°C/0,25 mbar

### b) 2,3-Dimethyl-2,7-diazabicyclo[3.3.0]octan

Man erhitzt 7,25 g (34,2 mmol) 2,3-Dimethyl-2,7-diazabicyclo[3.3.0]octan-7-carbonsäureethylester mit 50 ml konzentrierter Salzsäure über Nacht unter Rückfluß. Man stellt mit Kaliumcarbonat alkalisch, extrahiert zehnmal mit je 50 ml Chloroform, trocknet über Kaliumcarbonat, engt ein und destilliert den Rückstand.
Ausbeute: 3 g (62,5 % der Theorie)
Siedepunkt: 72-74°C/10 mbar

### Beispiel G

### 2,8-Dimethyl-2,7-diazabicyclo[3.3.0]octan

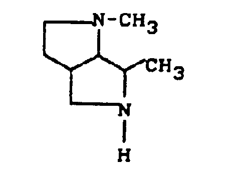

### a) N-(1.1-Dimethoxyprop-2-yl)-carbamidsäureethylester

Zu 86,2 g (0,72 mol) 2-Aminopropionaldehyddimethylacetal in 350 ml Toluol und 32 g (0,8 mol) NaOH in 300 ml Wasser tropft man unter Eiskühlung 80 g (0,73 mol) Chlorameisensäureethylester. Man rührt noch 2 Stunden bei Raumtemperatur, trennt die organische Phase ab, extrahiert die wäßrige Phase mit Toluol und trocknet die Toluollösungen über K₂CO₃. Man engt ein und destilliert.
Ausbeute: 132 g (95 % der Theorie)
Siedepunkt: 55°C/0,06 mbar

### b) N-Allyl-N-(1.1-dimethoxyprop-2-yl)-carbamidsäureethylester

Man legt 151 g (0,79 mol) N-(1,1-Dimethoxyprop-2-yl)-carbamidsäureethylester, 175 g gepulvertes Kaliumhydroxid und 2,8 g Triethylbenzylammoniumchlorid in 750 ml Toluol vor und tropft bei Raumtemperatur 94 g (0,777 mol) Allylbromid hinzu. Nach Rühren über Nacht bei Raumtemperatur wurden weitere 10 g (82,6 mmol) Allylbromid zugetropft und ein Tag bei Raumtemperatur gerührt Man setzt Wasser zu bis alle Salze in Lösung gegangen sind, trennt die wäßrige Phase ab und extrahiert sie zweimal mit je 150 ml Toluol. Man trocknet über K₂CO₃, engt ein und destilliert den Rückstand.
Ausbeute: 173 g (94,7 % der Theorie)
Siedepunkt: 68°C/0,1 mbar

### c) Allyl-(1,1-dimethoxyprop-2-yl)-amin

Zu 12 g (0,1 mol) 1,1-Dimethoxyaceton in 100 ml Ethanol setzt man 20 g Molsieb zu und tropft dann 7 g (0,12 mol) Allylamin hinzu. Man läßt über Nacht bei Raumtemperatur stehen, dekantiert vom Molsieb, kühlt im Eisbad auf 0°C und versetzt mit 4 g (0,1 mol) Natriumborhydrid in kleinen Portionen. Man rührt über Nacht bei Raumtemperatur, engt ein, nimmt in 100 ml Wasser auf, sättigt mit Kaliumcarbonat und extrahiert fünfmal mit je 100 ml Chloroform. Man trocknet über Kaliumcarbonat, engt ein und destilliert.
Ausbeute 10,3 g (64,7 % der Theorie)
Siedepunkt: 75°C/25 mbar

### d) N-Allyl-N-(1,1-dimethoxyprop-2-yl)-carbamidsäureethylester

Man legt 125 g (0,785 mol) Allyl-(1;1-dimethoxyprop-2-yl)amin in 400 ml Toluol vor, setzt eine Lösung von 40 g Natriumhydroxid in 200 ml Wasser hinzu, kühlt im Eisbad auf 0°C und tropft 95 g (0,876 mol) Chlorameisensäureethylester hinzu. Anschließend rührt man drei Stunden bei Raumtemperatur, trennt die wäßrige Phase ab und extrahiert sie zweimal mit je 100 ml Toluol. Man trocknet über Kaliumcarbonat, engt ein und destilliert.
Ausbeute: 170,8 g (94 % der Theorie)
Siedepunkt: 55°C/0,05 mbar

### e) N-Allyl-N-(1-oxoprop-2-yl)-carbamidsäureethylester

Man erhitzt 182 g (0,787 mol) N-Allyl-N-(1,1-dimethoxyprop-2-yl)-carbamidsäureethylester in 1,5 1 Wasser mit 80 ml Ameisensäure drei Stunden unter Rückfluß. Man sättigt den Ansatz mit Kochsalz, trennt die organische Phase ab und extrahiert die wäßrige Phase zweimal mit je 500 ml Methylenchlorid. Die organischen Lösungen werden mit gesättigter Natriumhydrogencarbonatlösung neutral gewaschen, über Magnesiumsulfat getrocknet, eingeengt und destilliert.
Ausbeute: 134 g (91,9 % der Theorie)
Siedepunkt: 65°C/0,23 mbar

### f) 2,8-Dimethyl-2.7-diazabicyclo[3.3.0]octan-7-carbonsäureethylester

Man erhitzt 18,5 g (0,1 mol) N-Allyl-N-(1-oxoprop-2-yl)-carbamidsäureethylester mit 9 g (0,1 mol) Sarkosin in 300 ml Toluol über Nacht am Wasserabscheider unter Rückfluß. Man engt ein und destilliert den Rückstand.
Ausbeute: 17 g (80 % der Theorie)
Siedepunkt: 140-150°C/8 mbar

### g) 2,8-Dimethyl-2,7-diazabicyclo[3.3.0]octan

Man erhitzt 16,9 g (79,6 mol) 2,8-Dimethyl-2,7-diazabicyclo[3.3.0]octan-7-carbonsäureethylester mit 130 ml konzentrierter Salzsäure über Nacht unter Rückfluß. Man engt ein, nimmt in 50 ml Wasser auf, stellt mit Kaliumcarbonat alkalisch und extrahiert fünfmal mit je 50 ml Chloroform. Man trocknet über Kaliumcarbonat, engt ein und destilliert den Rückstand.
Ausbeute: 6,6 g (58,5 % der Theorie)
Siedepunkt: 60-62°C/6 mbar

### Beispiel H

### 5-Chlor-2-methyl-2,7-diazabicyclo[3.3.0]octan

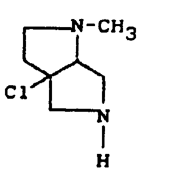

### a) N-(2-Chlorallyl)-N-(2,2-dimethoxyethyl)-carbamidsäureethylester

Man legt 115 g (0,65 mol) N-(2,2-Dimethoxyethyl)-carbamidsäureethylester, 130 g gepulvertes Kaliumhydroxid und 2 g Triethylbenzylammoniumchlorid in 650 ml Toluol vor und tropft bei Raumtemperatur 142 g (0,7 mol) 2-Chlorallyliodid hinzu. Nach Rühren über Nacht zeigte ein Gaschromatogramm unvollständigen Umsatz, daher wurden erneut 65 g gepulvertes Kaliumhydroxid und 1 g Triethylbenzylammoniumchlorid zugesetzt und weitere 71 g (0,35 mol) 2-Chlorallyliodid zugetropft. Nach Rühren über Nacht bei Raumtemperatur wurden die Salze abgesaugt, das Filtrat mit gesättigter Kochsalzlösung gewaschen, über Magnesiumsulfat getrocknet, eingeengt und der Rückstand destilliert.
Ausbeute: 140,9 g (86 % der Theorie)
Siedepunkt: 92-97°C/0,8 mbar

### b) N-(2-Chlorallyl)-N-(2-oxoethyl)-carbamidsäureethylester

Man erhitzt 151 g (0,6 mol) N-(2-Chlorallyl)-N-(2,2-dimethoxyethyl)-carbamidsäureethylester mit 60 ml Ameisensäure in 1,2 1 Wasser drei Stunden unter Rückfluß. Man sättigt mit Kochsalz, trennt die wäßrige Phase ab und extrahiert sie zweimal mit je 300 ml Methylenchlorid. Die organischen Phasen werden mit gesättigter Natriumhydrogencarbonatlösung neutral gewaschen, über Magnesiumsulfat getrocknet, eingeengt und destilliert.
Ausbeute 97,1 g (78 % der Theorie)
Siedepunkt: 88-91°C/0,06 mbar

### c) 5-Chlor-2-methyl-2,7-diazabicyclo[3.3.0]octan-7-carbonsäureethylester

Man erhitzt 10,3 g (50 mmol) N-(2-Chlorallyl)-N-(2-oxoethyl)-carbamidsäureethylester mit 4,5 g (50 mmol) Sarkosin in 200 ml Toluol über Nacht unter Rückfluß. Man engt ein und destilliert den Rückstand.
Ausbeute: 10,6 g (91 % der Theorie)
Siedepunkt: 80°C/0,1 mbar

### d) 5-Chlor-2-methyl-2,7-diazabicyclo[3.3.0]octan

Man erhitzt 9,3 g (40 mmol) 5-Chlor-2-methyl-2,7-diazabicyclo[3.3.0]octan-7-carbonsäureethylester mit 50 ml konzentrierter Salzsäure über Nacht unter Rückfluß. Man engt ein, nimmt in 30 ml Wasser auf, stellt mit Kaliumcarbonat alkalisch und extrahiert fünfmal mit je 50 ml Chloroform. Man trocknet über Kaliumcarbonat, engt ein und destilliert.
Ausbeute: 4,7 g (72 % der Theorie)
Siedepunkt: 73°C/4 mbar

### Beispiel

### 5-Chlor-2,3-dimethyl-2,7-diazabicyclo[3.3.0]octan

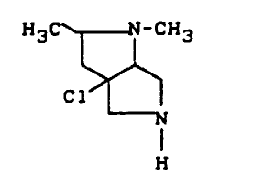

### a) 5-Chlor-2,3-dimethyl-2,7-diazabicyclo[3.3.0]octan-7-carbonsäureethylester

Man erhitzt 10,3 g (50 mmol) N-(2-Chlorallyl)-N-(2-oxoethyl)-carbamidsäureethylester mit 5,2 g (50,5 mmol) N-Methylalanin in 200 ml Toluol über Nacht unter Rückfluß. Man engt ein und destilliert den Rückstand.
Ausbeute 8,1 g (65,7 % der Theorie)
Siedepunkt: 87°C/0,08 mbar

### b) 5-Chlor-2,3-dimethyl-2,7-diazabicyclo[3.3.0]octan

Man erhitzt 7,6 g (30,8 mmol) 5-Chlor-2,3-dimethyl-2,7-diazabicyclo[3.3.0]octan-7-carbonsäureethylester mit 30 ml konzentrierter Salzsäure über Nacht unter Rückfluß. Man engt ein, nimmt in 30 ml Wasser auf, stellt mit Kaliumcarbonat alkalisch, extrahiert fünfmal mit je 50 ml Chloroform, trocknet über Kaliumcarbonat, engt ein und destilliert.
Ausbeute: 3,7 g (68,4 % der Theorie)
Siedepunkt: 95-97°C/6 mbar

### Beispiel J

### 1,4-Diazatricyclo[6.2.0.0^{2,6}]decan

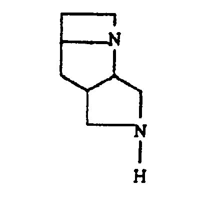

### a) 1,4-Diazatricyclo[6.2.0.0^{2,6}]decan-4-carbonsäureethylester

Man erhitzt 17,1 g (0,1 mol) N-Allyl-N-(2-oxoethyl)-carbamidsäureethylester mit 10 g (0,1 mol) Azetidin-2-carbonsäure in 200 ml Toluol über Nacht am Wasserabscheider unter Rückfluß. Man saugt nicht umgesetzte Aminosäure ab, engt das Filtrat ein und destilliert den Rückstand.
Ausbeute: 13,8 g (65,6 % der Theorie)
Siedepunkt: 1 08°C/0,35 mbar

### b) 1,4-Diazatricyclo[6.2.0.0^{2,6}]docan

Man erhitzt 13,7 g (65,1 mmol) 1,4-Diazatricyclo[6.2.0.0^{2,6}]decan-4-carbonsäureethylester mit 42 g Ba(OH)₂. 8H₂0 in 150 ml Wasser über Nacht unter Rückfluß. Man versetzt mit Kaliumcarbonat, saugt Bariumcarbonat ab und extrahiert das Filtrat zehnmal mit je 100 ml Chloroform. Man trocknet über Kaliumcarbonat, engt ein und destilliert den Rückstand.
Ausbeute 5,3 g (58,9 % der Theorie)
Siedepunkt: 85°C/6 mbar

### Beispiel K

### 1,4-Diazatricyclo[6.3.0.^{2,6}]undecan

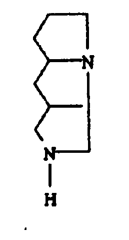

### a) 1,4-Diazatricyclo[6.3.0.0^{2,6}]undecan-4-carbonsäureethylester

Man erhitzt 8,6 g (50 mmol) N-Allyl-N-(2-oxoethyl)-carbamidsäureethylester mit 5,8 g (50 mmol) Prolin in 200 ml Toluol über Nacht unter Rückfluß. Man engt ein und destilliert den Rückstand.
Ausbeute: 9,6 g (86 % der Theorie)
Siedepunkt: 102-112°C/0,13-0,15 mbar

### b) 1,4-Diazatricyclo[6.3.0.0^{2,6}]undecan

Man erhitzt 9 g (40 mmol) 1,4-Diazatricyclo[6.3 0.0^{2,6}]undecan-4-carbonsäureelhylester mit 50 ml konzentrierter Salzsäure über Nacht unter Rückfluß. Man stellt mit Kaliumcarbonat alkalisch, extrahiert zehnmal mit je 50 ml Chloroform, trocknet über Kaliumcarbonat, engt ein und destilliert den Rückstand.
Ausbeute 4,9 g (80,5 % der Theorie)
Siedepunkt: 50°C/0,05 mbar

### Beispiel

### 10-Hydroxy-1,4-diazatricyclo[6.3.0.0^{2,6}]undecan

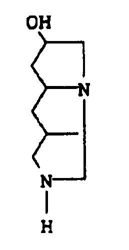

### a) 10-Hydroxy-1,4-diazatricyclo[6.3.0.0^{2,6}]undecan-4-carbonsäureethylester

Man erhitzt 8,6 g (50 mmol) N-Allyl-N-(2-oxoethyl)-carbamidsäureethylester mit 6,6 g (50 mmol) trans-4-Hy- droxyprolin in 200 ml Dimethylformamid über Nacht auf 120°C. Man engt ein und destilliert den Rückstand.
Ausbeute: 9,7 g (81 % der Theorie)
Siedepunkt: 170°C/0,3 mbar

Das Produkt besteht überwiegend aus zwei Stereoisomeren im Verhältnis 1:1.

### b) 10-Hvdroxv-1,4-diazathcyclo[6.3.0^{2,6}]undecan

Man erhitzt 8 g (33,3 mmol) 10-Hydroxy-1,4-diazatricyclo[6.3.0.0^{2,6}]undecan-4-carbonsäureethylester mit 21 g Ba(OH)₂.8H₂O in 150 ml Wasser über Nacht unter Rückfluß. Man sättigt mit Kaliumcarbonat, saugt Bariumcarbonat ab und extrahiert zehnmal mit je 100 ml Chloroform. Man trocknet über Kaliumcarbonat, engt ein und destilliert.
Ausbeute: 4,6 9 (82% der Theorie)
Siedepunkt: 110-115°C/0,1 mbar

### Beispiel M

### 1,4-Diazatricyclo[6.4.0.0^{2,6}]dodecan

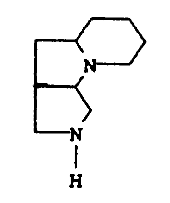

### a) 1,4-Diazatricyclo[6.4.0.0^{2,6}]dodecan-4-carbonsäureethylester

Man erhitzt 17,1 g (0,1 mol) N-Allyl-N-(2-oxoethyl)-carbamidsäureethylester mit 13 g (0,1 mol) Piperidin-2-carbonsäure in 200 ml Toluol über Nacht unter Rückfluß. Man engt ein und destilliert den Rückstand.
Ausbeute 20,8 g (87,2 % der Theorie)
Siedepunkt: 105-112°C/0,12 mbar
b) 1,4-Diazatricyclo[6.4.0.0^{2,6}]decan

Man erhitzt 20,7 g (86,8 mmol) 1,4-Diazatricyclo[6.4.0.0^{2,6}]dodecan-4-carbonsäureethylester mit 250 ml konzentrierter Salzsäure über Nacht unter Rückfluß. Man engt ein, nimmt in 50 ml Wasser auf und stellt mit Kaliumcarbonat alkalisch. Man extrahiert zehnmal mit je 50 ml Chloroform, trocknet über Kaliumcarbonat, engt ein und destilliert den Rückstand.
Ausbeute: 8,5 9 (58,9 % der Theorie)
Siedepunkt: 108°C/8 mbar

### Beispiel N

### 10-Thia-1,4-diazatricyclo[6.3.0.0^{2,6}]undecan

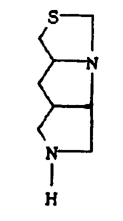

### a) 10-Thia-1,4-diazatricyclo[6.3.0.0^{2,6}]undecan-4-carbonsäureethylester

Man erhitzt 17,2 g (0,1 mol) N-Allyl-N-(2-oxoethyl)-carbamidsäureethylester mit 13,5 g (0,1 mol) Thiazolidin-4-carbonsäure in 300 ml Toluol über Nacht unter Rückfluß. Man engt ein und destilliert.
Ausbeute: 20 g (82,5 % der Theorie)
Siedepunkt: 155-156°C/0,5 mbar

### b) 10-Thia-1,4-diazatricyclo[6.3.0.0^{2,6}]undecan

Man erhitzt 12,5 g (50 mmol) 10-Thia-1,4-diazatricyclo[6.3.0 0^{2,6}]undecan-4-carbonsäureethylester mit 32g Ba(OH)₂.8H₂O in 225 ml Wasser über Nacht unter Rückfluß. Man versetzt mit Kaliumcarbonat, saugt Bariumcarbonat ab und extrahiert das Filtrat zehnmal mit je 100 ml Chloroform. Man trocknet über Kaliumcarbonat, engt ein und destilliert.
Ausbeute: 6,2 g (72,8 % der Theorie)
Siedepunkt: 90-94°C/0,05 mbar

### Beispiel

### 9,9-Dimethyl-10-thia-1,4-diazatricyclo[6.3.0.02,6]undecan

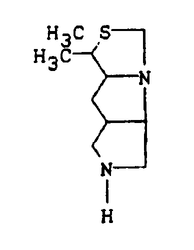

### a) 9,9-Dimethyl-10-thia-1,4-diazatricyclo[6.3.0.0^{2,6}]undecan-4-carbonsäureethylester

Man erhitzt 8,6 g (50 mmol) N-Allyl-N-(2-oxoethyl)-carbamidsäureethylester mit 8,1 g (50 mmol) 5,5-Dime- thylthiazolidin-4-carbonsäure in 200 ml Toluol über Nacht unter Rückfluß. Man engt ein und destilliert den Rückstand.
Ausbeute 8,4 9 (62,2 % der Theorie)
Siedepunkt: 141-155°C/0,03-0,05 mbar

### b) 9,9-Dimethyl-10-thia-1,4-diazatricyclo[6.3.0.0^{2,6}]undecan

Man erhitzt 6 g (22,2 mmol) 9,9-Dimethyl-10-thia-1,4-diazatricyclo[6.3.0.0^{2,6}]undecan-4-carbonsäureethyle- ster mit 12 g Ba(OH)₂.8H₂O in 100 ml Wasser über Nacht unter Rückfluß. Man versetzt mit Kaliumcarbonat, saugt Bariumcarbonat ab und extrahiert das Filtrat zehnmal mit je 100 ml Chloroform. Man trocknet über Kaliumcarbonat, engt ein und destilliert den Rückstand.
Ausbeute: 2,25 g (51 % der Theorie)
Siedepunkt: 83°C/0,02 mbar

### Beispiel P

### 7-Methyl-2,7-diazabicyclo[3.3.0]octan

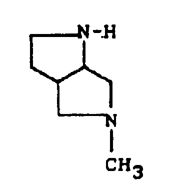

### a) 2-Benzyl-7-methyl-2,7-diazabicyclo[3.3.0]octan

Zu 3,8 g (0,1 mol) Lithiumaluminiumhydrid in 100 ml absolutem Tetrahydrofuran tropft man 13,7 g (50 mmol) 2-Benzyldiazabicyclo[3.3.0]octan-7-carbonsäureethylester in 20 ml absolutem Tetrahydrofuran. Man erhitzt über Nacht unter Rückfluß und zersetzt nacheinander mit je 4 ml Wasser, 15 %iger Kalilauge und Wasser. Die anorganischen Salze werden abgesaugt und dreimal mit je 50 ml Tetrahydrofuran ausgekocht. Die organischen Lösungen werden eingeengt und der Rückstand destilliert.
Ausbeute: 10,4 g (96 % der Theorie)
Siedepunkt: 90-100°C/0,1 mbar

### b) 7-Methyl-2,7-diazabicyclo[3.3.0]octan

Man hydriert 10,3 g (47,6 mmol) 2-Benzy-7-methyl-2,7-diazabicyclo[3.3.0]octan in 200 ml Ethanol an 2,5 g Palladium-Aktivkohle (10 % Pd) bei 100°C und 100 bar. Der Katalysator wird abgesaugt, das Filtrat eingeengt und der Rückstand destilliert.
Ausbeute: 4,2 g (69,9 % der Theorie)
Siedepunkt: 50-53°C/6 mbar

### Beispiel Q

### 2-Benzyl-8-methyl-2,7-diazabicyclo[3.3.0]octan

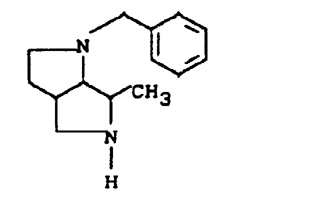

### a) 2-Benzyl-8-methyl-2,7-diazabicyclo[3.3.0]octan-7-carbonsäureethylester

Man erhitzt 37 g (0,2 mol) N-Allyl-N-(1-oxo-2-propyl)-carbamidsäureethylester mit 33 g (0,2 mol) N-Benzylglycin in 500 ml Toluol am Wasserabscheider über Nacht unter Rückfluß. Man engt den Ansatz ein und destilliert den Rückstand.
Ausbeute: 48,5 g (84 % der Theorie)
Siedepunkt: 140-145°C/0,2 mbar

Das Produkt ist gaschromatographisch ein einheitliches Stereoisomeres.

### b) 2-Benzyl-8-methyl-2,7-diazabicyclo[3.3.0]octan

Man erhitzt 16 g (55 mmol) 2-Benzyl-8-methyl-2;7-diazabicyclo[3.3.0]octan-7-carbonsäureethylester mit 50 ml konzentrierter Salzsäure über Nacht unter Rückfluß. Man engt den Ansatz ein, löst in 50 ml Wasser und stellt mit Kaliumcarbonat alkalisch. Man extrahiert fünfmal mit je 50 ml Chloroform, trocknet über K₂C0₃, engt ein und destilliert den Rückstand.
Ausbeute: 7,9 g (66,4 % der Theorie)
Siedepunkt: 108-113°C/0,17 mbar

### Beispiel R

### 8-Methyl-2,7-diazabicyclo[3.3.0]octan

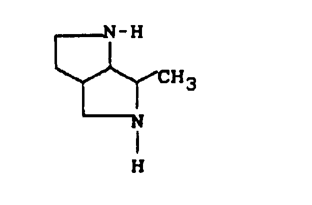

Man hydriert 7,8 g (36 mmol) 2-Benzyl-B-methyl-2,7-diazabicyclo[3.3.0]octan in 200 ml Ethanol an 2 g Palladium-Aktivkohle (10 % Pd) bei 100°C und 100 bar. Man saugt den Katalysator ab, engt das Filtrat ein und destilliert den Rückstand. Das Destillat kristallisiert.
Ausbeute: 3,3 g (72,7 % der Theorie)
Siedepunkt: 110°C/30 mbar
Schmelzpunkt: 72-75°C

### Beispiel S

### 8-Methyl-2,7-diazabicyclo[3.3.0]octan-7-carbonsäureethylester

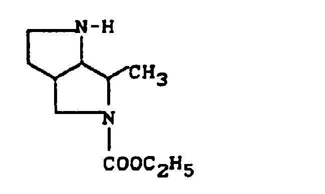

Man hydriert 16 g (55 mmol) 2-Benzyl-8-methyl-2,7-diazabicyclo[3.3.0]octan-7-carbonsäureethylester in 300 ml Ethanol an 3 g Palladium-Aktivkohle (10 % Pd) bei 100°C und 100 bar Man saugt den Katalysator ab, engt das Filtrat ein und destilliert den Rückstand.
Ausbeute: 9,7 g (89% der Theorie)
Siedepunkt: 100°C/0,1 mbar

### Beispiel T

### 7,8-Dimethyl-2,7-diazabicyclo[3.3.0]octan

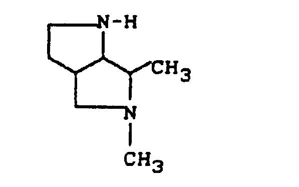

### a) 2-Benzyl-7,8-dimethyl-2,7-diazabicyclo[3.3.0]octan

Zu 3,8 g (0,1 mol) Lithiumaluminiumhydrid in 100 ml absolutem Tetrahydrofuran tropft man 14,4 g (50 mmol) 2-Benzyl-8-methyl-2,7-diazabicyclo[3.3.0]octan-7-carbonsäureethylester in 20 ml absolutem Tetrahydrofuran und erhitzt anschließend über Nacht unter Rückfluß. Man zersetzt nacheinander mit je 4 ml Wasser, 15 %iger Kalilauge und Wasser, saugt die anorganischen Salze ab und kocht sie dreimal mit je 50 ml Tetrahydrofuran aus. Die organischen Lösungen werden eingeengt und der Rückstand destilliert.
Ausbeute: 10,9 g (94,6 % der Theorie)
Siedepunkt: 105°C/0,08 mbar

### b) 7,8-Dimethyl-2,7-diazabicyclo[3.3.0]octan

Man hydriert 10,8 g (46,9 mmol) 2-Benzyl-7,8-dimathyl-2,7-diazabicyclo[3.3.0]octan in 200 ml Ethanol an 2,5 g Palladium-Aktivkohle bei 100°C und 100 bar. Man saugt den Katalysator ab, engt das Filtrat ein und destilliert den Rückstand.
Ausbeute: 4,3 g (65,4 % der Theorie)
Siedepunkt: 60-62°C/6 mbar

### Beispiel U

### 4-Methyl-2,7-diazabicyclo[3.3.0]octan

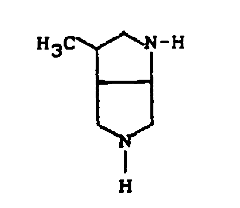

### a) N-(2-Buten-1-yl)-N-(2,2-dimethoxyethyl)-amin

Man legt 200 g Molsieb in 1000 ml Ethanol vor und setzt 105 g (1 mol) Aminoacetaldehyddimethylacetal und 70 g (1 mol) Crotonaldehyd hinzu. Man läßt über Nacht bei Raumtemperatur stehen, dekantiert vom Molsieb ab, kühlt auf 0°C und setzt 40 g Natriumborhydrid in 1 g-Portionen hinzu. Anschließend rührt man über Nacht bei Raumtemperatur, engt ein, nimmt in 500 ml Wasser auf und versetzt mit Kaliumcarbonat, bis sich eine organische Phase abscheidet. Man extrahiert mit Chloroform, trocknet über Kaliumcarbonat, engt ein und destilliert.
Ausbeute: 69,5 g (41,5 % der Theorie)
Siedepunkt: 85°C/12 mbar

### b) N-(2-Buten-1-yl)-N-(2,2-dimethoxyethyl)-carbamidsäureethylester

Man löst 69 g (0,41 mol) N-(2-Buten-1-yl)-N-(2,2-dimethoxyethyl)-amin in 200 ml Toluol, setzt 30 ml 45 %ige Natronlauge hinzu und tropft unter Eiskühlung 43 g (0,44 mol) Chlorameisensäureethylester hinzu, Man rührt noch drei Stunden bei Raumtemperatur, trennt die wäßrige Phase ab und extrahiert sie mit 100 ml Toluol. Man trocknet über Kaliumcarbonat, engt ein und destilliert.
Ausbeute: 92 g (94 % der Theorie)
Siedepunkt: 72°C/0,08 mbar

Man löst 90 g (0,5 mol) N-(2,2-Dimethoxyethyl)-carbamidsäureethylester in 500 ml Toluol, setzt 100 g gepulvertes Kaliumhydroxid und 1,5 g Triethylbenzylammoniumchlorid hinzu und tropft 80 g (0,6 mol) Crotylbromid (Isomerengemisch) hinzu. Man rührt über Nacht bei Raumtemperatur, löst die Salze in Wasser, trennt die wäßrige Phase ab und extrahiert sie einmal mit 100 ml Toluol. Man trocknet über Kaliumcarbonat, engt ein und destilliert.
Ausbeute: 112 g (96,8 % der Theorie)
Siedepunkt: 65°C/0,1 mbar

### c) N-(2-Buten-1-yl)-N-(2-oxoethyl)-carbamidsäureethylester

Man erhitzt 111 g (0,48 mol) N-(2-Buten-1-yl)-N-(2,2-dimethoxyethyl)-carbamidsäureethylester mit 50 g Ameisensäure in 950 ml Wasser drei Stunden unter Rückfluß. Man sättigt mit Kochsalz und extrahiert dreimal mit je 200 ml Methylenchlorid. Die organischen Phasen werden mit Natriumhydrogencarbonatlösung neutral gewaschen, über Magnesiumsulfat getrocknet, eingeengt und destilliert.
Ausbeute: 77 g (86,6 % der Theorie)
Siedepunkt: 94 bis 100°C/0,15 mbar

### d) 2-Benzyl-4-methyl-2,7-diazabicyclo[3.3.0]octan-7-carbonsäureethylester

Man erhitzt 18,5 g (0,1 mol) N-(2-Buten-1-yl)-N-(2-oxoethyl)-carbamidsäureethylester mit 16,5 g (0,1 mol) N-Benzylglycin in 300 ml Toluol über Nacht am Wasserabscheider unter Rückfluß. Man engt ein und destilliert.
Ausbeute 10 g (25 % der Theorie)
Siedepunkt: 135 bis 142°C/0,1 mbar

Das Produkt ist gaschromatographisch 76 %ig

### e) 2-Benzyl-4-methyl-2,7-diazabicyclo[3.3.0]octan

Man erhitzt 10 g (26,3 mmol) 2-Benzyl-4-methyl-2,7-diazabicyclo[3.3.0]octan-7-carbonsäureethylester mit 100 ml konzentrierter Salzsäure über Nacht unter Rückfluß. Man engt ein, nimmt in 20 ml Wasser auf, stellt mit Kaliumcarbonat alkalisch, extrahiert fünfmal mit je 50 ml Chloroform, trocknet über Kaliumcarbonat, engt ein und destilliert.
Ausbeute: 4,6 g (81 % der Theorie)
Siedepunkt: 87 bis 95°C/0,13 mbar

Das Produkt ist gaschromatographisch 76 %ig.

### f) 4-Methyl-2,7-diazabicyclo[3.3.0]octan

Man hydriert 4,1 g (19 mmol) 2-Benzyl-4-methyl-2_{;}7-diazabicyclo[3.3.0]octan in 80 ml Methanol an 1 g Palladium-Aktivkohle (10 % Pd) bei 100°C und 100 bar. Der Katalysator wird abgesaugt, das Filtrat eingeengt und der Rückstand destilliert.
Ausbeute: 1,2 g (50 % der Theorie)
Siedepunkt: 76°C/8 mbar

### Beispiel V

### 5-Fluormethyl-2-methyl-2,7-diazabicyclo[3.3.0]octan

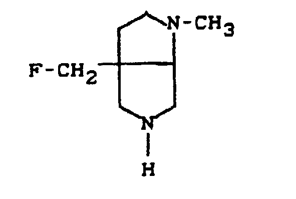

### a) N-(2-Fluormethylallyl)-N-(2,2-dimethoxyethyl)-carbamidsäureethylester

Man legt 8 g (0,26 mol) Natriumhydrid (80 %ig) in 200 ml Toluol vor und tropft bei 90°C 35,8 g (0,2 mol) N-(2,2-Dimethoxyethyl)-carbamidsäureethylester hinzu. Anschließend rührt man eine Stunde bei 90°C und tropft dann 32,6 g (0,3 mol) 1-Chlor-2-fluormethylpropen-2 hinzu. Man rührt über Nacht bei 90°C, löst Salze in Wasser, trennt die wäßrige Phase ab und extrahiert sie mit Toluol. Die organischen Phasen werden über Kaliumcarbonat getrocknet, eingeengt und destilliert.
Ausbeute: 28,2 g (56,6 % der Theorie)
Siedepunkt: 71 bis 79°C/0,07 mbar

### b) N-(2-Fluormethylallyl)-N-(2-oxoethyl)-carbamidsäureethylester

Man erhitzt 25 g (0,1 mol) N-(2-Fluormethylallyl)-N-(2,2-dimethoxyethyl)carbamidsäureethylester mit 5 g Ameisensäure in 100 ml Wasser zwei Stunden unter Rückfluß. Man sättigt mit Kochsalz, extrahiert mit Methylenchlorid und wäscht die organischen Phasen mit Natriumhydrogencarbonatlösung neutral. Man trocknet über Magnesiumsulfat, engt ein und destilliert.
Ausbeute: 18,5 g (87 % der Theorie)
Siedepunkt: 84°C/0,18 mbar

### c) 5-Fluormethyl-2-methyl-2,7-diazabicyclo[3.3.0]octan-7-carbonsäureethylester

Man erhitzt 9,1 g (43 mmol) N-(2-Fluormethylallyl)-N-(2-oxoethyl)-carbamidsäureethylester mit 3,9 g (43 mmol) gepulvertem Sarkosin in 170 ml Toluol über Nacht am Wasserabscheider unter Rückfluß. Man engt ein und destilliert den Rückstand.
Ausbeute: 7,5 g (75,8 % der Theorie)
Siedepunkt: 80 bis 100°C/0,25 bis 0,35 mbar

### d) 5-Fluormethyl-2-methyl-2,7-diazabicyclo[3.3.0]octan

Man erhitzt 7,1 g (26 mmol) 5-Fluormethyl-2-methyl-2,7-diazabicyclo[3.3.0]octan-7-carbonsäureethylester in 100 ml konzentrierter Salzsäure über Nacht unter Rückfluß. Man engt ein, nimmt in 20 ml Wasser auf, stellt mit Kaliumcarbonat alkalisch, extrahiert zehnmal mit je 50 ml Chloroform, trocknet über Kaliumcarbonat, engt ein und destilliert.
Ausbeute: 0,8 g (20 % der Theorie)
Siedepunkt: 34°C/0,07 mbar

### Beispiel W

### 5-Fluor-7-methyl-2.7-diazabicyclo[3.3.0]octan

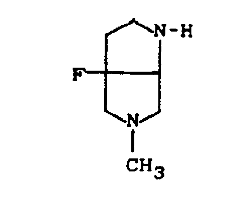

### a) N-(2,2-Dimethoxyethyl)-N-(2-fluorallyl)-carbamidsäureethylester

Man legt 11,6 g (65,5 mmol) N-(2,2-Dimethoxyethyl)-carbamidsäureethylester, 15 g gepulvertes Kaliumhydroxid und 0,25 g Triethylbenzylammoniumchlorid in 65 ml Toluol vor und tropft 10 g (72 mmol) 2-Fluorallylbromid bei Raumtemperatur hinzu. Man rührt über Nacht bei Raumtemperatur, setzt 100 ml Wasser hinzu, trennt die wäßrige Phase ab und extrahiert sie mit 30 ml Toluol. Die organischen Lösungen werden über Magnesiumsulfat getrocknet, eingeengt und der Rückstand destilliert.
Ausbeute: 14,1 g (91,5 % der Theorie)
Siedepunkt: 72°C/0,3 mbar

### b) N-(2-Fluorallyl)-N-(2-oxoethyl)-carbamidsäureethylester

Man erhitzt 14,1 g (60 mmol) N-(2,2-Dimethoxyethyl)-N-(2-fluorallyl)-carbamidsäureethylester mit 6,3 ml Ameisensäure in 120 ml Wasser drei Stunden unter Rückfluß. Man sättigt die Lösung mit Kochsalz, extrahiert mehrfach mit Methylenchlorid, wäscht die organischen Lösungen mit gesättigter Natriumhydrogencarbonatlösung, trocknet über Magnesiumsulfat, engt ein und destilliert den Rückstand.
Ausbeute: 9,8 g (86 % der Theorie)
Siedepunkt: 80°C/0,25 mbar

### c) 2-Benzyl-5-fluor-2,7-diazabicyclo[3.3.0]octan-7-carbonsäureethylester

Man erhitzt 20,8 g (0,11 mol) N-(2-Fluorallyl)-N-(2-oxoethyl)-carbamidsäureethylester mit 19 g (0,115 mol) N-Benzylglycin in 300 ml Toluol bis zum Ende der C0₂-Entwicklung unter Rückfluß. Man engt ein und destilliert.
Ausbeute: 16,4 g (44,8 % der Theorie)
Siedepunkt: 148-152°C/0,1 mbar

### Das Produkt ist gaschromatographisch 88 %ig

### d) 2-Benzyl-5-fluor-7-methyl-2,7-diazabicyclo[3.3.0]octan

Zu 4,3 g (0,11 mol) Lithiumaluminiumhydrid in 125 ml absolutem Tetrahydrofuran tropft man eine Lösung von 16,4 g (49,4 mmol, 88 %ig) 2-Benzyl-5-fluor-2,7-diazabicyclo[3.3.0]octan-7-carbonsäureethylester in 25 ml absolutem Tetrahydrofuran und erhitzt anschließend über Nacht unter Rückfluß. Man zersetzt nacheinander mit je 4,5 ml Wasser, 15 %iger Kalilauge und Wasser, saugt die anorganischen Salze ab und kocht sie dreimal mit je 50 ml Tetrahydrofuran aus. Die organischen Lösungen werden eingeengt und der Rückstand destilliert.
Ausbeute: 11 g (88 % der Theorie)
Siedepunkt: 98-108°C/0,08 mbar

Das Produkt ist gaschromatographisch 93 %ig.

### e) 5-Fluor-7-methyl-2,7-diazabicyclo[3.3.0]octan

Man hydriert 11 g (43,7 mmol, 93 %ig) 2-Benzyl-5-fluor-7-methyl-2,7-diazabicyclo[3.3.0]octan in 100 ml Ethanol an 2 g Palladium-Aktivkohle (10 % Pd) bei 100°C und 100 bar. Man saugt den Katalysator ab, engt das Filtrat ein und destilliert den Rückstand.
Ausbeute: 4,4 g (69,8 % der Theorie)
Siedepunkt; 85-90°C/25 mbar

### Beispiel X

### 6-Methyl-2,7-diazabicyclo[3.3.0]octan-7-carbonsäureethylester

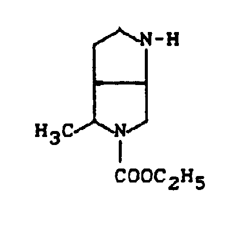

### a) N-(1-Buten-3-yl)-N-(2,2-dimethoxyethyl)-carbamidsäureethylester

Zu 35,5 g (0,2 mol) N-(2,2-Dimethoxyethyl)-carbamidsäureethylester und 26 g gepulvertem Kaliumhydroxid in 400 ml Dimethylformamid gibt man 22 g (0,24 mol) 3-Chlor-1-buten und erwärmt über Nacht auf 40°C. Man löst die Salze mit Wasser und extrahiert mehrfach mit Methylenchlorid. Die organischen Extrakte werden über Kaliumcarbonat getrocknet, eingeengt und destilliert.
Ausbeute: 28,5 g (61,6 % der Theorie)
Siedepunkt: 60°C/0,08 mbar

### b) N-(1-Buten-3-yl)-N-(2-oxoethyl)-carbamidsäureethylester

Man erhitzt 28,3 g (0,122 mol)N-(1-Buten-3-yl)-N-(2,2-dimethoxyethyl)-carbamidsäureethylester mit 65 ml Ameisensäure eine Stunde auf 100°C. Man gießt auf 200 g Eis, extrahiert mit Methylenchlorid, wäscht die organischen Extrakte mit gesättigter Natriumhydrogencarbonatlösung, trocknet über Magnesiumsulfat, engt ein und destilliert.
Ausbeute: 11,6 g (51,3 % der Theorie)
Siedepunkt: 62-65°C/0,03 mbar

### c) 2-Benzyl-6-methyl-2,7-diazabicyclo[3.3.0]octan-7-carbonsäureethylester

Man erhitzt 11,6 g (62,6 mmol) N-(1-Buten-3-yl)-N-(2-oxoethyl)-carbamidsäureethylester und 10,4 g (62,6 mmol) N-Benzylglycin in 170 ml Toluol über Nacht am Wasserabscheider unter Rückfluß. Man engt ein und destilliert.
Ausbeute 13,7 g (75,9 % der Theorie)
Siedepunkt: 140-153°C/0,1 mbar.

### d) 6-Methyl-2,7-diazabicyclo[3.3.0]octan-7-carbonsäureethylester

Man hydriert 13 g (44,9 mmol) 2-Benzyl-6-methyl-2,7-diazabicyclo[3.3.0]octan-7-carbonsäureethylester in 150 ml Ethanol an 2 g Palladium-Aktivkohle (10 % Pd) bei 100°C und 100 bar. Der Katalysator wird abfiltriert, das Filtrat eingeengt und destilliert.
Ausbeute 6,8 g (76,4 % der Theorie)
Siedepunkt: 81°C/0,09mbar

### Beispiel Y

### 2,7-Diazabicyclo[3.3.0]octan-3,7-dicarbonsäurediethylester

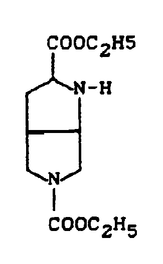

### a) 2-Benzyl-2,7-diazabicyclo[3.3.0]octan-3,7-dicarbonsäurediethylester

Man erhitzt 50 g (0,25 mol) N-Benzylglycinethylester in 1 1 Toluol am Wasserabscheider unter Rückfluß und tropft innerhalb von zwei Stunden 43 g (0,25 mol) N-Allyl-N-(2-oxoethyl)-carbamidsäureethylester hinzu Man erhitzt unter Rückfluß bis kein Wasser mehr abgeschieden wird, engt ein und destilliert den Rückstand.
Ausbeute: 82,1 g (94,8 % der Theorie)
Siedepunkt: 160-165°C/0,05 mbar

### b) 2,7-Diazabicyclo[3,3,0]octan-3,7-dicarbonsäurediethylester

Man hydriert 96,5 g (0,279 mol) 2-Benzyl-2,7-diazabicyclo[3.3.0]octan-3,7-dicarbonsäurediethylester in 1 Ethanol an 5 g Palladium-Aktivkohle (10 % Pd) bei 100°C und 100 bar. Man saugt ab, engt ein und destilliert.
Ausbeute: 63,3 g (84,6 % der Theorie)
Siedepunkt: 137-140°C/0,18-0,2 mbar

### Beispiel Z

### 5-Methyl-2,7-diazabicyclo[3.3.0]octan-7-carbonsäure-tert.-butylester

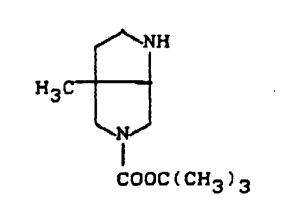

### a) N-(2-Hydroxyethyl)-N-methallylcarbamidsäure-tert.-butylester

Man legt 34,5 g (0,3 mol) N-Methallylethanolamin und 46,2 ml (0,33 mol) Triethylamin in 300 ml Toluol vor und tropft 71,2 g (0,32 mol) Pyrokohlensäure-tert.-butylester hinzu. Man rührt über Nacht bei Raumtemperatur, wäscht mit gesättigter Kochsalzlösung, trocknet über Kaliumcarbonat, engt ein und destilliert.
Ausbeute: 57,1 g (83% der Theorie)
Siedepunkt: 93°C/0,25 mbar

### Das Produkt ist gaschromatographisch 94%ig.

### b) N-Methallyl-N-(2-oxoethyl)-carbamidsäure-tert.-butylester

Man legt 49,4 ml (0,59 ml) Oxalylchlorid in 480 ml absolutem Methylenchlorid vor und tropft 83,3 g (1,07 mol) Dimethylsulfoxid in 120 ml absolutem Methylenchlorid bei -60°C hinzu. Anschließend werden 56,6 g (0,25 mol) N-(2-Hydroxyethyl)-N-methallylcarbamidsäure-tert.-butylester in 120 ml absolutem Methylenchlorid bei -30°C zugetropft. Ebenfalls bei -30°C werden dann 169 ml (1,23 mol) Triethylamin zugetropft. Man läßt auf Raumtemperatur kommen, gießt auf 1,2 1 Eiswasser, trennt die organische Phase ab und extrahiert die wäßrige Phase mit Methylenchlorid. Man wäscht die organischen Lösungen mit Kochsalzlösung, trocknet über Magnesiumsulfat, engt ein und destilliert.
Ausbeute: 38,9 g (71% der Theorie)
Siedepunkt: 75°C/0,18 mbar

### c) 2-Benzyl-5-methyl-2,7-diazabicyclo[3.3.0]octan-7-carbonsäure-tert.-butylester

Man erhitzt 10,9 g (50 mmol) N-Methallyl-N-(2-oxoethyl)-carbamidsäure-tert.-butylester und 8,3 g (50 mmol) N-Benzylglycin in 150 ml Toluol 15 Stunden am Wasserabscheider unter Rückfluß. Man engt ein und destilliert.
Ausbeute: 7,2 g (36% der Theorie)
Siedepunkt: 142°C/0,35 mbar

Das Produkt ist gaschromatographisch 80%ig.

### d) 5-Methyl-2,7-diazabicyclo[3.3.0)octan-7-carbonsäure-tert.-butylester

Man hydriert 2,6 g (10,3 mmol) 2-Benzyl-5-methyl-2;7-diazabicyclo[3.3.0]octan-7-carbonsäure-tert.-butylester in 50 ml Ethanol an 0,5 g Palladium-Aktivkohle (10%Pd) bei 130°C und 100 bar. Man saugt den Katalysator ab, engt ein und destilliert.
Ausbeute: 0,9 g (38,6% der Theorie)
Siedepunkt: 76°C/0,07 mbar

### Beispiel ZA

### 5-Brom-2-methyl-2, 7-diazab icyc l0[3. 3.0]octan

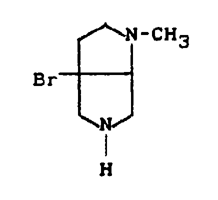

### a) N-(2-Bromallyl)-N-(2,2-dimethoxyethyl)-carbamidsäureethylester

Man legt 88,5 g (0,5 mol) N-(2,2-Dimethoxyethyl)-carbamidsäure-ethylester, 83 g gepulvertes Kaliumhydroxyd und 2 g Triethylbenzylammoniumchlorid in 500 ml Toluol vor und tropft 110 g (0,55 mol) 2,3-Dibrompropen hinzu. Man rührt über Nacht bei Raumtemperatur, saugt die Salze ab, wäscht das Filtrat mit gesättigter Kochsalzlösung, trocknet über Magnesiumsulfat, engt ein und destilliert über eine Vigreux-Kolonne.
Ausbeute: 53,7 (36 % der Theorie)
Siedepunkt: 70°C/0,07 mbar

### b) N-(2-bromallyl)-N-(2-oxoethyl)-carbamidsäureethylester

Man erhitzt 54 g (0,18 mol) N-(2-Bromallyl)-N-(2,2-dimethoxyethyl)-carbamidsäureethylester mit 20 ml Ameisensäure in 400 ml Wasser drei Stunden unter Rückfluß. Man sättigt mit Kochsalz, trennt die wäßrige Phase ab und extrahiert sie zweimal mit je 150 ml Methylenchlorid. Die organischen Lösungen werden mit gesättigter Natriumhydrogencarbonatlösung neutral gewaschen, über Magnesiumsulfat getrocknet, eingeengt und destilliert.
Ausbeute: 38,9 g (84,4 % der Theorie)
Siedepunkt: 106-109°C/0,25 mbar

### c) 5-Brom-2-melhyl-2,7-diazabicyclo[3.3.0]loctan-7-carbonsäure-ethylester

Man erhitzt 25 g (0,1 mol) N-(2-Bromallyl)-N-(2-oxoethyl)-carbamidsäure-ethylester mit 9 g (0,1 mol) Sarkosin in 400 ml Toluol über Nacht am Wasserabscheider unter Rückfluß. Man engt ein und destilliert den Rückstand.
Ausbeute 19,4 g (64 % der Theorie)
Siedepunkt: 95-96°C/0,04 mbar

### Das Produkt ist gaschromatographisch 91,4 %ig.

### d) 5-Brom-2-methyl-2,7-diazobicyclo[3,3,0]octan

Man erhitzt 18,4 g (60,7 mmol, 91,4 %ig) 5-Brom-2-methyl-2,7-diazabicyclo[3.3.0]octan-7-carbonsäureethy- ester mit 120 ml konzentrierter Salzsäure über Nacht unter Rückfluß. Man engt ein, nimmt in 50 ml Wasser auf, stellt mit Kaliumcarbonat alkalisch und extrahiert fünfmal mit je 100 ml Chloroform. Man trocknet über Kaliumcarbonat, engt ein und destilliert.
Ausbeute: 7,2 g (54 % der Theorie)
Siedepunkt: 90°C/4 mbar

### Beispiel ZB

### 2,1-Diazabicyclo[3.3.0]octan-1-carbonsäureethylester

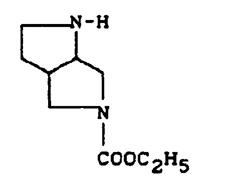

Man hydriert 21,2 g (77,3 mmol) 2-Benzyl-2,7-diazabicyclo[3.3.0]octan-7-carbonsäureethylester (Beispiel Ae) in 400 ml Ethanol an 3 g Palladium-Aktivkohle (10 % Pd) bei 100°C und 100 bar. Der Katalysator wird abfiltriert, das Filtrat eingeengt und der Rückstand destilliert.
Ausbeute: 10,3 g (72,3 % der Theorie)
Siedepunkt: 82 - 92°C / 0,1 mbar Herstellung der Wirkstoffe

### Beispiel 1

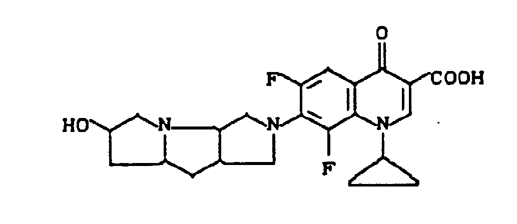

A. 2,85 g (10 mmol) 1-Cyclopropyl-6,7,8-trifluor-1,4-dihydro-4-oxo-3-chinolincarbonsäure werden in einer Mischung aus 30 ml Acetonitril und 15 ml Dimethylformamid in Gegenwart von 1,9 g (17 mmol) 1,4-Diazabicyclo [2.2.2]octan mit 2,0 g (12 mmol) 10-Hydroxy-1,4-diazatricyclo[6.3.0.0^{2,6}]undecan 3 Stunden unter Rückfluß erhitzt. Die Mischung wird eingeengt, der Rückstand mit Wasser verrührt, der ungelöste Niederschlag abgesaugt, mit Wasser gewaschen und getrocknet.

Ausbeute: 3,1 g (72% der Theorie) 1-Cyclopropyl-6,8-difluor-1,4-dihydro-7-(10-hydroxy-1,4-diazatricyclo [6.3.0.0^{2,6}]undec-4-yl)-4-oxo-3-chinolincarbonsäure, Schmelzpunkt: 242-245°C (unter Zersetzung).

B. 3 g (7 mmol) des Betains aus Stufe A werden in 20 ml in-Salzsäure unter Erwärmen gelöst, die Lösung filtriert, eingedampft und der Rückstand mit Ethanol verrührt. Das erhaltene Salz wird abgesaugt, mit Ethanol gewaschen und bei 120°C/12 mbar getrocknet.

Ausbeute: 3,1 g (95% der Theorie) 1-Cyclopropyl-6,8-difluor-1,4-dihydro-7-(10-hydroxy-1,4-diazatricyclo [6.3.0.0^{2,6}]undec-4-yl)-4-oxo-3-chinolincarbonsäure-Hydrochlorid,Schmelzpunkt: 267-275°C (unter Zersetzung).

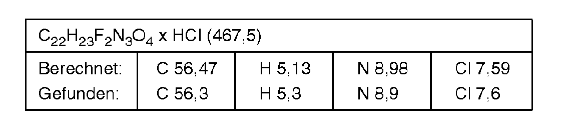

Entsprechend Beispiel 1 werden hergestellt:

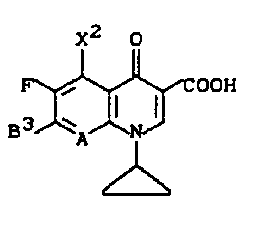

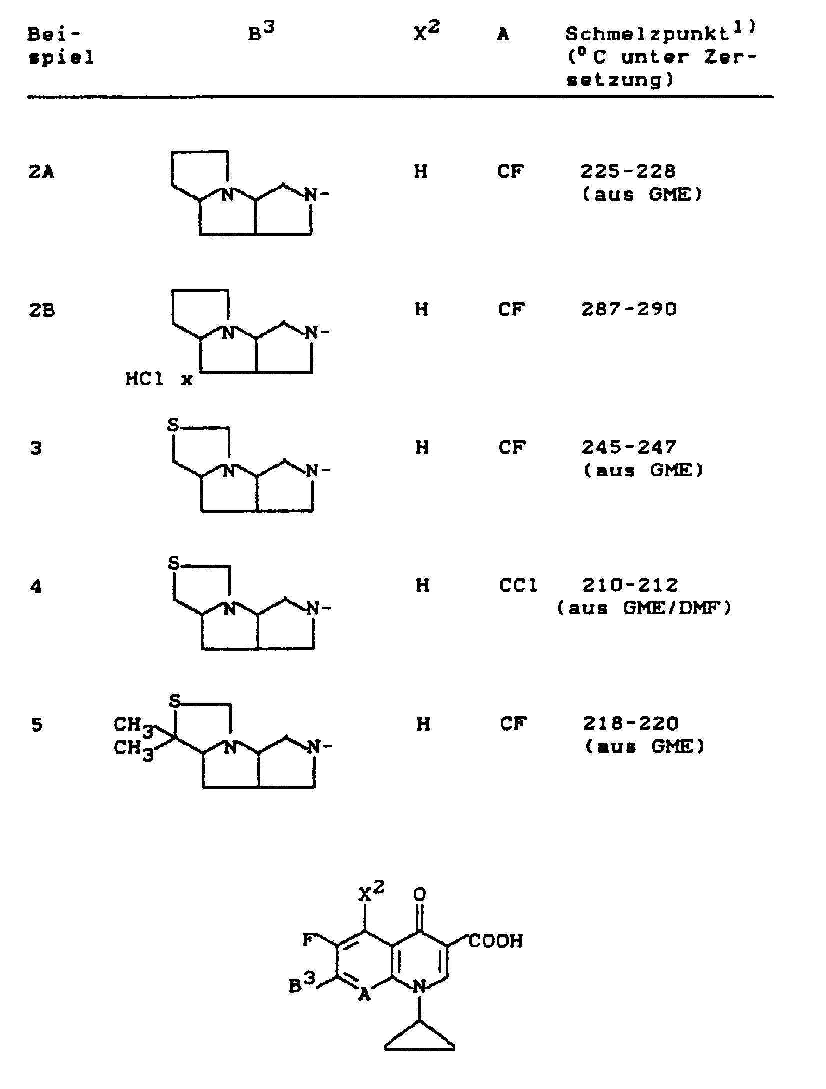

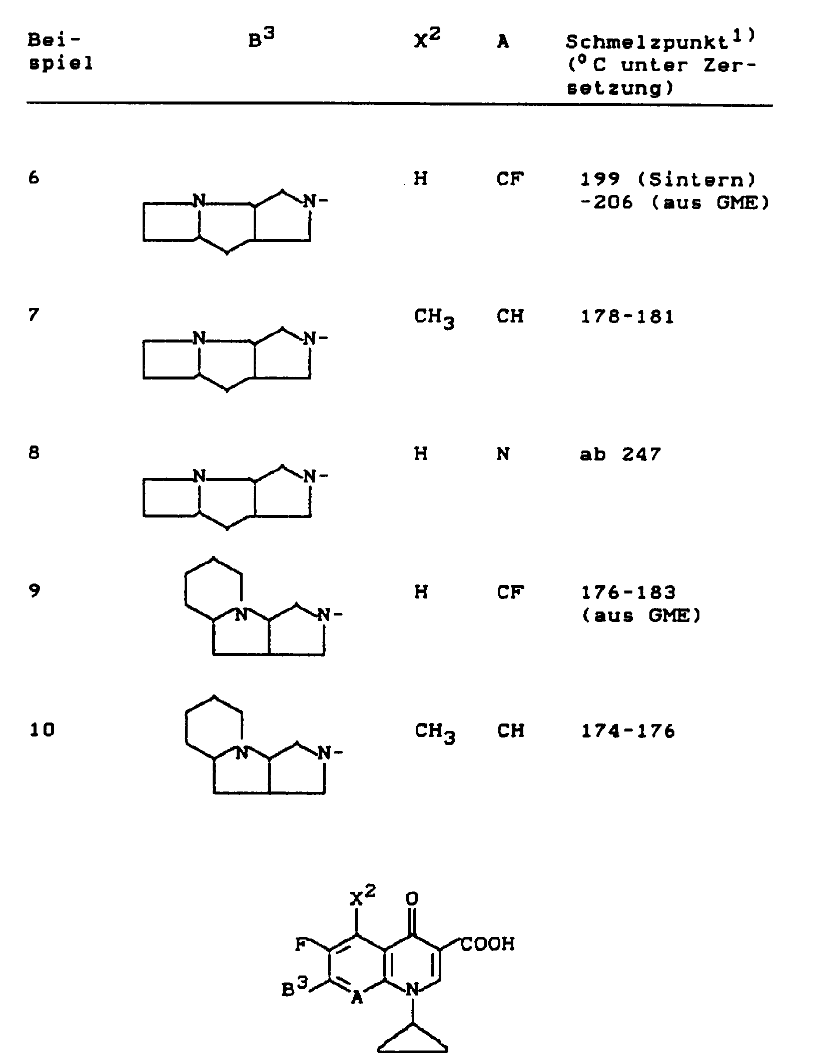

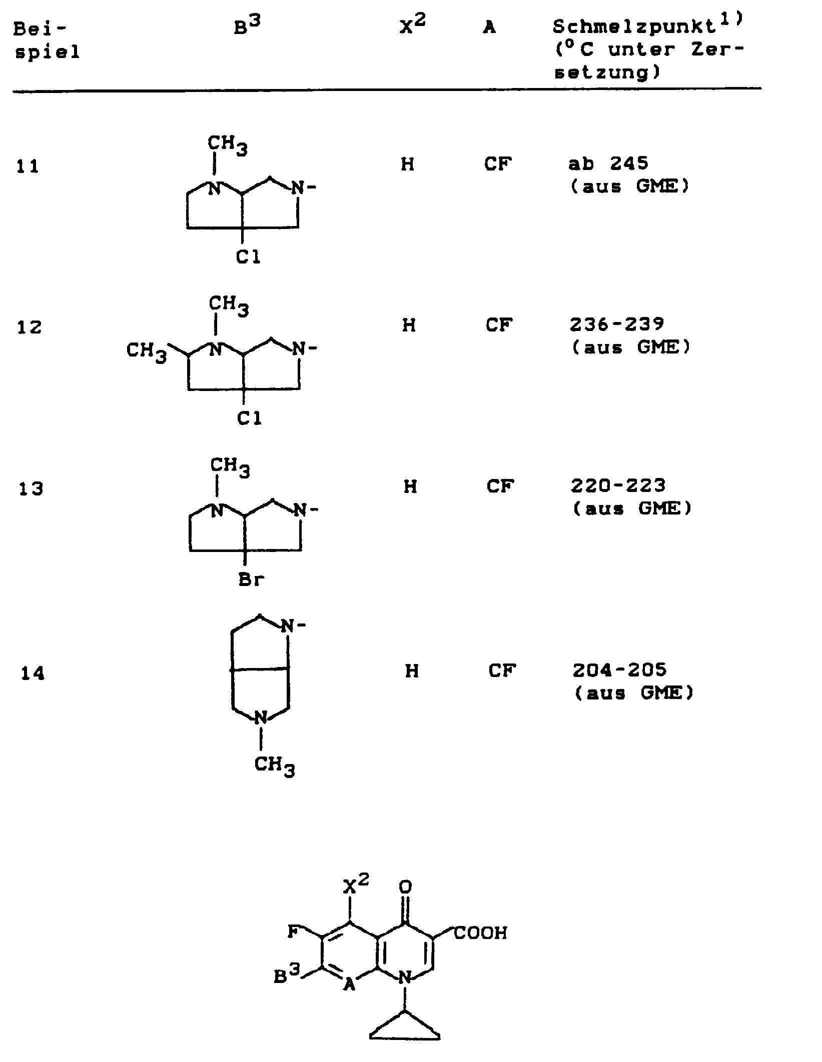

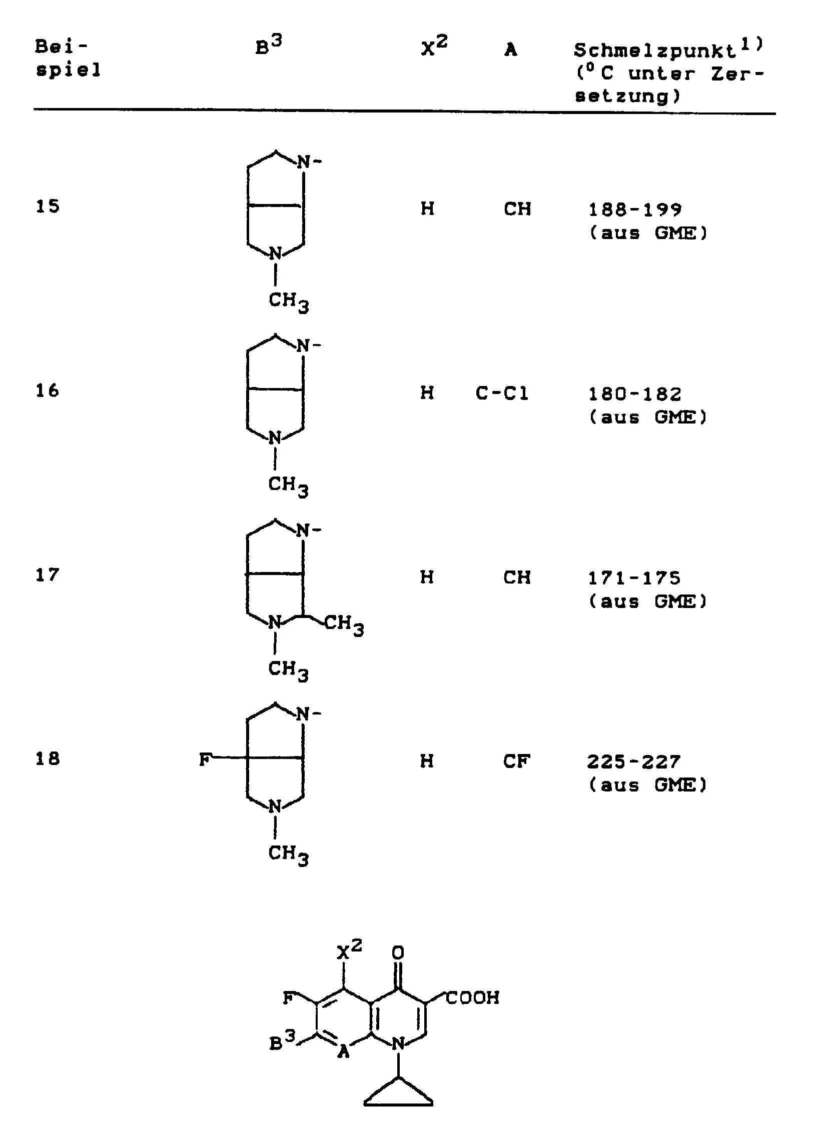

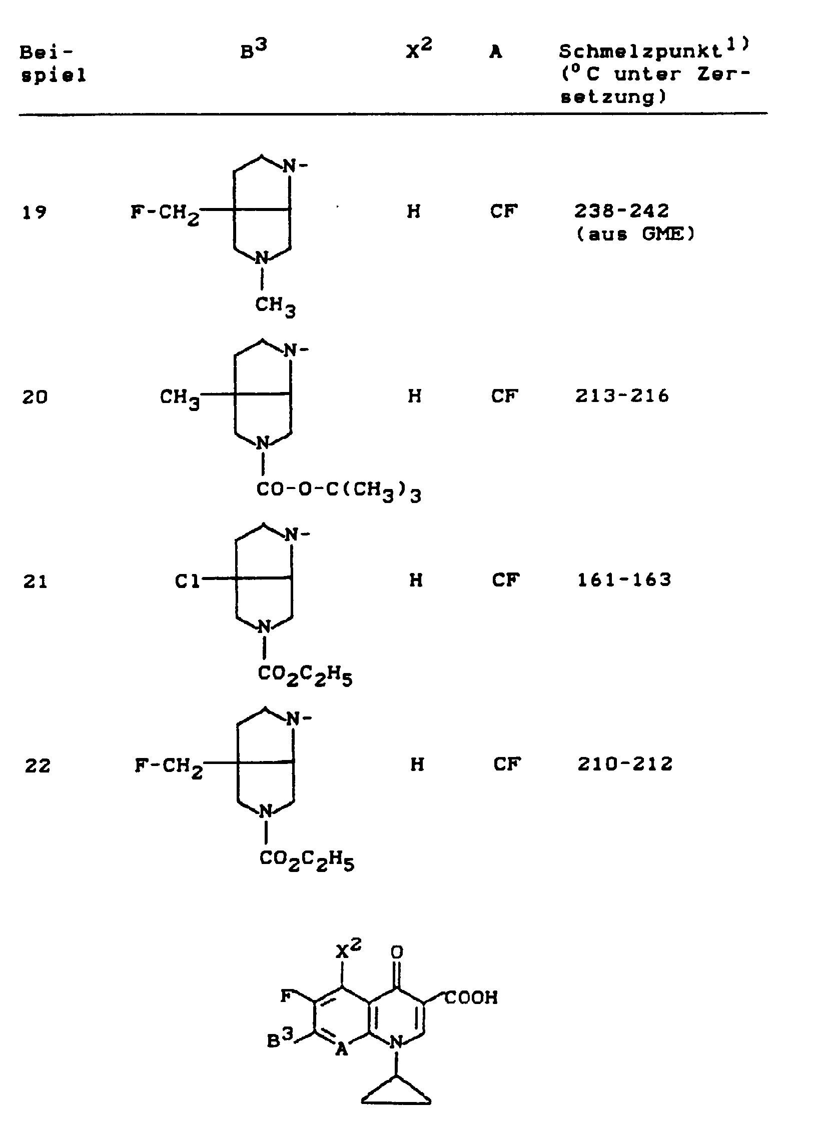

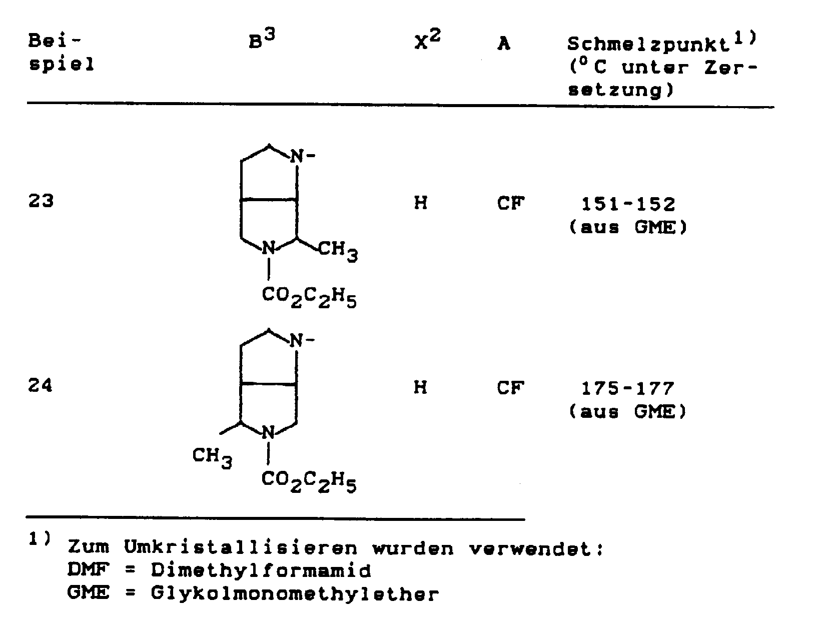

### Beispiel 25

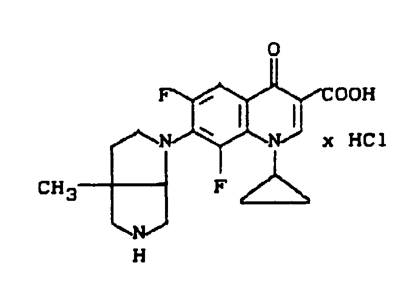

260 mg (0,53 mmol) 7-(7-tert.-Butoxycarbonyl-5-methyl-2,7-diazabicyclo[3.3.0]oct-2-yl)-1-cyclopropyl-6,8-difluor-1,4-dihydro-4-oxo-3-chinolincarbonsäure (aus Beispiel 20) werden in einer Mischung aus 1,5 ml Methanol und 2,5 ml konzentrierter Salzsäure etwa 10 Minuten auf 80°C erhitzt. Die klare Lösung wird eingeengt, der Rückstand mit Ethanol verrührt, der Niederschlag abgsaugt, mit Ethanol gewaschen und bei 80°/1 mbar getrocknet.

Ausbeute: 150 mg (66,5% der Theorie) 1-Cyclopropyl-6,8-difluor-1,4-dihydro-7-(5-methyl-2,7-diazabicyclo[3.3.0] oct-2-yl)-4-oxo-3-chinolincarbonsäure-Hydrochlorid, Schmelzpunkt: 278-279°C (unter Zersetzung).

### Beispiel 26

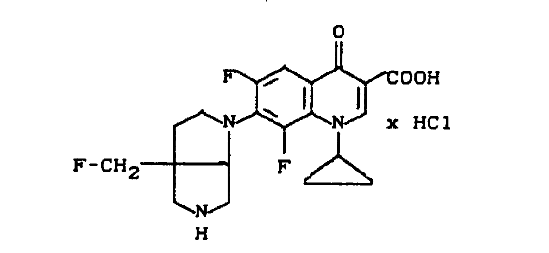

3 g (6,3 mmol) 1-Cyclopropyl-7-(7-ethoxycarbonyl-5-fluormethyl-2,7-diazabicyclo[3.3.0]oct-2-yl)-6,8-difluor-1,4-dihydro-4-oxo-3-chinolincarbonsäure (aus Beispiel 22) werden in 60 ml konzentrierter Salzsäure 8 Stunden unter Rückfluß erhitzt. Die Suspension wird eingeengt, der Rückstand mit Ethanol verrührt, abgesaugt, mit Ethanol gewaschen und bei 100°C/12 mbar getrocknet

Ausbeute: 1,7 g (61% der Theorie) 1-Cyclopropyl-6,8-difluor-7-(5-fluormethyl-2,7-diazabicyclo[3.3.0]oct-2-yl)-1,4-dihydro-4-oxo-3-chinolincarbonsäure-Hydrochlorid, Schmelzpunkt: 253-261°C (unter Zersetzung); nach Umkristallisation aus Glykolmonomethylether: Schmelzpunkt: 263-267°C (unter Zersetzung).

Entsprechend Beispiel 26 werden hergestellt:

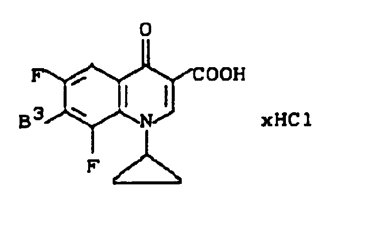

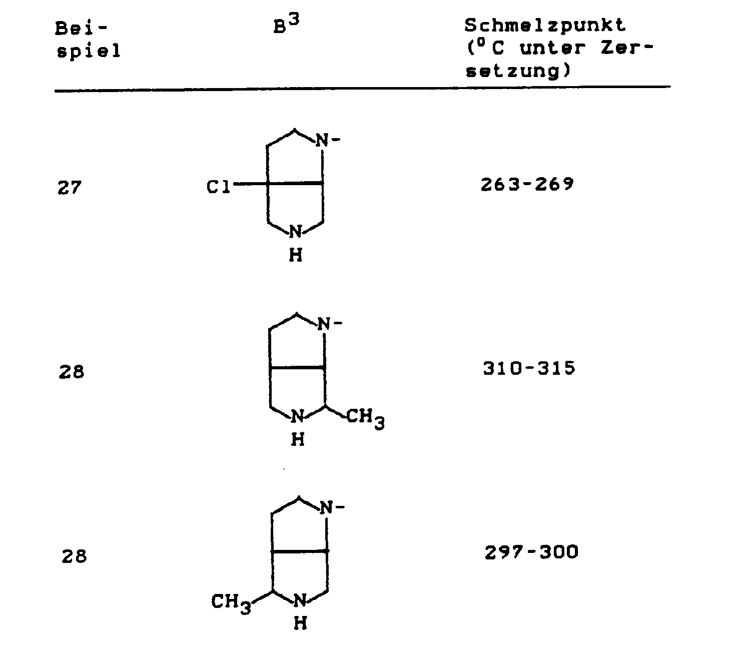

### Beispiel 29

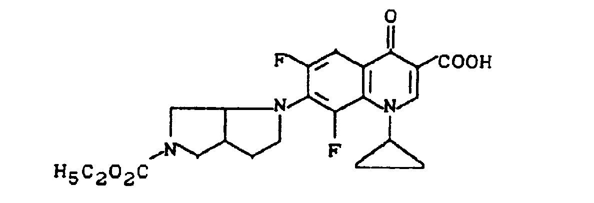

Analog Beispiel 1 wird mit 2,7-Diazabicyclo[3.3.0]octan-7-carbonsäureethylester zu 1-Cyclopropyl-7-(7-ethoxycarbonyl-2,7-diazabicyclo[3.3.0]oct-2-yl)-6,8-difluor-1,4-dihydro-4-oxo-3-chinolincarbonsäure vom Schmelzpunkt 192-193°C umgesetzt.

### Beispiel 30

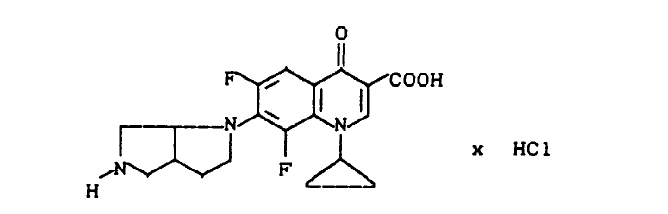

2,5 g des Produkts aus Beispiel 29 werden in 50 ml konzentrierter Salzsäure 17 Stunden unter Rückfluß erhitzt. Die Lösung wird eingeengt, der Rückstand mit Ethanol versetzt und das ungelöste Festprodukt abgesaugt, mit Ethanol gewaschen und bei 100°C im Vakuum getrocknet.

Ausbeute: 1,3 g 1-Cyclopropyl-7-(2,7-diazabicyclo[3.3.0]oct-2-yl)-6,8-difluor-1,4-dihydro-4-oxo-3-chinolincarbon- säure-Hydrochlorid, Schmelzpunkt: 275-278°C (unter Zersetzung); Massenspektrum: m/e 375 (M⁺), 332 (100 %, M⁺ - 43), 331 (M⁺ - CO₂), 315, 289, 274.

## Patentansprüche (Patentansprüche für folgende(n) Vertragsstaat(en) : AT, BE, CH, DE, DK, FR, GB, IT, LI, LU, NL, SE)

1. 7-(2,7-Diazabicyclo[3.3.0]octyl)-3-chinolon- und -naphthyridoncarbonsäure-Derivate der Formel (I) 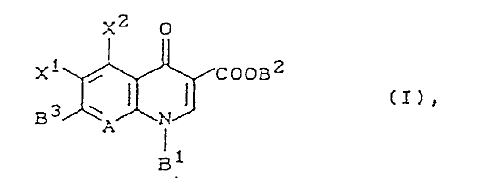 in welcher
X¹ für Halogen,
X² für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen, Methyl,
B¹ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
B² für Wasserstoff, Alkyl mit 1 bis 4 Kohlenstoffatomen oder (5-Methyl-2-oxo-1,3-dioxol-4-yl)-methyl,
B³ für einen Rest der Struktur 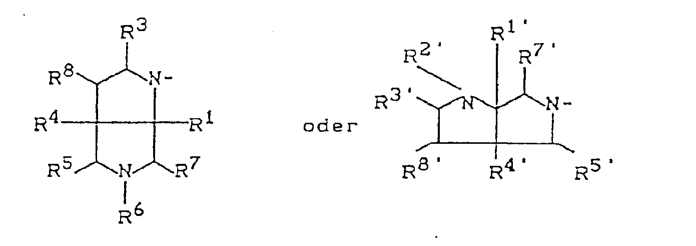 worin
R¹, R³, R⁴, R⁵, R⁷ und R⁸ gleich oder verschieden sind und jeweils für Wasserstoff oder Methyl, das gegebenenfalls durch Halogen oder Hydroxy substituiert sein kann, C₁-C₂-Alkoxycarbonyl oder Carboxy stehen,
R⁴ und R⁸ zusätzlich für Halogen, Amino, Hydroxy oder Methoxy stehen können,
R⁶ für Wasserstoff, C₁-C₆-Alkyl, Benzyl, C₆-C₁₂-Aryl, C₁-C₃-Alkanoyl, Benzoyl, C₁-C₅-Alkoxycarbonyl steht,
R^{1'}, R^{2'}, R^{3'}, R5, R^{7'} und R^{8'} gleich oder verschieden sind und jeweils für Wasserstoff oder Methyl stehen,
R⁴' für Halogen, Amino, Hydroxy, Methoxy oder für durch Halogen, Amino, Hydroxy oder Methoxy substituiertes Methyl steht oder dann für Wasserstoff steht, wenn
R²' und R³' zusammen eine C₂-C₄-Alkylenbrücke oder eine CH₂-S-CH₂-Brücke bedeuten, die gegebenenfalls durch Hydroxy, Methoxy, Amino oder Methyl ein-oder zweifach substituiert sein kann,
A für N oder C-R⁹ steht, worin
R⁹ für H, Halogen, Methyl, Cyano, Nitro, Hydroxy oder Methoxy steht
und deren pharmazeutisch verwendbaren Hydrate und Säureadditionssalze sowie deren Alkali-, Erdalkali-, Silber-und Guanidiniumsalze.

2. Verbindungen gemäß Anspruch 1 in welchen
X¹ für Fluor oder Chlor,
X² für Wasserstoff, Amino, Alkylamino mit 1 bis 2 Kohlenstoffatomen, Dimethylamino, Hydroxy, Methoxy, Mercapto, Methylthio, Fluor, Methyl,
B¹ für Alkyl mit 1 bis 3 Kohlenstoffatomen, Alkenyl mit 2 bis 3 Kohlenstoffatomen, Cycloalkyl mit 3 bis 5 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
B² für Wasserstoff, Alkyl mit 1 bis 3 Kohlenstoffatomen oder (5-Methyl-2-oxo-1,3-dioxol-4-yl)-methyl,
B³ für einen Rest der in Anspruch 1 beschriebenen Struktur
stehen, worin
R¹, R³, R⁴, R⁵, R⁷ und R⁸ gleich oder verschieden sind und jeweils für Wasserstoff oder Methyl, das gegebenenfalls du rch Halogen substituiert sein kann, C₁-C₃-Alkoxycarbonyl stehen,
R⁴ zusätzlich für Halogen, wie Fluor, Chlor oder Brom stehen kann,
R⁶ für Wasserstoff, Cᵢ-C₃-Alkyl, Benzyl, Phenyl, Acetyl, Benzoyl, C₁-C₄-Alkoxycarbonyl steht,
R^{1'}, R^{2'}, R^{3'}, R^{5'}, R^{7'} und R⁸ gleich oder verschieden sind und jeweils für Wasserstoff oder Methyl stehen,
R⁴' für Halogen wie Chlor oder Brom steht oder dann für Wasserstoff steht, wenn
R²' und R³' zusammen eine C₂-C₄-Alkylenbrücke oder eine CH₂-S-CH₂-Brücke bedeuten, die gegebenenfalls durch Hydroxy oder Methyl ein-oder zweifach substituiert sein kann,
A für N oder C-R⁹ steht, worin
_{R}⁹ für H, Halogen oder Methoxy steht
und deren pharmazeutisch verwendbaren Hydrate und Saureadditionssalze sowie deren Alkali- und Erdalkalisalze.

3. Verbindungen gemäß Anspruch 1, in welchen
X¹ für Fluor,
X² für Wasserstoff, Amino, Fluor, Methyl,
B¹ für Alkyl mit 1 bis 2 Kohlenstoffatomen, Vinyl, Cyclopropyl, 2-Hydroxyethyl, 2-Fluorethyl, Methoxy, Methylamino, 4-Fluorphenyl, 2,4-Difluorphenyl,
B² für Wasserstoff, Alkyl mit 1 bis 2 Kohlenstoffatomen,
B³ für einen Rest der in Anspruch 1 beschriebenen Struktur steht,
worin
R¹, R³, R⁴, R⁵, R⁷ und R⁸ gleich oder verschieden sind und jeweils für Wasserstoff oder Methyl steht, das gegebenenfalls durch Fluor substituiert sein kann,
R⁴ zusätzlich für Fluor, Chlor oder Brom stehen kann,
R⁶ für Wasserstoff, Methyl, Acetyl, C₁-C₄-Alkoxycarbonyl steht,
R¹', R²', R³', R⁵', R⁷' und R8 gleich oder verschieden sind und jeweils für Wasserstoff oder Methyl stehen,
R⁴' für Chlor oder Brom steht oder dann für Wasserstoff steht, wenn
R²' und R³' zusammen eine C₂-C₄-Alkylenbrücke oder eine CH₂-S-CH₂-Brücke bedeuten, die gegebenenfalls durch Hydroxy oder Methyl ein-oder zweifach substituiert sein kann, A für N oder C-R⁹ steht, worin
_{R}⁹ für H, Halogen oder Methoxy steht
und deren pharmazeutisch verwendbaren Hydrate und Säureadditionssalze sowie deren Alkalisalze.

4.1-Cyclopropyl-6,8-difluor-1,4-dihydro-7-(7-methyl-2,7-diazabicycl0[3,3.0]oct-2-yl)-4-oxo-3-chinolincarbonsäure der Formel

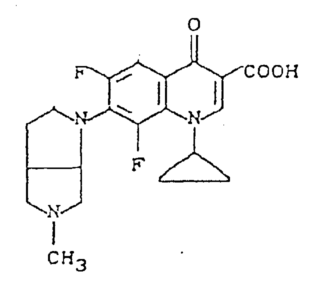

5. 8-Chlor-1-cyclopropyl-6-fluor-1,4-dihydro-7-(7-methyl-2,7-diazabicyclo[3,3,0]oct-2-yl)-4-oxo-3-chinolincarbon- säure der Formel

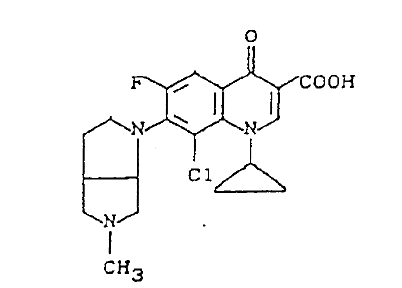

6. 1-Cyclopropyl-6,8-difluor-1,4-dihydro-7-(5-chlor-2,7-diazabicyclo[3,3,0]oct-2-yl)-4-oxo-3-chinolincarbonsäureder Formel

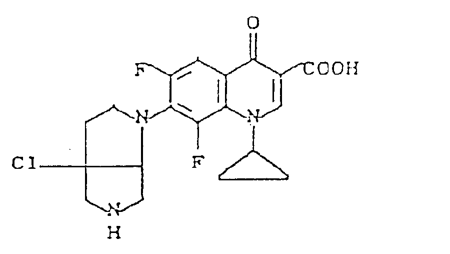

7. 1-Cyclopropyi-7-(1,4-diazatricyclo[6.2.0.0^{2,6}]dec-4-yl)-6,8-diflur-1,4-dihydro-4-oxo-3-chinolincarbonsäure der

Formel

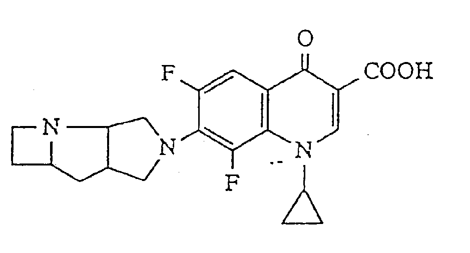

8. 1-Cyclopropyl-7-(2,7-diazabicyclo[3.3.0]oct-2-yl)-6,8-diflur-1,4-dihydro-4-oxo-3-chinolincarbonsäure der Formel

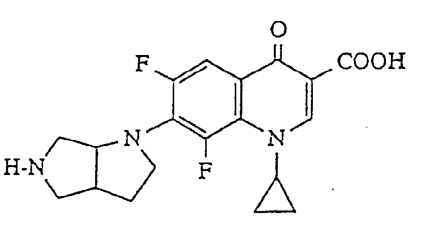

9. Verfahren zur Herstellung von Verbindungen gemäß Anspruch 1, dadurch gekennzeichnet, daß an Verbindungen der Formel (II)

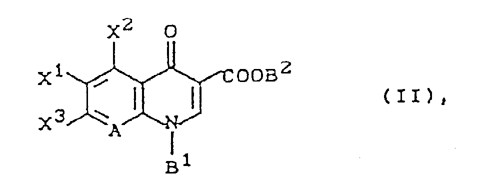

in welcher
A, B¹, B², X¹ und X² die in Anspruch 1 angegebene Bedeutung haben und
X³ für Halogen, insbesondere Fluor oder Chlor steht, mit Verbindungen der Formel (III) 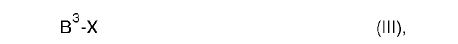
in welcher
B³ die in Anspruch 1 angegebene Bedeutung hat,
gegebenenfalls in Gegenwart von Säurefängern umsetzt, und gegebenenfalls in B³ enthaltene Schutzgruppen abspaltet, wobei solche Verbindungen der Formel (I)
in welcher
X¹, B¹, B², B³ und A die in Anspruch 1 angegebene Bedeutung haben und
X² für Amino, Alkylamino mit 1 bis 4 Kohlenstoffatomen Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen oder Arylthio steht,
auch erhalten werden können, indem man eine Verbindung der Formel (IV) 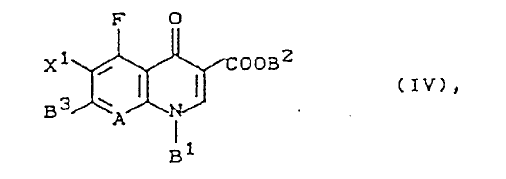 in welcher
X¹, B¹, B², B³ und A die in Anspruch 1 angegebene Bedeutung haben,
mit Verbindungen der Formel (V) 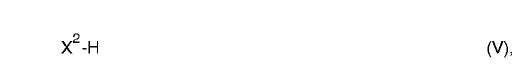
in welcher
X² die oben angegebene Bedeutung hat, gegebenenfalls in Gegenwart von Säurefängern umsetzt,

10. Arzneimittel enthalten Verbindungen gemäß Ansprüchen 1 - 8.

11. Antibiotische Mittel enthaltend Verbindungen gemäß Ansprüchen 1 - 8.

12. Verwendung von Verbindungen gemäß Ansprüchen 1 - 8 zur Herstellung von Arzneimitteln.

13. Verwendung von Verbindungen gemäß Ansprüchen 1 - 8 als Tierfutterzusatzmittel.

14. Tierfutter bzw. Tierfutterzusatzmittel und Prämixe enthaltend Verbindungen gemäß Ansprüchen 1 - 8.

## Patentansprüche (Patentansprüche für folgende(n) Vertragsstaat(en) : ES)

1. Verfahren zur Herstellung von 7-(2,7-Diazabicyclo[3.3.0]octyl)-3-chinolon- und -naphthyridoncarbonsäure-Derivate der Formel (I)

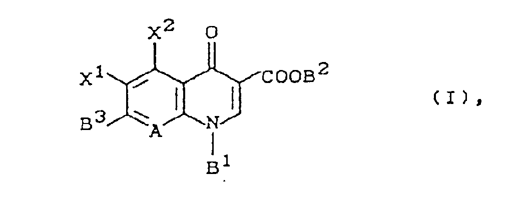

in welcher
X¹ für Halogen, X² für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen, Methyl,
B¹ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
B² für Wasserstoff, Alkyl mit 1 bis 4 Kohlenstoffatomen oder (5-Methyl-2-oxo-1,3-dioxol-4-yl)-methyl,
B³ für einen Rest der Struktur 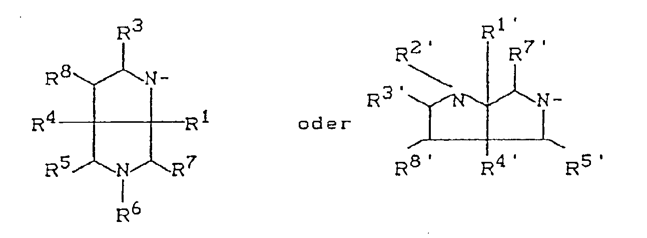 worin
R¹, R³, R⁴, R⁵, R⁷ und R⁸ gleich oder verschieden sind und jeweils für Wasserstoff oder Methyl,
das gegebenenfalls durch Halogen oder Hydroxy substituiert sein kann, C₁-C₂-Alkoxycarbonyl oder Carboxy stehen,
R⁴ und R⁸ zusätzlich für Halogen, Amino, Hydroxy oder Methoxy stehen können,
R⁶ für Wasserstoff, C₁-C₆-Alkyl, Benzyl, C₆-Cᵢ₂-Aryl, C₁-C₃-Alkanoyl, Benzoyl, C₁-C₅-Alkoxycarbonyl steht,
R¹', R²', R³', R⁵', R⁷' und R8 gleich oder verschieden sind und jeweils für Wasserstoff oder Methyl stehen,
R⁴' für Halogen, Amino, Hydroxy Methoxy oder für durch Halogen, Amino, Hydroxy oder Methoxy substituiertes Methyl steht oder dann für Wasserstoff steht, wenn
R²' und R³' zusammen eine C₂-C₄-Alkylenbrücke oder eine CH₂-S-CH₂-Brücke bedeuten, die gegebenenfalls durch Hydroxy, Methoxy, Amino oder Methyl ein-oder zweifach substituiert sein kann,
A für N oder C-R⁹ steht, worin
R⁹ für H, Halogen, Methyl, Cyano, Nitro, Hydroxy oder Methoxy steht
und deren pharmazeutisch verwendbaren Hydrate und Säureadditionssalze sowie deren Alkali-, Erdalkali-, Silber-und Guanidiniumsalze,
dadurch gekennzeichnet, daß man Verbindungen der Formel (II) 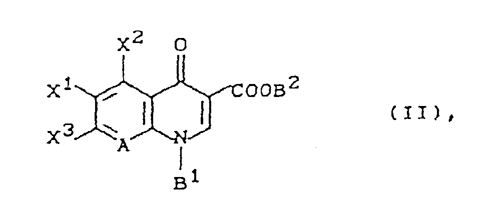 in welcher
A B¹, B², X¹ und X² die in Anspruch 1 angegebene Bedeutung haben und
X³ für Halogen, insbesondere Fluor oder Chlor steht, mit Verbindungen der Formel (III) 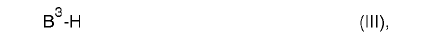 in welcher
B³ die in Anspruch 1 angegebene Bedeutung hat,
gegebenenfalls in Gegenwart von Säurefängern umsetzt, und gegebenenfalls in B³ enthaltene Schutzgruppen abspaltet, wobei solche Verbindungen der Formel (I)
in welcher
X¹, B¹, B², B³ und A die in Anspruch 1 angegebene Bedeutung haben und
X² für Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen oder Arylthio steht,
auch erhalten werden können, indem man eine Verbindung der Formel (IV) 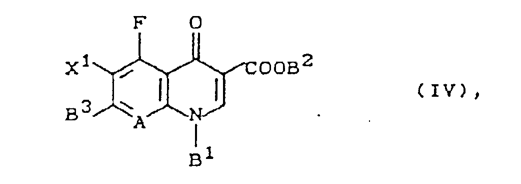 in welcher
X¹, B¹, B², B³ und A die in Anspruch 1 angegebene Bedeutung haben,
mit Verbindungen der Formel (V) 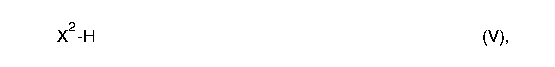
in welcher
X² die oben angegebene Bedeutung hat, gegebenenfalls in Gegenwart von Säurefängern, umsetzt, und gegebenenfalls auf an sich bekannte Weise die so erhaltenen Verbindungen der Formel (I) in ihre pharmazeutisch verwendbaren Hydrate und Säureadditionssalze sowie deren Alkali-, Erdalkali-, Silber- und Guanidiniumsalze überführt.

## Patentansprüche (Patentansprüche für folgende(n) Vertragsstaat(en) : GR)

1. 7-(2,7-Diazabicyclo[3.3.0]octyl)-3-chinolon- und -naphthyridoncarbonsäure-Derivate der Formel (I) 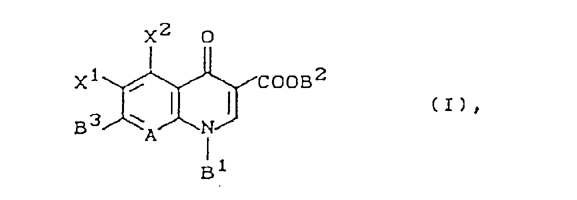 in welcher
X¹ für Halogen,
X² für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen, Methyl
B¹ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy Amino, Methylamino, Ethylamino, Dimethylamino, gegebenenfalls durch 1 oder 2 Fluoratome substituiertes Phenyl,
R⁴' für Halogen, Amino, Hydroxy, Methoxy oder für durch Halogen, Amino, Hydroxy oder Methoxy substituiertes Methyl steht oder dann für Wasserstoff steht, wenn
R²' und R³' zusammen eine C₂-C₄-Alkylenbrücke oder eine CH₂-S-CH₂-Brücke bedeuten, die gegebenenfalls durch Hydroxy, Methoxy, Amino oder Methyl ein-oder zweifach substituiert sein kann,
A für N oder C-R⁹ steht, worin
R⁹ für H, Halogen, Methyl, Cyano, Nitro, Hydroxy oder Methoxy steht
und deren pharmazeutisch verwendbaren Hydrate und Säureadditionssalze sowie deren Alkali-, Erdalkali-, Silber-und Guanidiniumsalze.
B² für Wasserstoff, Alkyl mit 1 bis 4 Kohlenstoffatomen oder (5-Methyl-2-oxo-1,3-dioxol-4-yl)-methyl,
B³ für einen Rest der Struktur 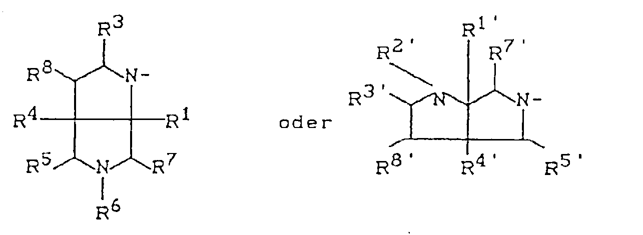 worin
R¹, R³, R⁴, R⁵, R⁷ und R⁸ gleich oder verschieden sind und jeweils für Wasserstoff oder Methyl, das gegebenenfalls durch Halogen oder Hydroxy substituiert sein kann, C₁-C₂-Alkoxycarbonyl oder Carboxy stehen,
R⁴ und R⁸ zusätzlich für Halogen, Amino, Hydroxy oder Methoxy stehen können,
R⁶ für Wasserstoff, C₁-C₆-Alkyl, Benzyl, C₆-C₁₂-Aryl, C₁-C₃-Alkanoyl, Benzoyl, C₁-C₅-Alkoxycarbonyl steht,
R^{1'}, R^{2'}, R^{3'}, R^{5'}, R^{7'} und R^{8'} gleich oder verschieden sind und jeweils für Wasserstoff oder Methyl stehen,

2. Verbindungen gemäß Anspruch 1,
in welchen
X¹ für Fluor oder Chlor,
X² für Wasserstoff, Amino, Alkylamino mit 1 bis 2 Kohlenstoffatomen, Dimethylamino, Hydroxy, Methoxy, Mercapto, Methylthio, Fluor, Methyl,
B¹ für Alkyl mit 1 bis 3 Kohlenstoffatomen, Alkenyl mit 2 bis 3 Kohlenstoffatomen, Cycloalkyl mit 3 bis 5 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
B² für Wasserstoff, Alkyl mit 1 bis 3 Kohlenstoffatomen oder (5-Methyl-2-oxo-1,3-dioxol-4-yl)-methyl,
B³ für einen Rest der in Anspruch 1 beschriebenen Struktur
stehen, worin
R¹, R³, R⁴, R⁵, R⁷ und R⁸ gleich oder verschieden sind und jeweils für Wasserstoff oder Methyl, das gegebenenfalls du rch Halogen substituiert sein kann, C₁-C₃-Alkoxycarbonyl stehen,
R⁴ zusätzlich für Halogen, wie Fluor, Chlor oder Brom stehen kann,
R⁶ für Wasserstoff, C₁-C₃-Alkyl, Benzyl, Phenyl, Acetyl, Benzoyl, C₁-C₄-Alkoxycarbonyl steht,
R¹', R²', R³', R⁵', R⁷' und R8 gleich oder verschieden sind und jeweils für Wasserstoff oder Methyl stehen,
R⁴' für Halogen wie Chlor oder Brom steht oder dann für Wasserstoff steht, wenn
R²' und R³' zusammen eine C₂-C₄-Alkylenbrücke oder eine CH₂-S-CH₂-Brücke bedeuten, die gegebenenfalls durch Hydroxy oder Methyl ein- oder zweifach substituiert sein kann,
A für N oder C-R⁹ steht, worin
_{R}9 für H, Halogen oder Methoxy steht
und deren pharmazeutisch verwendbaren Hydrate und Säureadditionssalze sowie deren Alkali- und Erdalkalisalze.

3. Verbindungen gemäß Anspruch 1,
in welchen
X¹ für Fluor,
X² für Wasserstoff, Amino, Fluor, Methyl,
B¹ für Alkyl mit 1 bis 2 Kohlenstoffatomen, Vinyl, Cyclopropyl, 2-Hydroxyethyl, 2-Fluorethyl, Methoxy, Methylamino, 4-Fluorphenyl, 2,4-Difluorphenyl,
B² für Wasserstoff, Alkyl mit 1 bis 2 Kohlenstoffatomen,
B³ für einen Rest der in Anspruch 1 beschriebenen Struktur steht,
worin
R¹, R³, R⁴, R⁵, R⁷ und R⁸ gleich oder verschieden sind und jeweils für Wasserstoff oder Methyl steht, das gegebenenfalls durch Fluor substituiert sein kann,
_{R}⁴ zusätzlich für Fluor, Chlor oder Brom stehen kann,
R⁶ für Wasserstoff, Methyl, Acetyl, C₁-C₄-Alkoxycarbonyl steht,
R¹', R²', R³', R5, R⁷' und R8 gleich oder verschieden sind und jeweils für Wasserstoff oder Methyl stehen,
R⁴' für Chlor oder Brom steht oder dann für Wasserstoff steht, wenn
R²' und R³' zusammen eine C₂-C₄-Alkylenbrücke oder eine CH₂-S-CH₂-Brücke bedeuten, die gegebenenfalls durch Hydroxy oder Methyl ein-oder zweifach substituiert sein kann,
A für N oder C-R⁹ steht, worin
_{R}9 für H, Halogen oder Methoxy steht
und deren pharmazeutisch verwendbaren Hydrate und Säureadditionssalze sowie deren Alkalisalze.

4. 1-Cyclopropyl-6,8-difluor-1,4-dihydro-7-(7-methyl-2,7-diazabicycl0[3.3.0]oct-2-yl)-4-oxo-3-chinolincarbonsäure der Formel

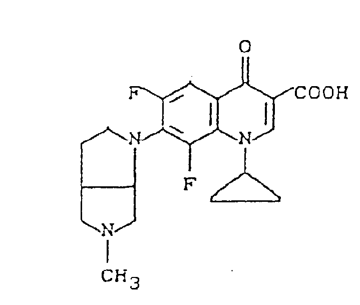

5. 8-Chlor-1-cyclopropyl-6-fluor-1,4-dihydro-7-(7-methyl-2,7-diazabicyclo[3.3.0]oct-2-yl)-4-oxo-3-chinolincarbon- säure der Formel

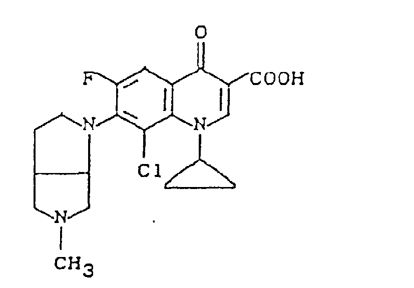

6. 1-Cyclopropyl-6,8-difluor-1,4-dihydro-7-(5-chlor-2,7-diazabicyclo[3.3.0]oct-2-yl)-4-oxo-3-chinolincarbonsäure der Formel

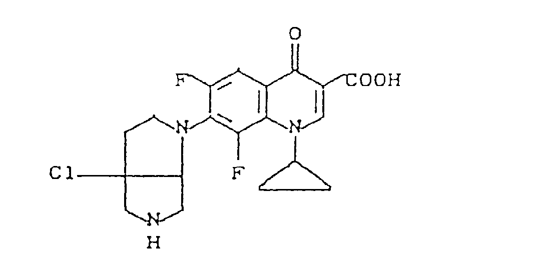

7. 1-Cyclopropyl-7-(1,4-diazatricyclo[6.2.0.0^{2,6}]dec-4-yl)-6,8-diflur-1,4-dihydro-4-oxo-3-chinolincarbonsäureder Formel

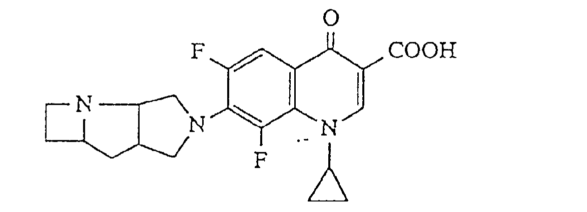

8. 1-Cyclopropyl-7-(2,7-diazabicyclo[3.3.0]oct-2-yl)-6,8-diflur-1,4-dihydro-4-oxo-3-chinolincarbonsäure der Formel

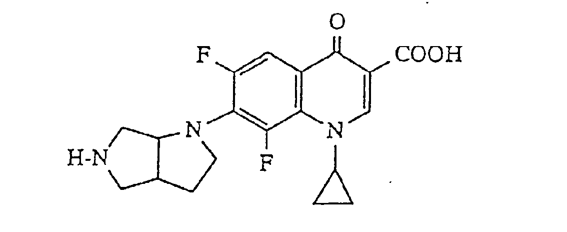

9. Verfahren zur Herstellungvon Verbindungen gemäß Anspruch 1, dadurch gekennzeichnet, daß man Verbindungen der Formel (II) 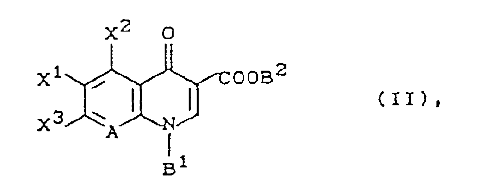 in welcher
A, B¹, B², X¹ und X² die in Anspruch 1 angegebene Bedeutung haben und
X³ für Halogen, insbesondere Fluor oder Chlor steht, mit Verbindungen der Formel (III) 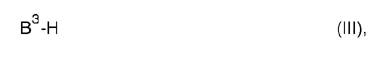 in welcher
B³ die in Anspruch 1 angegebene Bedeutung hat,
gegebenenfalls in Gegenwart von Säurefängern umsetzt, und gegebenenfalls in B³ enthaltene Schutzgruppen abspaltet, wobei solche Verbindungen der Formel (I)
in welcher
X¹, B¹, B², B³ und A die in Anspruch 1 angegebene Bedeutung haben und
X² für Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen oder Arylthio steht,
auch erhalten werden können, indem man eine Verbindung der Formel (IV) 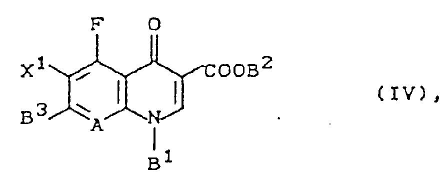 in welcher
X¹, B¹, B², B³ und A die in Anspruch 1 angegebene Bedeutung haben,
mit Verbindungen der Formel (V) 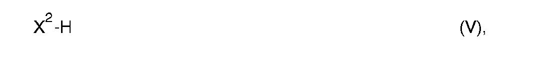 in welcher
X² die oben angegebene Bedeutung hat, gegebenenfalls in Gegenwart von Säurefängern umsetzt,

10. Verwendung von Verbindungen gemäß Ansprüchen 1 - 8 zur Herstellung von Arzneimitteln.

11. Verwendung von Verbindungen gemäß Ansprüchen 1 - 8 als Tierfutterzusatzmittel.

12. Tierfutter bzw. Tierfutterzusatzmittel und Prämixe enthaltend Verbindungen gemäß Ansprüchen 1 - 8.

## Claims (Claims for the following Contracting State(s) : AT, BE, CH, DE, DK, FR, GB, IT, LI, LU, NL, SE)

1. 7-(2,7-Diazabicyclo[3.3.0]octyl)-3-quinolone- and -naphthyridonecarboxylic acid derivatives of the formula (I) 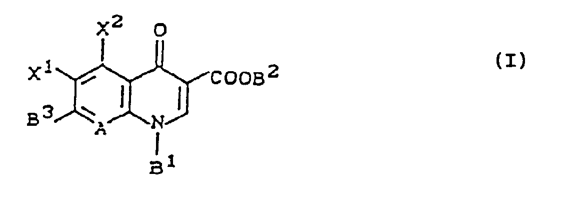 in which
X¹ represents halogen,
X² represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen or methyl,
B¹ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy, amino, methylamino, ethylamino, dimethylamino, or phenyl which is optionally substituted by 1 or 2 fluorine atoms,
B² represents hydrogen, alkyl having 1 to 4 carbon atoms or (5-methyl-2-oxo-1,3-dioxol-4-yl)-methyl,
B³ represents a radical of the structure 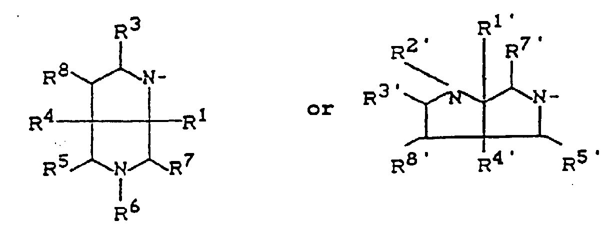 where
R¹, R³, R⁴, R⁵, R⁷ and R⁸ are identical or different and in each case represent hydrogen or methyl which can optionally be substituted by halogen or hydroxyl, or represent C₁-C₂-alkoxycarbonyl or carboxyl,
R⁴ and R⁸ can additionally represent halogen, amino, hydroxyl or methoxy,
R⁶ represents hydrogen, C₁-C₆-alkyl, benzyl, C₆-C₁₂-aryl, C₁-C₃-alkanoyl, benzoyl or C₁-C₅-alkoxycarbonyl,
R¹', R²', R³', R5, R¹' and R⁸' are identical or different and in each case represent hydrogen or methyl,
R⁴' represents halogen, amino, hydroxyl, methoxy or methyl which is substituted by halogen, amino, hydroxyl or methoxy, or represents hydrogen when
R²' and R³' together denote a C₂-C₄-alkylene bridge or a CH₂-S-CH₂ bridge which can optionally be monosubstituted or disubstituted by hydroxyl, methoxy, amino or methyl,
A represents N or C-R⁹, where
R⁹ represents H, halogen, methyl, cyano, nitro, hydroxyl or methoxy
and their hydrates and acid addition salts which can be used in pharmaceutics, as well as their alkali metal salts, alkaline earth metal salts, silver salts and guanidinium salts.

2. Compounds according to Claim 1,
in which
X¹ represents fluorine or chlorine,
X² represents hydrogen, amino, alkylamino having 1 to 2 carbon atoms, dimethylamino, hydroxyl, methoxy, mercapto, methylthio, fluorine or methyl,
B¹ represents alkyl having 1 to 3 carbon atoms, alkenyl having 2 to 3 carbon atoms, cycloalkyl having 3 to 5 carbon atoms, 2-hydroxyethyl, 2-fluoroethyl, methoxy, amino, methylamino, ethylamino, dimethylamino, or phenyl which is optionally substituted by 1 or 2 fluorine atoms,
B² represents hydrogen, alkyl having 1 to 3 carbon atomsor(5-methyl-2-oxo-1,3-dioxol-4-yl)-methyl,
B³ represents a radical of the structure described in Claim 1,
where
R¹, R³, R⁴, R⁵, R⁷ and R⁸ are identical or different and in each case represent hydrogen or methyl which can optionally be substituted by halogen, or represent C₁-C₃- alkoxycarbonyl,
R⁴ can additionally represent halogen such as fluorine, chlorine or bromine,
R⁶ represents hydrogen, C₁-C₃-alkyl, benzyl, phenyl, acetyl, benzoyl or C₁-C₄-alkoxycarbonyl,
R¹', R²', R³', R⁵', R⁷' and R⁸' are identical or different and in each case represent hydrogen or methyl,
R⁴' represents halogen such as chlorine or bromine, or represents hydrogen when
R²' and R³' together denote a C₂-C₄-alkylene bridge or a CH₂-S-CH₂ bridge which can optionally be monosubstituted or disubstituted by hydroxyl or methyl,
A represents N or C-R⁹, where
_{R}9 represents H, halogen or methoxy
and their hydrates and acid addition salts which can be used in pharmaceutics, as well as their alkali metal salts and alkaline earth metal salts.

3. Compounds according to Claim 1,
in which
X¹ represents fluorine,
X² represents hydrogen, amino, fluorine or methyl,
B¹ represents alkyl having 1 to 2 carbon atoms, vinyl, cyclopropyl, 2-hydroxyethyl, 2-fluoroethyl; methoxy methylamino, 4-fluorophenyl or 2,4-difluorophenyl,
B² represents hydrogen or alkyl having 1 to 2 carbon atoms,
B³ represents a radical of the structure described in Claim 1,
where
R¹, R³, R⁴, R⁵, R⁷ and R⁸ are identical or different and in each case represent hydrogen or methyl which can optionally be substituted by fluorine,
R⁴ can additionally represent fluorine, chlorine or bromine,
R⁶ represents hydrogen, methyl, acetyl or C₁-C₄-alkoxycarbonyl,
R^{1'}, R^{2'}, R^{3'}, R^{5'}, R^{7'} and R8 are identical or different and in each case represent hydrogen or methyl,
R⁴' represents chlorine or bromine, or represents hydrogen when
R²' and R³' together denote a C₂-C₄-alkylene bridge or a CH₂-S-CH₂ bridge which can optionally be monosubstituted or disubstituted by hydroxyl or methyl,
A represents N or C-R⁹, where
_{R}⁹ represents H, halogen or methoxy
and their hydrates and acid addition salts which can be used in pharmaceutics, as well as their alkali metal salts.

4. 1-Cyclopropyl-6,8-difluoro-1,4-dihydro-7-(7-methyl-2;7-diazabicyclo[3.3.0]oct-2-yl)-4-oxo-3-quinolinecarboxylic acid, of the formula

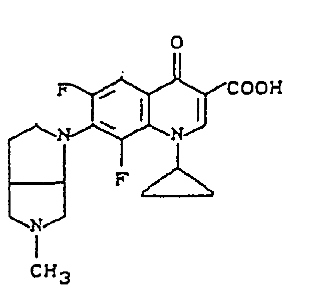

5. 8-Chloro-1-cyclopropyl-6-fluoro-1,4-dihydro-7-(7-methyl-2,7-diazabicyclo[3.3.0]oct-2-yl)-4-oxo-3-quinolinecarboxylic acid, of the formula

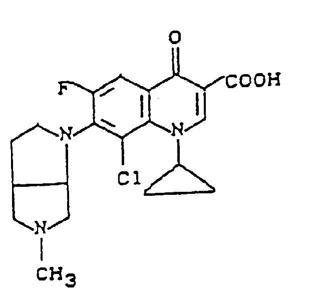

6. 1-Cyclopropyl-6,8-difluoro-1,4-dihydro-7-(5-chloro-2,7-diazabicycl0[3.3.0]oct-2-yl)-4-oxo-3-quinoline-carboxylic acid, of the formula

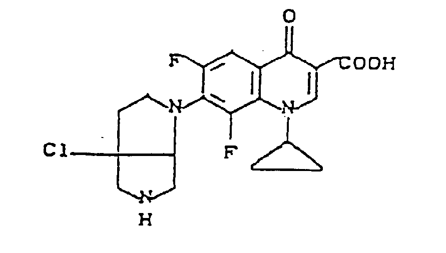

7. 1-Cyclopropyl-7-(1,4-diazatricyclo[6.2.002,6]dec-4-yl)-6,8-difluoro-1,4-dihydro-4-oxo-3-quinolinecarboxylic acid, of the formula

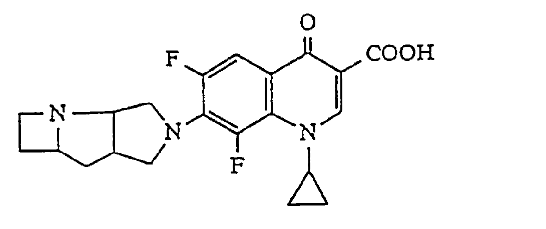

8. 1-Cyclopropyl-7-(2,7-diazabicyclo[3.3.0]oct-2-yl)-6,8-difluoro-1,4-dihydro-4-oxo-3-quinolinecarboxylic acid, of the formula

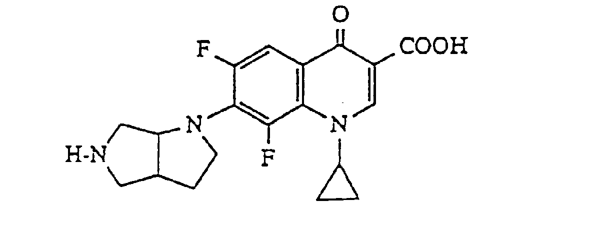

9. Process for the preparation of compounds according to Claim 1, characterised in that compounds of the formula (II) 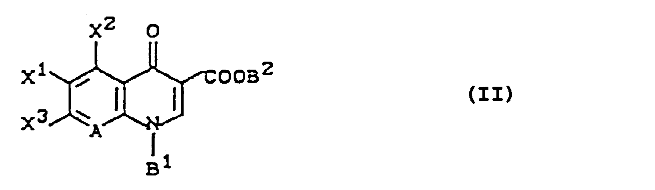 in which
A B¹, B², X¹ and X² have the meaning given in Claim 1 and
X³ represents halogen, in particular fluorine or chlorine, are reacted with compounds of the formula (III) 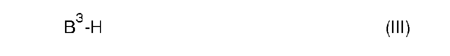 in which
B³ has the meaning given in Claim 1,
if appropriate in the presence of acid scavengers, and, if appropriate, protective groups contained in B³ are eliminated, it also being possible to obtain those compounds of the formula (I)
in which
X¹, B¹, B², B³ and A have the meaning given in Claim 1 and
X² represents amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, or arylthio,
by reacting a compound of the formula (IV) 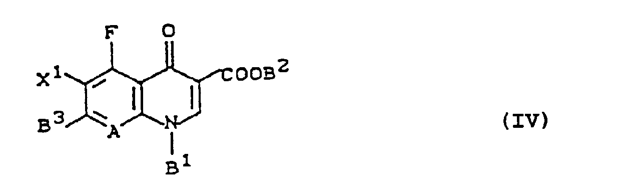 in which
X¹, B¹, B², B³ and A have the meaning given in Claim 1,
with compounds of the formula (V) 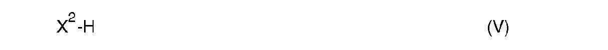 in which
X² has the meaning given above, if appropriate in the presence of acid scavengers.

10. Medicaments containing compounds according to Claims 1 - 8.

11. Antibiotics containing compounds according to Claims 1 - 8.

12. Use of compounds according to Claims 1 - 8 for preparing medicaments.

13. Use of compounds according to Claims 1 - 8 as animal-feed additives.

14. Animal feeds or animal-feed additives and premixes containing compounds according to Claims 1 - 8.

## Claims (Claims for the following Contracting State(s) : ES)

1. Process for the preparation of 7-(2,7-diazabicyclo-[3.3.0]octyl)-3-quinolone- and -naphthyridonecarboxylic acid derivatives of the formula (I) 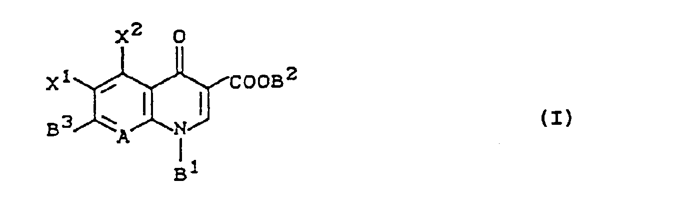 in which
X¹ represents halogen,
X² represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen or methyl,
B¹ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy, amino, methylamino, ethylamino, dimethylamino, or phenyl which is optionally substituted by 1 or 2 fluorine atoms,
B² represents hydrogen, alkyl having 1 to 4 carbon atoms or (5-methyl-2-oxo-1,3-dioxol-4-yl)-methyl,
B³ represents a radical of the structure 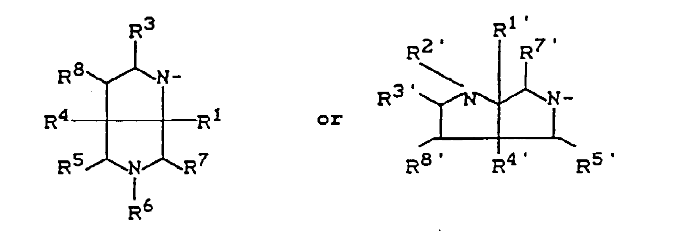 where
R¹, R³, R⁴, R⁵, R⁷ and R⁸ are identical or different and in each case represent hydrogen or methyl which can optionally be substituted by halogen or hydroxyl, or represent C₁-C₂-alkoxycarbonyl or carboxyl,
R⁴ and R⁸ can additionally represent halogen, amino, hydroxyl or methoxy,
R⁶ represents hydrogen, C₁-C₆-alkyl, benzyl, C₆ C₁₂-aryl, C₁-C₃-alkanoyl, benzoyl or C₁-C₅-alkoxycarbonyl,
R¹', R²', R³', R⁵', R⁷' and R8 are identical or different and in each case represent hydrogen or methyl,
R⁴' represents halogen, amino, hydroxyl, methoxy or methyl which is substituted by halogen, amino, hydroxyl or methoxy, or represents hydrogen when
R²' and R³' together denote a C₂-C₄-alkylene bridge or a CH₂-S-CH₂ bridge which can optionally be monosubstituted or disubstituted by hydroxyl, methoxy, amino or methyl,
A represents N or C-R⁹, where
R⁹ represents H, halogen, methyl, cyano, nitro, hydroxyl or methoxy
and their hydrates and acid addition salts which can be used in pharmaceutics, as well as their alkali metal salts, alkaline earth metal salts, silver salts and guanidinium salts
characterised in that compounds of the formula (II) 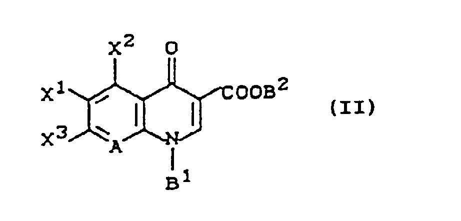 in which
A, B¹, B², X¹ and X² have the meaning given in Claim 1 and
X³ represents halogen, in particular fluorine or chlorine, are reacted with compounds of the formula (III) 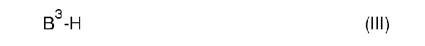 in which
B³ has the meaning given in Claim 1,
if appropriate in the presence of acid scavengers, and, if appropriate, protective groups contained in B³ are eliminated, it also being possible to obtain those compounds of the formula (I)
in which
X¹, B¹, B², B³ and A have the meaning given in Claim 1 and
X² represents amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, or arylthio,
by reacting a compound of the formula (IV) 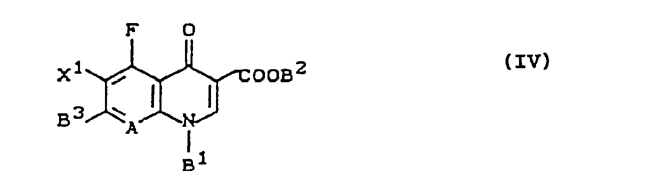 in which
X¹, B¹, B², B³ and A have the meaning given in Claim 1,
with compounds of the formula (V) 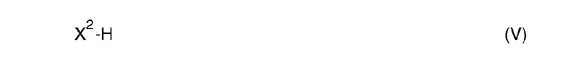 in which
X² has the meaning given above, if appropriate in the presence of acid scavengers, and, if appropriate, converting the resulting compounds of the formula (I) in a manner known per se into their pharmaceutically utilizable hydrates and acid addition salts, as well as their alkali metal salts, alkaline earth metal salts, silver salts and guanidinium salts.

## Claims (Claims for the following Contracting State(s) : GR)

1. 7-(2,7-Diazabicyclo[3.3.0]octyl)-3-quinolone- and -naphthyridonecarboxylic acid derivatives of the formula (I) 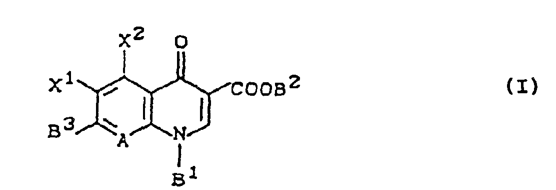 in which
X¹ represents halogen,
X² represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen or methyl,
B¹ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy, amino, methylamino, ethylamino, dimethylamino, or phenyl which is optionally substituted by 1 or 2 fluorine atoms,
B² represents hydrogen, alkyl having 1 to 4 carbon atoms or (5-methyl-2-oxo-1,3-dioxol-4-yl)-methyl,
B³ represents a radical of the structure 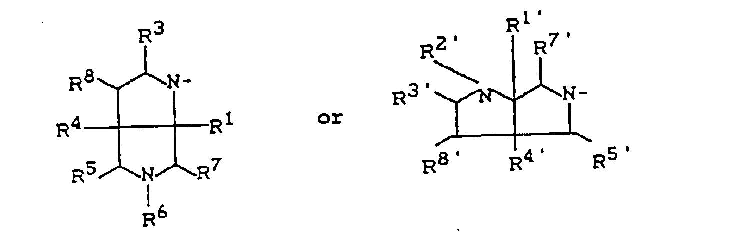 where
R¹, R³, R⁴, R⁵, R⁷ and R⁸ are identical or different and in each case represent hydrogen or methyl which can optionally be substituted by halogen or hydroxyl, or represent C₁-C₂-alkoxycarbonyl or carboxyl,
R⁴ and R⁸ can additionally represent halogen, amino, hydroxyl or methoxy,
R⁶ represents hydrogen, C₁-C₈-alkyl, benzyl, C₆-C₁₂-aryl, C₁-C₃-alkanoyl, benzoyl or C₁-C₅-alkoxycarbonyl,
R¹', R²', R³', R⁵', R⁷' and R⁸' are identical or different and in each case represent hydrogen or methyl,
R⁴' represents halogen, amino, hydroxyl, methoxy or methyl which is substituted by halogen, amino, hydroxyl or methoxy, or represents hydrogen when
R²' and R³' together denote a C₂-C₄-alkylene bridge or a CH₂-S-CH₂ bridge which can optionally be monosubstituted or disubstituted byhydroxyl, methoxy, amino or methyl,
A represents N or C-R⁹, where
R⁹ represents H, halogen, methyl, cyano, nitro, hydroxyl or methoxy
and their hydrates and acid addition salts which can be used in pharmaceutics, as well as their alkali metal salts, alkaline earth metal salts, silver salts and guanidinium salts.

2. Compounds according to Claim 1,
in which
X¹ represents fluorine or chlorine,
X² represents hydrogen, amino, alkylamino having 1 to 2 carbon atoms, dimethylamino, hydroxyl, methoxy, mercapto, methylthio, fluorine or methyl,
B¹ represents alkyl having 1 to 3 carbon atoms, alkenyl having 2 to 3 carbon atoms, cycloalkyl having 3 to 5 carbon atoms, 2-hydroxyethyl, 2-fluoroethyl, methoxy, amino, methylamino, ethylamino, dimethylamino, or phenyl which is optionally substituted by 1 or 2 fluorine atoms,
B² represents hydrogen, alkyl having 1 to 3 carbon atoms or (5-methyl-2-oxo-1, 3-dioxol-4-yl)-methyl,
B³ represents a radical of the structure described in Claim 1, where
R¹, R³, R⁴, R⁵, R⁷ and R⁸ are identical or different and in each case represent hydrogen or methyl which can optionally be substituted by halogen, or represent C₁-C₃- alkoxycarbonyl,
R⁴ can additionally represent halogen such as fluorine, chlorine or bromine,
R⁶ represents hydrogen, C₁-C₃-alkyl, benzyl, phenyl, acetyl, benzoyl or C₁-C₄-alkoxycarbonyl,
R¹', R²', R³', R⁵', R⁷' and R8 are identical or different and in each case represent hydrogen or methyl,
R⁴' represents halogen such as chlorine or bromine, or represents hydrogen when
R²' and R³' together denote a C₂₋C₄-alkylene bridge or a CH₂-S-CH₂ bridge which can optionally be monosubstituted or disubstituted by hydroxyl or methyl,
A represents N or C-R⁹, where
_{R}9 represents H, halogen or methoxy
and their hydrates and acid addition salts which can be used in pharmaceutics, as well as their alkali metal salts and alkaline earth metal salts.

3. Compounds according to Claim 1,
in which
X¹ represents fluorine,
X² represents hydrogen, amino, fluorine or methyl,
B¹ represents alkyl having 1 to 2 carbon atoms, vinyl, cyclopropyl, 2-hydroxyethyl, 2-fluoroethyl, methoxy, methylamino, 4-fluorophenyl or 2,4-difluorophenyl,
B² represents hydrogen or alkyl having 1 to 2 carbon atoms,
B³ represents a radical of the structure described in Claim 1,
where
R¹, R³, R4 R⁵, R⁷ and R⁸ are identical or different and in each case represent hydrogen or methyl which can optionally be substituted by fluorine,
R⁴ can additionally represent fluorine, chlorine or bromine,
R⁶ represents hydrogen, methyl, acetyl or C₁-C₄-alkoxycarbonyl,
R¹', R²', R³', R⁵', R⁷' and R8 are identical or different and in each case represent hydrogen or methyl,
R⁴' represents chlorine or bromine, or represents hydrogen when
R²' and R³' together denote a C₂-C₄-alkylene bridge or a CH₂-S-CH₂ bridge which can optionally be monosubstituted or disubstituted by hydroxyl or methyl,
A represents N or C-R⁹, where
R⁹ represents H, halogen or methoxy and their hydrates and acid addition salts which can be used in pharmaceutics, as well as their alkali metal salts.

4. 1-Cyclopropyl-6,8-difluoro-1,4-dihydro-7-(7-methyl-2;7-diazabicyclo[3.3.0]oct-2-yl)-4-oxo-3-quinolinecarboxylic acid, of the formula

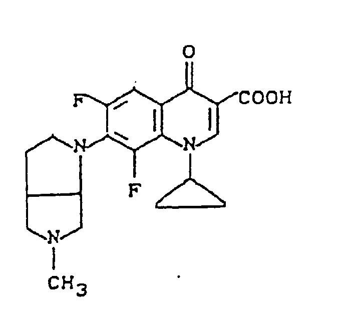

5. 8-Chloro-1-cyclopropyl-6-fluoro-1,4-dihydro-7-(7-methyl-2,7-diazabicyclo[3.3.0]oct-2-yl)-4-oxo-3-quinolinecarboxylic acid, of the formula

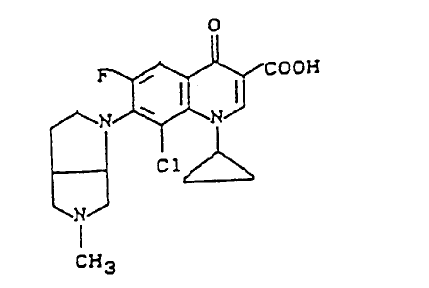

6. 1-Cyclopropyl-6,8-difluoro-1,4-dihydro-7-(5-chloro-2,7-diazabicyclo[3.3.0]oct-2-yl)-4-oxo-3-quinolinecarboxylic acid, of the formula

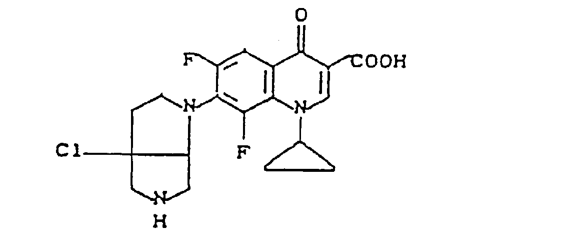

7. 1-Cyclopropyl-7-(1,4-diazatricyclo[6.200^{2,6}]dec-4-yl)-6,e-difluoro-1,4-dihydro-4-oxo-3-quinclinecarboxylic acid, of the formula

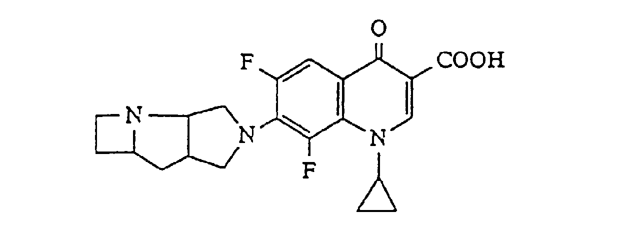

8. 1-Cyclopropyl-7-(2,7-diazabicyclo[3.3.0]oct-2-yl)-6,8-difluoro-1,4-dihydro-4-oxo-3-quinolinecarboxylic acid, of the formula

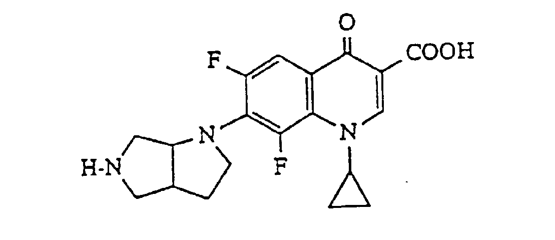

9. Process for the preparation of compounds according to Claim 1, characterised in that compounds of the formula (II) 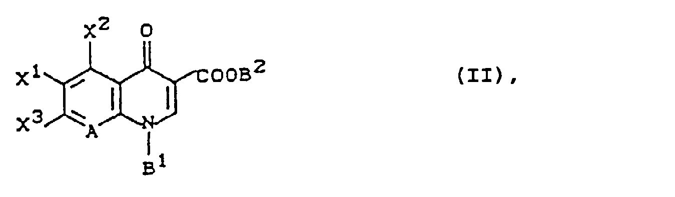 in which
A, B¹, B², X¹ and X² have the meaning given in Claim 1 and
X³ represents halogen, in particular fluorine or chlorine, are reacted with compounds of the formula (III) 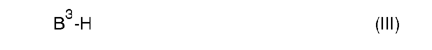 in which
B³ has the meaning given in Claim 1,
if appropriate in the presence of acid scavengers, and, if appropriate, protective groups contained in B³ are eliminated, it also being possible to obtain those compounds of the formula (I)
in which
X¹, B¹, B², B³ and A have the meaning given in Claim 1 and
X² represents amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, or arylthio,
by reacting a compound of the formula (IV) 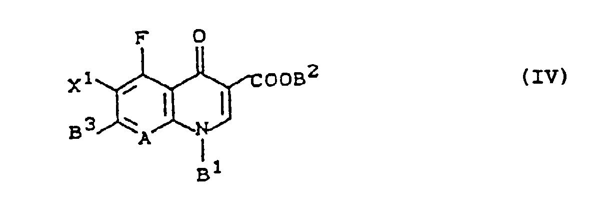
in which
X¹, B¹, B², B³ and A have the meaning given in Claim 1,
with compounds of the formula (V) 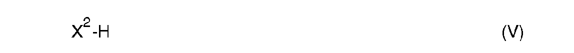
in which
X² has the meaning given above, if appropriate in the presence of acid scavengers.

10. Use of compounds according to Claims 1 - 8 for preparing medicaments.

11. Use of compounds according to Claims 1 - 8 as animal-feed additives.

12. Animal feeds or animal-feed additives and premixes containing compounds according to Claims 1 - 8.

## Revendications (Revendications pour l'(les) Etat(s) contractant(s) suivant(s) : AT, BE, CH, DE, DK, FR, GB, IT, LI, LU, NL, SE)

1. Dérivés d'acides 7-(2,7-diazabicyclo[3.3.0]octyl)-3-quinolone- et -naphtyridone-carboxyliques de formule (I) 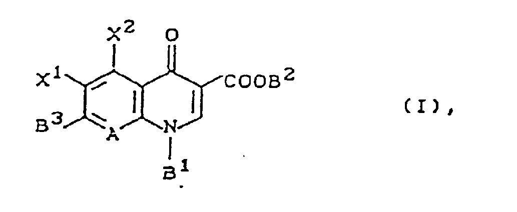 dans laquelle
X¹ représente un halogène,
X² est de l'hydrogène, un groupe amine, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, halogéno, méthyle,
B¹ est un groupe alkyle ayant 1 à 4 atomes de carbone, alcényle ayant 2 à 4 atomes de carbone, cycloalkyle ayant 3 à 6 atomes de carbone, 2-hydroxyéthyle, 2-fluoréthyle, méthoxy, amino, méthylamino, éthylamino, diméthylamino, phényle éventuellement substitué par 1 ou 2 atomes de fluor,
B² est de l'hydrogène, un groupe alkyle ayant 1 à 4 atomes de carbone ou le groupe (5-méthyl-2-oxo-1,3-dioxole-4-yl)méthyle,
B³ représente un reste de structure 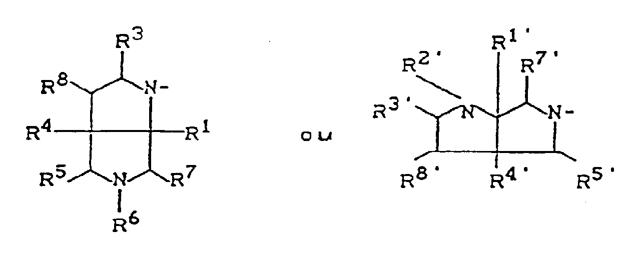 dans laquelle
R¹, R3, R4, R5, R7 et R⁸ sont identiques ou différents et représentent chacun de l'hydrogène ou un groupe méthyle qui peut être substitué, le cas échéant, par un halogène ou un substituant hydroxy, un groupe (alkoxy en C₁ ou C₂)carbonyle ou carboxy,
R⁴ et R⁸ peuvent représenter en outre un halogène, un groupe amino, hydroxy ou méthoxy,
R⁶ représente de l'hydrogène, un groupe alkyle en C₁ à C₆, benzyle, aryle en C₆ à C₁₂, alcanoyle en C₁ à C₃, benzoyle, (alkoxy en C₁ à C₅)carbonyle,
R¹', R²', R³', R⁵', R⁷' et R⁸' sont identiques ou différents et représentent chacun de l'hydrogène ou un groupe méthyle,
R⁴' est un halogène, un groupe amino, hydroxy, méthoxy ou un groupe méthyle substitué par un radical halogéno, amino, hydroxy ou méthoxy ou représente de l'hydrogène, lorsque
R²' et R³' forment conjointement un pont alkylène en C₂ à C₄ ou un pont CH₂-S-CH₂ qui peut être substitué une ou deuxfois, le cas échéant par un radical hydroxy, méthoxy, amino ou méthyle,
A représente N ou C-R⁹, où
R⁹ est de l'hydrogène, un halogène, un groupe méthyle, cyano, nitro, hydroxy ou méthoxy
et leurs hydrates et leurs sels d'addition d'acides acceptables du point de vue pharmaceutique ainsi que leurs sels de métaux alcalins, de métaux alcalino-terreux, d'argent et de guanidinium.

2. Composés suivant la revendication 1,
dans lesquels
X¹ est du fluor ou du chlore,
X² est de l'hydrogène, un groupe amino, alkylamino ayant 1 ou 2 atomes de carbone, diméthylamino, hydroxy méthoxy, mercapto, méthylthio, fluoro, méthyle,
B¹ est un groupe alkyle ayant 1 à 3 atomes de carbone, un groupe alcényle ayant 2 ou 3 atomes de carbone, un groupe cycloalkyle ayant 3 à 5 atomes de carbone, un groupe 2-hydroxyéthyle, 2-fluoréthyle, méthoxy, amino, méthylamino, éthylamino, diméthylamino, phényle éventuellement substitué par 1 ou 2 atomes de fluor,
B² est de l'hydrogène, un groupe alkyle ayant 1 à 3 atomes de carbone ou le groupe (5-méthyl-2-oxo-1,3-dioxole-4-yl)méthyle,
B³ est un reste de la structure décrite dans la revendication 1,
où
R¹, R³, R⁴ R⁵, R⁷ et R⁸ sont identiques ou différents et représentent chacun de l'hydrogène ou un groupe méthyle qui peut être substitué, le cas échéant, par un halogène, ou un groupe (alkoxy en C₁ à C₃)carbonyle,
R⁴ peut être en outre un halogène tel que le fluor, le chlore ou le brome,
R⁶ représente de l'hydrogène, un groupe alkyle en C₁ à C₃, benzyle, phényle, acétyle, benzoyle, (alkoxy en C₁ à C₄)carbonyle,
R¹', R²', R³', R⁵', R⁷' et R⁸' sont identiques ou différents et représentent chacun de l'hydrogène ou un groupe méthyle,
R⁴' est un halogène tel que le chlore ou le brome ou représente de l'hydrogène lorsque
R²' et R³' forment conjointement un pont alkylène en C₂ à C₄ ou un pont CH₂-S-CH₂ qui peut être substitué une ou deuxfois, le cas échéant par un radical hydroxy ou méthyle,
A représente N ou C-R⁹, dans lequel
R⁹ est de l'hydrogène, un halogène ou un groupe méthoxy,
et leurs hydrates et leurs sels d'addition d'acides acceptables du point de vue pharmaceutique ainsi que leurs sels de métaux alcalins et de métaux alcalino-terreux.

3. Composés suivant la revendication 1, dans lesquels
X¹ est du fluor,
X² est de l'hydrogène, un groupe amino, du fluor, un groupe méthyle,
B¹ est un groupe alkyle ayant 1 ou 2 atomes de carbone, vinyle, cyclopropyle, 2-hydroxyéthyle, 2-fluoréthyle, méthoxy, méthylamino, 4-fluorophényle, 2,4-difluorophényle,
B² est de l'hydrogène, un groupe alkyle ayant 1 ou 2 atomes de carbone,
B³ est un reste de la structure décrite dans la revendication 1, où
R¹, R³, R⁴ R⁵, R⁷ et R⁸ sont identiques ou différents et représentent chacun de l'hydrogène ou un groupe méthyle qui peut être substitué, le cas échéant, par du fluor,
R⁴ peut représenter en outre du fluor, du chlore ou du brome,
R⁶ est de l'hydrogène, un groupe méthyle, acétyle, (alkoxy en C₁ à C₄)carbonyle,
R¹', R²', R³', R⁵', R⁷' et R⁸' sont identiques ou différents et représentent chacun de l'hydrogène ou un groupe méthyle,
R⁴' est du chlore ou du brome ou représente de l'hydrogène lorsque
R²' et R³' forment conjointement un pont alkylène en C₂ à C₄ ou un pont CH₂-S-CH₂ qui peut éventuellement être substitué une ou deux fois par un radical hydroxy ou méthyle,
A représente N ou un groupe C-R⁹, dans lequel
R⁹ est de l'hydrogène, un halogène ou un groupe méthoxy,
et leurs hydrates et leurs sels d'addition d'acides acceptables du point de vue pharmaceutique ainsi que leurs sels de métaux alcalins.

4. L'acide 1-cyclopropyl-6,8-difluoro-1,4-dihydro-7-(7-méthyl-2,7-diazabicyclo[3.3.0]oct-2-yl)-4-oxo-3-quinoléine- carboxylique de formule

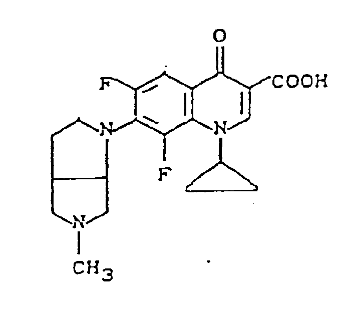

5. L'acide 8-chloro-1-cyclopropyl-6-fluoro-1,4-dihydro-7-(7-méthyl-2,7-diazabicyclo[3.3.0]oct-2-yl)-4-oxo-3-quinoléine-carboxylique de formule

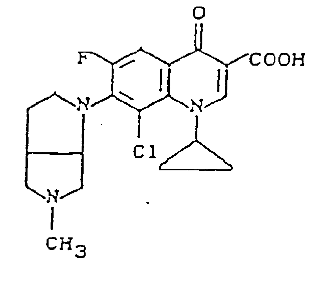

6. L'acide 1-cyclopropyl-6;8-difluoro-1,4-dihydro-7-(5-chloro-2,7-diazabicyclo[3.3.0]oct-2-yl)-4-oxo-3-quinoléine- carboxylique de formule

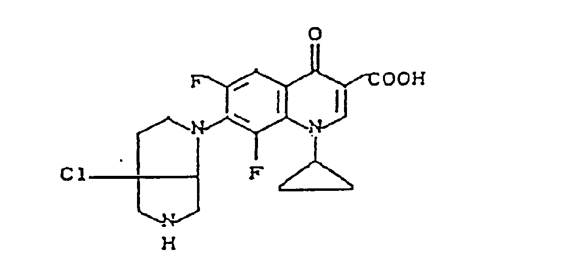

7. L'acide 1-cyclopropyl-7-(1,4-diazatricyclo-[6.2.0.0^{2,6}]déc-4-yl)-6,8-difluoro-1,4-dihydro-4-oxo-3-quinoléine-carboxylique de formule

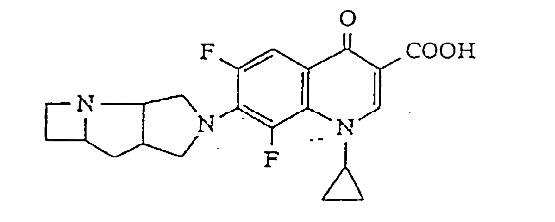

8. L'acide 1-cyclopropyl-7-(2,7-diazabicyclo[3.3.0]oct-2-yl)-6,8-difluoro-1,4-dihydroxy-4-oxo-3-quinoléine-carboxylique de formule

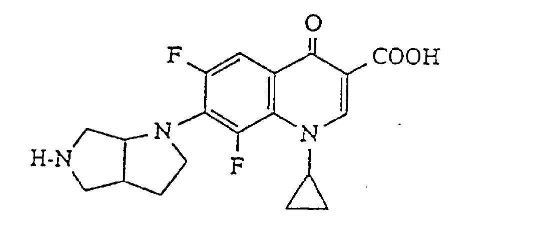

9. Procédé de production de composés suivant la revendication 1, caractérisé en ce qu'on fait réagir des composés de formule (II) 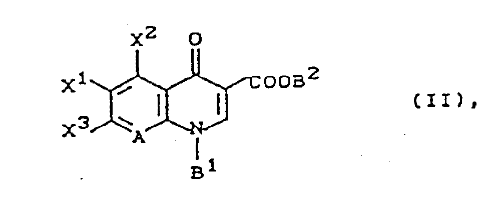 dans laquelle
A B¹, B², X¹ et X² ont la définition indiquée dans la revendication 1 et
X³ représente un halogène, en particulier le fluor ou le chlore, avec des composés de formule (III) 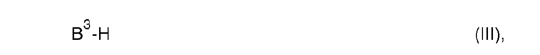 dans laquelle
B³ a la définition indiquée dans la revendication 1,
le cas échéant en présence d'accepteurs d'acides, et on élimine éventuellement des groupes protecteurs contenus dans B³, des composés de formule (I)
dans laquelle
X¹, B¹, B², B³ et A ont la définition indiquée dans la revendication 1 et
X² représente un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone ou arylthio,
pouvant aussi être obtenus par réaction d'un composé de formule (IV) 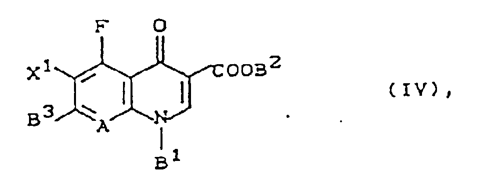 dans laquelle
X¹, B¹, B², B³ et A ont la définition indiquée dans la revendication 1,
avec des composés de formule (V) 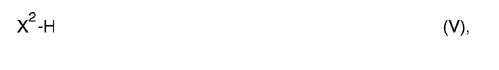 dans laquelle
X² a la définition indiquée ci-dessus,
le cas échéant en présence d'accepteurs d'acides.

10. Médicaments contenant des composés suivant les revendications 1 à 8.

11. Compositions antibiotiques contenant des composés suivant les revendications 1 à 8.

12. Utilisation de composés suivant les revendications 1 à 8 pour la préparation de médicaments.

13. Utilisation de composés suivant les revendications 1 à 8 comme additifs dans des aliments pour le bétail.

14. Aliments pour le bétail et additifs pour aliments pour le bétail et mélanges préalables contenant des composés suivant les revendications 1 à 8.

## Revendications (Revendications pour l'(les) Etat(s) contractant(s) suivant(s) : ES)

1. Procédé de production de dérivés d'acides 7-(2,7-diazabicyclo[3.3.0]octyl)-3-quinolone- et -naphtyridone-carboxyliques de formule (I) 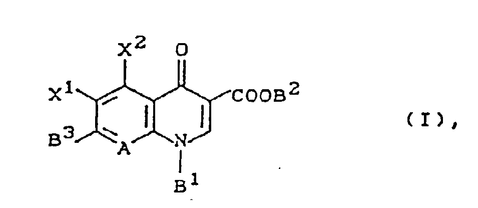 dans laquelle
X¹ représente un halogène,
X² est de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, halogéno, méthyle,
B¹ est un groupe alkyle ayant 1 à 4 atomes de carbone, alcényle ayant 2 à 4 atomes de carbone, cycloalkyle ayant 3 à 6 atomes de carbone, 2-hydroxyéthyle, 2-fluoréthyle, méthoxy, amino, méthylamino, éthylamino, diméthylamino, phényle éventuellement substitué par 1 ou 2 atomes de fluor,
B² est de l'hydrogène, un groupe alkyle ayant 1 à 4 atomes de carbone ou le groupe (5-méthyl-2-oxo-1,3-dioxole-4-yl)méthyle,
B³ représente un reste de structure 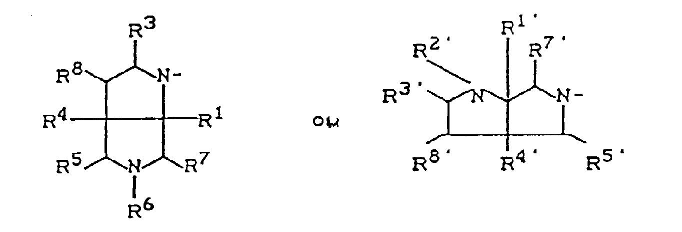 dans laquelle
R¹, R³, R⁴, R⁵, R⁷ et R⁸ sont identiques ou différents et représentent chacun de l'hydrogène ou un groupe méthyle qui peut être substitué, le cas échéant, par un halogène ou un substituant hydroxy, un groupe (alkoxy en C₁ ou C₂)carbonyle ou carboxy,
R⁴ et R⁸ peuvent représenter en outre un halogène, un groupe amino, hydroxy ou méthoxy,
R⁶ représente de l'hydrogène, un groupe alkyle en C₁ à C₆, benzyle, aryle en C₆ à C₁₂, alcanoyle en Ci à C₃, benzoyle, (alkoxy en C₁ à C₅)carbonyle,
R^{1'}, R^{2'}, R^{3'}, R⁵, R^{7'} et R^{8'} sont identiques ou différents et représentent chacun de l'hydrogène ou un groupe méthyle,
R⁴' est un halogène, un groupe amino, hydroxy, méthoxy ou un groupe méthyle substitué par un radical halogéno, amino, hydroxy ou méthoxy ou représente de l'hydrogène, lorsque
R²' et R³' forment conjointement un pont alkylène en C₂ à C₄ ou un pont CH₂-S-CH₂ qui peut être substitué une ou deux fois, le cas échéant par un radical hydroxy, méthoxy, amino ou méthyle,
A représente N ou C-R⁹, où
R⁹ est de l'hydrogène, un halogène, un groupe méthyle, cyano, nitro, hydroxy ou méthoxy
et de leurs hydrates et leurs sels d'addition d'acides acceptables du point de vue pharmaceutique ainsi que de leurs sels de métaux alcalins, de métaux alcalino-terreux, d'argent et de guanidinium,
caractérisé en ce qu'on fait réagir des composés de formule (II) 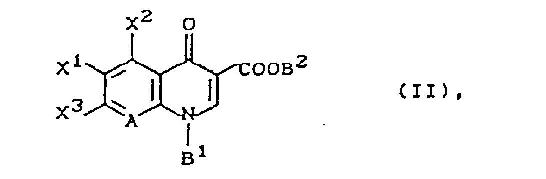 dans laquelle
A B¹, B², X¹ et X² ont la définition indiquée dans la revendication 1 et
X³ représente un halogène, en particulier le fluor ou le chlore, avec des composés de formule (III) 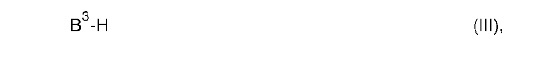 dans laquelle
B³ a la définition indiquée dans la revendication 1,
le cas échéant en présence d'accepteurs d'acides, et on élimine éventuellement des groupes protecteurs contenus dans B³, des composés de formule (I)
dans laquelle
X¹, B⁷, B², B³ et A ont la définition indiquée dans la revendication 1 et
X² représente un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone ou arylthio,
pouvant aussi être obtenus par réaction d'un composé de formule (IV) 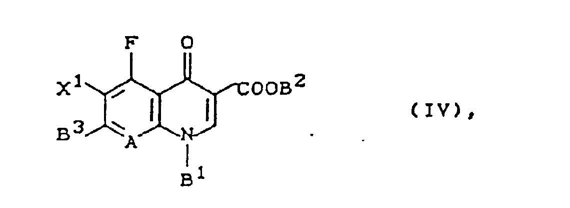 dans laquelle
X¹, B¹, B², B³ et A ont la définition indiquée dans la revendication 1,
avec des composés de formule (V) 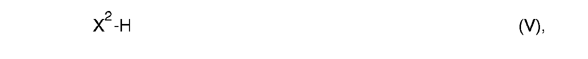 dans laquelle
X² a la définition indiquée ci-dessus,
le cas échéant en présence d'accepteurs d'acides, et, le cas échéant, on transforme d'une manière connue les composés de formule (I) ainsi obtenus en leurs hydrates et leurs sels d'addition d'acides acceptables du point de vue pharmaceutique ainsi qu'en leurs sels de métaux alcalins, de métaux alcalino-terreux, d'argent et de guanidinium.

## Revendications (Revendications pour l'(les) Etat(s) contractant(s) suivant(s) : GR)

1. Dérivés d'acides 7-(2,7-diazabicyclo[3.3.0]octyl)-3-quinolone- et -naphtyridone-carboxyliques de formule (I) 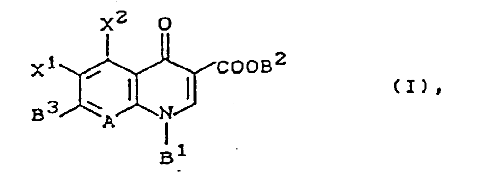 dans laquelle
X¹ représente un halogène
X² est de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, halogéno, méthyle,
B¹ est un groupe alkyle ayant 1 à 4 atomes de carbone, alcényle ayant 2 à 4 atomes de carbone, cycloalkyle ayant 3 à 6 atomes de carbone, 2-hydroxyéthyle, 2-fluoréthyle, méthoxy, amino, méthylamino, éthylamino, diméthylamino, phényle éventuellement substitué par 1 ou 2 atomes de fluor,
B² est de l'hydrogène, un groupe alkyle ayant 1 à 4 atomes de carbone ou le groupe (5-méthyl-2-oxo-1,3-dioxole-4-yl)méthyle,
B³ représente un reste de structure 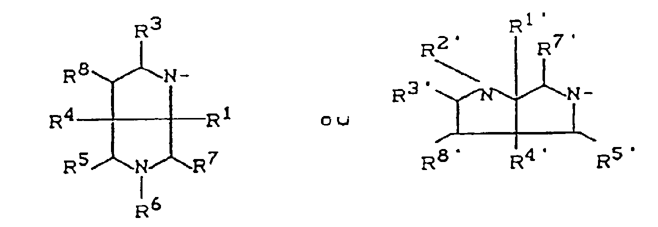 dans laquelle
R¹, R³, R⁴, R⁵, R⁷ et R⁸ sont identiques ou différents et représentent chacun de l'hydrogène ou un groupe méthyle qui peut être substitué, le cas échéant, par un halogène ou un substituant hydroxy, un groupe (alkoxy en C₁ ou C₂)carbonyle ou carboxy,
R⁴ et R⁸ peuvent représenter en outre un halogène, un groupe amino, hydroxy ou méthoxy,
R⁶ représente de l'hydrogène, un groupe alkyle en C₁ à C_{6;} benzyle, aryle en C₆ à C₁₂ , alcanoyle en C₁ à C₃, benzoyle, (alkoxy en C₁ à C₅)carbonyle,
R^{1'}, R^{2'}, R^{3'}, R^{5'}, R^{7'} et R^{8'} sont identiques ou différents et représentent chacun de l'hydrogène ou un groupe méthyle,
R⁴' est un halogène, un groupe amino, hydroxy, méthoxy ou un groupe méthyle substitué par un radical halogéno, amino, hydroxy ou méthoxy ou représente de l'hydrogène, lorsque
R2' et R3' forment conjointement un pont alkylène en C₂ à C₄ ou un pont CH₂-S-CH₂ qui peut être substitué une ou deux fois, le cas échéant par un radical hydroxy, méthoxy, amino ou méthyle,
A représente N ou C-R⁹, où
R⁹ est de l'hydrogène, un halogène, un groupe méthyle, cyano, nitro, hydroxy ou méthoxy
et leurs hydrates et leurs sels d'addition d'acides acceptables du point de vue pharmaceutique ainsi que leurs sels de métaux alcalins, de métaux alcalino-terreux, d'argent et de guanidinium.

2. Composés suivant la revendication 1,
dans lesquels
X¹ est du fluor ou du chlore,
X² est de l'hydrogène, un groupe amino, alkylamino ayant 1 ou 2 atomes de carbone, diméthylamino, hydroxy méthoxy, mercapto, méthylthio, fluoro, méthyle,
B¹ est un groupe alkyle ayant 1 à 3 atomes de carbone, un groupe alcényle ayant 2 ou 3 atomes de carbone, un groupe cycloalkyle ayant 3 à 5 atomes de carbone, un groupe 2-hydroxyéthyle, 2-fluoréthyle, méthoxy, amino, méthylamino, éthylamino, diméthylamino, phényle éventuellement substitué par 1 ou 2 atomes de fluor,
B² est de l'hydrogène, un groupe alkyle ayant 1 à 3 atomes de carbone ou le groupe (5-méthyl-2-oxo-1,3-dioxole-4-yl)méthyle,
B³ est un reste de la structure décrite dans la revendication 1, où
R¹, R³, R⁴, R⁵, R⁷ et R⁸ sont identiques ou différents et représentent chacun de l'hydrogène ou un groupe méthyle qui peut être substitué, le cas échéant, par un halogène, ou un groupe (alkoxy en C₁ à C₃)carbonyle,
R⁴ peut être en outre un halogène tel que le fluor, le chlore ou le brome,
R⁶ représente de l'hydrogène, un groupe alkyle en C₁ à C₃, benzyle, phényle, acétyle, benzoyle, (alkoxy en C₁ à C₄)carbonyle,
R¹', R²', R³', R⁵, R^{7'} et R^{8'} sont identiques ou différents et représentent chacun de l'hydrogène ou un groupe méthyle,
R⁴' est un halogène tel que le chlore ou le brome ou représente de l'hydrogène lorsque
R2' et R3' forment conjointement un pont alkylène en C₂ à C₄ ou un pont CH₂-S-CH₂ qui peut être substitué une ou deux fois, le cas échéant par un radical hydroxy ou méthyle,
A représente N ou C-R⁹, dans lequel
R⁹ est de l'hydrogène, un halogène ou un groupe méthoxy,
et leurs hydrates et leurs sels d'addition d'acides acceptables du point de vue pharmaceutique ainsi que leurs sels de métaux alcalins et de métaux alcalino-terreux.

3. Composés suivant la revendication 1,
dans lesquels
X¹ est du fluor,
X² est de l'hydrogène, un groupe amino, du fluor, un groupe méthyle,
B¹ est un groupe alkyle ayant 1 ou 2 atomes de carbone, vinyle, cyclopropyle, 2-hydroxyéthyle, 2-fluoréthyle, méthoxy, méthylamino, 4-fluorophényle, 2,4-difluorophényle,
B² est de l'hydrogène, un groupe alkyle ayant 1 ou 2 atomes de carbone,
B³ est un reste de la structure décrite dans la revendication 1, où
R¹, R³, R⁴, R⁵, R⁷ et R⁸ sont identiques ou différents et représentent chacun de l'hydrogène ou un groupe méthyle qui peut être substitué, le cas échéant, par du fluor,
R⁴ peut représenter en outre du fluor, du chlore ou du brome,
R⁶ est de l'hydrogène, un groupe méthyle, acétyle, (alkoxy en C₁ à C₄)carbonyle,
R^{1'}, R^{2'}, R^{3'}, R^{5'}, R^{7'} et R^{8'} sont identiques ou différents et représentent chacun de l'hydrogène ou un groupe méthyle,
R⁴' est du chlore ou du brome ou représente de l'hydrogène lorsque
R²' et R³' forment conjointement un pont alkylène en C₂ à C₄ ou un pont CH₂-S-CH₂ qui peut éventuellement être substitué une ou deuxfois par un radical hydroxy ou méthyle,
A représente N ou un groupe C-R⁹, dans lequel
R⁹ est de l'hydrogène, un halogène ou un groupe méthoxy,
et leurs hydrates et leurs sels d'addition d'acides acceptables du point de vue pharmaceutique ainsi que leurs sels de métaux alcalins.

4. L'acide 1-cyclopropyl-6,8-difluoro-1,4-dihydro-7-(7-méthyl-2,7-diazabicyclo[3.3.0]oct-2-yl)-4-oxo-3-quinoléine- carboxylique de formule

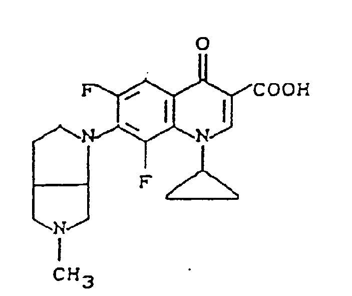

5. L'acide 8-chloro-1-cyclopropyl-6-fluoro-1,4-dihydro-7-(7-méthyl-2,7-diazabicyclo[3.3.0]oct-2-yl)-4-oxo-3-quinoléine-carboxylique de formule

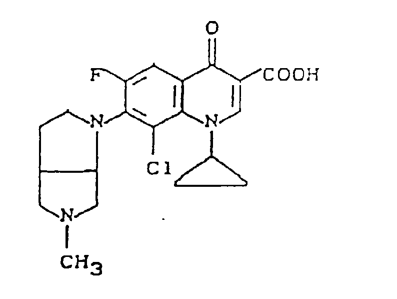

6. L'acide 1-cyclopropyl-6;8-difluoro-1,4-dihydro-7-(5-chloro-2,7-diazabicyclo[3.3.0]oct-2-yl)-4-oxo-3-quinoléine- carboxylique de formule

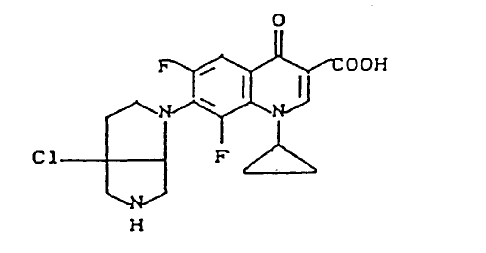

7. L'acide 1-cyclopropyl-7-(1,4-diazatricyclo[6.2.0.0^{2,6}]dec-4-yl)-6,8-difluoro-1,4-dihydro-4-oxo-3-quinoléine-carboxylique de formule

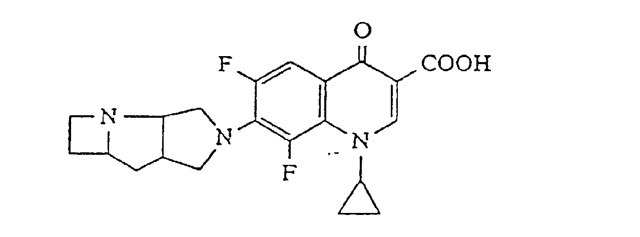

8. L'acide 1-cyclopropyl-7-(2,7-diazabicyclo[3.3.0]oct-2-yl)-6,8-difluoro-1,4-dihydroxy-4-oxo-3-quinoléine-carboxylique de formule

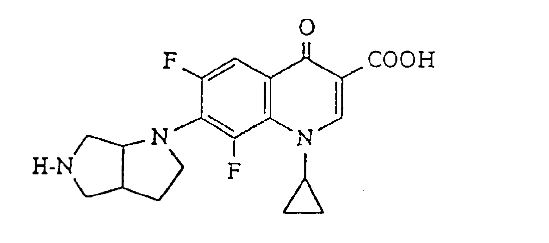

9. Procédé de production de composés suivant la revendication 1, caractérisé en ce qu'on fait réagir des composés de formule (II) 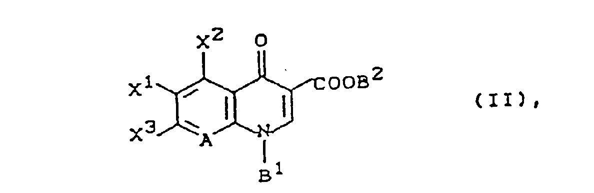 dans laquelle
A B¹, B², X¹ et X² ont la définition indiquée dans la revendication 1 et
X³ représente un halogène, en particulier le fluor ou le chlore, avec des composés de formule (III) 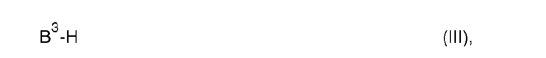 dans laquelle
B³ a la définition indiquée dans la revendication 1,
le cas échéant en présence d'accepteurs d'acides, et on élimine éventuellement des groupes protecteurs contenus dans B³, des composés de formule (I)
dans laquelle
X¹, B¹, B², B³ et A ont la définition indiquée dans la revendication 1 et
X² représente un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone ou arylthio,
pouvant aussi être obtenus par réaction d'un composé de formule (IV) 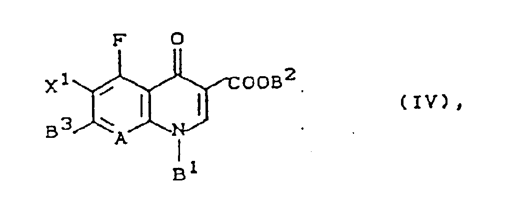 dans laquelle
X¹, B¹, B², B³ et A ont la définition indiquée dans la revendication 1,
avec des composés de formule (V) 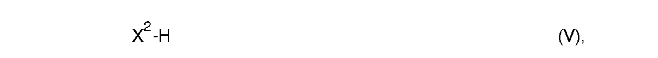 dans laquelle
X² a la définition indiquée ci-dessus,
le cas échéant en présence d'accepteurs d'acides.

10. Utilisation de composés suivant les revendications 1 à 8 pour la préparation de médicaments.

11. Utilisation de composés suivant les revendications 1 à 8 comme additifs dans des aliments pour le bétail.

12. Aliments pour le bétail et additifs pour aliments pour le bétail et mélanges préalables contenant des composés suivant les revendications 1 à 8.
